(19)

**Europäisches Patentamt**
**European Patent Office**
**Office européen des brevets**

(11) **EP 4 531 057 A1**

(12) **EUROPEAN PATENT APPLICATION**

(43) Date of publication:
02.04.2025 Bulletin 2025/14

(21) Application number: 24203337.1

(22) Date of filing: 27.09.2024

(51) International Patent Classification (IPC):
*G16H 50/70* (2018.01)  *G16H 50/20* (2018.01)
*G06N 20/20* (2019.01)

(52) Cooperative Patent Classification (CPC):
G16H 50/70; G06N 20/20; G16H 50/20

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.09.2023 US 202318478889

(71) Applicant: Tempus AI, Inc.
Chicago, IL 60654-2282 (US)

(72) Inventors:
• VIDMAR, David Michael
Amherst, MA 01003 (US)
• THOMSON IV, William E.
Denver, CO 80219 (US)
• CHEN, Rui Jun
Livingston, NJ 07039 (US)

(74) Representative: Grund, Martin et al
Grund Intellectual Property Group
Patentanwälte und Solicitor PartG mbB
Steinsdorfstraße 2
80538 München (DE)

(54) **METHODS AND SYSTEMS FOR DISEASE PHENOTYPING USING MULTIMODAL EHR DATA AND WEAK SUPERVISION**

(57) Methods, systems, and software are provided for training a model for phenotyping subjects with respect to a medical condition. The method includes generating a plurality of labeling functions for the medical condition, wherein each respective labeling function in the plurality of labeling functions comprises a corresponding set of one or more criterion that, when satisfied, indicate a presence or an absence of the medical condition. The method also includes assigning, to each respective medical record in a first plurality of medical records, a corresponding label indicating a status of the medical condition by evaluating first information from the respective medical record using an ensemble model comprising the plurality of labeling functions to obtain as output from the ensemble model a prediction for the status of the medical condition, wherein the evaluating comprises natural language processing of at least a portion of the respective medical record.

FIG. 3

**Description**

**BACKGROUND**

The near ubiquitous use of electronic health records (EHRs) in academic, clinical, and healthcare institutions has the potential to generate large datasets for studying medical conditions and treatment thereof across large patient populations. Such EHRs can store vast amounts of patient-specific data over multiple healthcare visits, including large datasets from genetic sequencing and digital imaging examinations. Despite its potential as a valuable resource for large patient population studies and clinical decision-making, the quality and interpretability of the data contained in EHRs can be inconsistent. For instance, EHRs can include clinical and research data stored across multiple systems in a variety of formats and structures, or recorded at varying levels of accuracy, completeness, and depth, depending on the source of intake. An analysis of 10 years of EHR data from a representative clinical data provider reported between 6% and 46% incompleteness for several study variables related to pancreatic cancer. Similar completeness issues were observed in EHR data related to clinical trial recruitment. See, for instance, Coorevits et al., "Electronic health records: new opportunities for clinical research." J Intern Med. 2013;274(6):547-560, which is hereby incorporated herein by reference in its entirety.

Moreover, to perform studies across large patient populations, the information contained within EHRs must be extracted and put into context. For instance, supervised machine learning of models that can identify patient subpopulations and/or predict therapeutic outcomes based on EHR data require labeled training data. Manual phenotyping of EHRs is laborious and not practical on the scale of records required for population scale studies. Several electronic methods for EHR phenotyping have been proposed, such as rapid phenotyping using structured medical billing codes and rule-based phenotyping that require confirmation of structured medical billing codes with a secondary indication of a medical condition. However, these approaches have limited accuracy. See, for instance, Pendergrass and Crawford, "Using Electronic Health Records to Generate Phenotypes For Research," Curr Protoc Hum Genet., 100(1):e80 (2019), which is hereby incorporated herein by reference in its entirety. For example, billing codes do not always accurately reflect disease status and/or may be used when a clinician is referring a patient to a specialist for further evaluation prior to diagnosis. Further, rules-based phenotyping that require confirmation of such billing codes are rigid, requiring specific tests have been run on the patient. Moreover, these phenotyping approaches do not allow for the identification of patients with an undiagnosed medical condition and, in fact, obfuscate model training by labeling such patients as negative for a medical condition they have.

**SUMMARY**

As described above, EHRs hold the potential for large-scale studies of medical disorders and treatment across populations. However, there is a need in the art for improved systems and methods for phenotyping patients from EHRs. Advantageously, the present disclosure provides, in some aspects, methods and systems for phenotyping EHRs for one or more medical conditions using an ensemble of labeling functions that evaluate multimodal data contained within the EHRs.

In one aspect, the disclosure provides methods, as well as systems and non-transitory computer readable media for performing such methods, for training a model for phenotyping subjects with respect to a medical condition. The method is performed at a computer system that includes one or more processors and memory. The method includes generating a plurality of labeling functions for the medical condition. Each respective labeling function in the plurality of labeling functions comprises a corresponding set of one or more criterion that, when satisfied, indicate a presence or an absence of the medical condition. The method also includes assigning, to each respective medical record in a first plurality of medical records, a corresponding label indicating a status of the medical condition by evaluating first information from the respective medical record using an ensemble model comprising the plurality of labeling functions to obtain as output from the ensemble model a prediction for the status of the medical condition, where the evaluating comprises natural language processing of at least a portion of the respective medical record.

In one aspect, the disclosure provides methods, systems, and non-transitory computer readable media for training a model for phenotyping subjects with respect to a medical condition. The method is performed at a computer system that includes one or more processors and memory. The method includes generating a plurality of labeling functions for the medical condition, where each respective labeling function in the plurality of labeling functions comprises a corresponding set of one or more criterion that, when satisfied, indicate a presence of the medical condition. The method includes assigning, to each respective subject in a first plurality of subjects, a corresponding label indicating a status of the medical condition by evaluating first information from a corresponding medical record for the respective subject using an ensemble model comprising the plurality of labeling functions to obtain as output from the ensemble model a prediction for the status of the medical condition, wherein the evaluating comprises natural language processing of at least a portion of the respective medical record.

In some embodiments, the method also includes, prior to generating the plurality of labeling functions, filtering a second plurality of medical records in accordance with a first set of characteristics for the medical condition, wherein the filtering comprises applying an extraction filter to the second plurality of medical records, to obtain the first plurality of medical records, wherein the first plurality of medical records is a subset of the second plurality of medical records.

In some embodiments, the method also includes, training a classification model for phenotyping subjects with respect to the medical condition using, for each respective medical record in the first plurality of the subset of medical records, (i) the corresponding label as a dependent variable and (ii) second information from the respective medical record as independent variables.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

## BRIEF DESCRIPTION OF THE DRAWINGS

FIGS. **1A, 1B, 1C,** and **1D** collectively illustrate a block diagram of an example computing device for training a model for phenotyping subjects with respect to a medical condition, in accordance with some embodiments of the present disclosure.

FIGS. **2A, 2B, 2C, 2D, 2E, 2F, 2G, and 2H** collectively provide a flow chart of processes and features for training a model for phenotyping subjects with respect to a medical condition, in which dashed boxes represent optional portions of the method, in accordance with some embodiments of the present disclosure.

FIG. **3** illustrates an example schematic for training a model for phenotyping subjects with respect to a medical condition, in accordance with some embodiments of the present disclosure.

FIGS. **4A, 4B, 4C, 4D, 4E, 4F, and 4G** collectively illustrate representations of target patient populations within a healthcare records data base and efforts to identify the population through various search strategies, in accordance with some embodiments of the present disclosure.

FIG. **5** illustrates data from clinical and electronic review of patient records to identify instances of pulmonary hypertension, in accordance with some embodiments of the present disclosure.

FIG. **6A** illustrates a majority voting Probabilistic Graphical Modeling (PGM) model, in accordance with some embodiments of the present disclosure.

FIG. **6B** illustrates a generalized majority voting PGM model, in accordance with some embodiments of the present disclosure.

## DETAILED DESCRIPTION

### *Introduction.*

Electronic health records (EHRs) provide a useful resource for individualized health data, by combining huge amounts of patient-specific data collected during the provision of healthcare services. For instance, EHRs can include one or more of clinical, demographic, administrative, claims (e.g., medical and pharmacy), and patient-centered (e.g., vital statistics or quality-of-life information obtained from medical instruments or caregiver assessments) data. Examples of data types that can be included in EHRs include medication history (*e.g.,* current prescriptions, concomitant medications, medication classes, medication codes, and/or ontological terms), disease conditions *(e.g.,* pre-existing conditions, co-morbidities, symptoms, diagnoses, and/or prognoses), laboratory test results or clinical data (*e.g.,* biomarkers, genomic variants, medical images, and/or sequencing data), and free-text observations and other notes (*e.g.,* by a clinician). In some instances, EHRs include longitudinal data, such as information collected over multiple visits to a healthcare provider or over a period of time. See, for example, Coorevits et al., "Electronic health records: new opportunities for clinical research." J Intern Med. 2013;274(6):547-560; Cowie et al., "Electronic health records to facilitate clinical research." Clin Res Cardiol. 2017; 106(1):1-9; and Xiao et al., "Opportunities and challenges in developing deep learning models using electronic health records data: a systematic review." J Am Med Inform Assoc. 2018;25(10): 1419-1428, each of which is hereby incorporated herein by reference in its entirety.

Existing EHR phenotyping commonly seen in observational clinical research may vary widely in methods and quality, and often suffers from a number of limitations. Many studies utilize diagnosis codes such as ICD-9, ICD-10, or SNOMED codes without validating the quality of these codes to determine how well they represent their desired cohort of interest. Within studies utilizing claims data, there is limited ability to perform detailed chart reviews or manual abstraction for the development of phenotypes or code-based cohort definitions, although some population and cohort-level metrics may be used in aggregate to ascertain if the cohort seems grossly appropriate from a clinical perspective. Unfortunately, many studies do not perform any kind of cohort characterization or chart review validation, blindly trusting that codes primarily developed for non-research purposes (most often billing purposes, making research a secondary use) represent exactly what the code description says they should represent. However, modern coding in EHR systems is messy, incomplete, and often incorrect-whether by design, due to changes in a patient's clinical status and working diagnosis as they proceed along a diagnostic pathway or by accident, due to busy clinical schedules or data entry or mapping errors. Therefore, codes for either common diseases or those linked with high levels of reimbursement may be accurately captured, but more often than not, there are pitfalls and shortcomings. The same issues hold true for procedure codes, problem lists (some institutions update them regularly, some ignore them altogether), medications, and most other sources of EHR data. Based on practice patterns or geographic regions, codes to best capture the same disease may change dramatically, making code-based phenotypes limited in their generalizability.

Conventional methods for EHR data analysis is further limited by the lack of labels in many EHR databases. Labels impart considerable benefit in model training, by providing "ground truth" indications for clinical outcomes or phenotypes that can be used to strengthen a model's capability to accurately generate a desired output. Such labels are often not consistently recorded in EHR data and thus are typically unavailable for model training. Manual labeling of EHRs requires considerable resources and expense, as it relies on experts trained to read, comprehend, and accurately classify the information in each record according to each desired analytic goal. Additionally, it is exceedingly laborious and time-intensive, particularly for large databases of training EHRs (EHR databases can contain millions, tens of millions, or hundreds of millions of individual records). While some conventional approaches utilize supervised learning based on the detection of medical codes (*e.g.,* diagnosis, procedure, or medication codes) to obtain labels for EHRs, such methods are not readily generalizable to all records, such as those that do not conform to specific institutional or organizational coding standards. See, for example, Xiao et al., "Opportunities and challenges in developing deep learning models using electronic health records data: a systematic review." J Am Med Inform Assoc. 2018;25(10):1419-1428, which is hereby incorporated herein by reference in its entirety.

Various sources of patient information can be used to build a structured phenotype for determining the PH status of a patient. For example, information available in administrative claims and/or medical billing databases may indicate whether a patient has PH. Advantageously, these databases are large, readily accessible, and provide information for a diverse national or international population. However, the data can be difficult to validate, does not include medication data such as OTC medications, and oftentimes provide a biased patient sample, *e.g.,* patients having a certain insurance.

Code-based structured phenotypes can also be derived from electronic health records (EHRs). Such phenotypes are relatively fast to develop since EHR codes have defined correspondences and are relatively simple to validate through clinician review. However, code-based structural phenotypes are more rigid, lack generalizability (*e.g.,* because of significant variation in code use and/or clinical practice across different geographical regions and even from site-to-site), and provide a view of the patient that is limited to fielded codes.

Thus, improved methods and systems for phenotyping medical records are needed. Advantageously, the present disclosure addresses these and other needs by providing methods and systems for building and validating disease phenotypes using multimodal EHR data and weak supervision. In some embodiments, the methods and systems described herein use signal aggregation together with natural language processing (NLP) and machine learning to better identify all medical condition subgroups of interest using more specific boundaries.

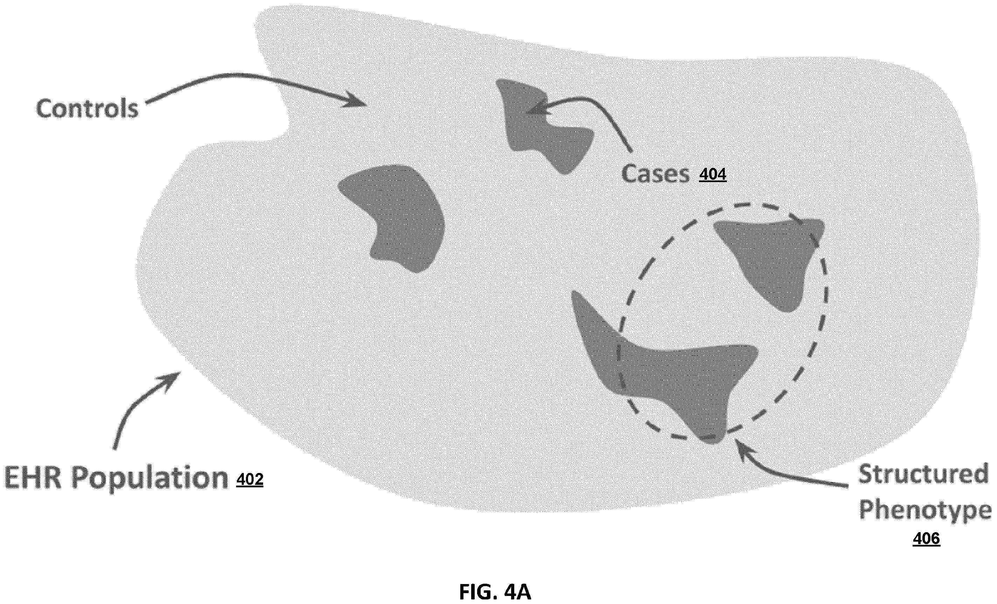

Many medical conditions have a relatively low prevalence within the general population and will be indicated by different subsets of multimodal data in EHRs, owing to different underlying disease etiology, variation in disease manifestation, and the inconsistencies in medical record keeping described above. This is illustrated in Figure 4A, where a plurality of EHRs are represented as a function of information stored in each EHR that indicates whether or not a subject has experienced a medical condition. EHRs from patients that have experienced the medical condition are shown as darker shapes with the representation. The use of a conventional code-based structured phenotype, indicated by a dashed oval, to identify patients who have experienced the medical condition may initially capture one or more groups fairly well but are rigid, resulting in the identification of false positives-EHRs within an oval that are not part of the darker shape-and missing other instances of the medical condition-darker shapes not circumscribed by a dashed oval. In this analogy, adding or subtracting a diagnostic code may expand or shrink the oval, with the concomitant tradeoffs in terms of PPV or sensitivity of such an action, but may not be able to customize or reshape the borders of this cohort to best fit the actual border of the cases themselves.

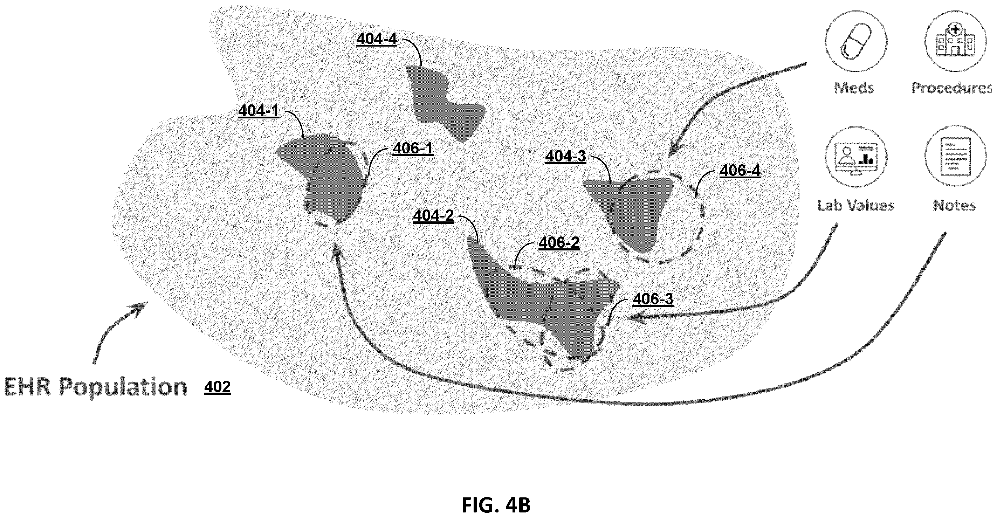

One way of improving these fairly rigid constraints is to add additional sources, tables, or modalities of data, such as adding medications, procedures, lab values, imaging, or notes to an initial diagnosis code-based phenotype, as illustrated in Figure 4B. These additional criteria may better fit or identify additional subgroups (additional darker shapes) which may not be able to be identified by other types of data. However, by themselves, each additional data type may still remain fairly rigid in a structured phenotype, essentially creating multiple overlapping and non-overlapping rigid ovals of varying sizes, but still unable to closely represent the exact unique cutoffs or jagged borders between cases and controls. In addition, there may be subgroups, such as the subgroup near the top of the figure, which may not be captured at all by structured data elements. In short, using multiple modalities or types of data does help, but in most cases, significant limitations remain, especially for a challenging, relatively rare and heterogeneous disease such as pulmonary hypertension (PH), which has a less than 1% prevalence in the population.

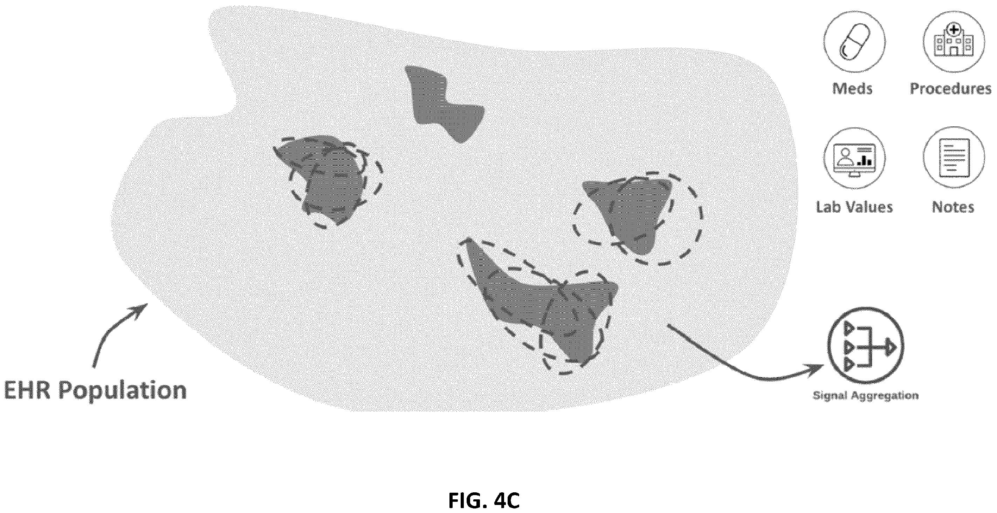

Beyond just adding additional data elements with accompanying additional logic to integrate them, the methods and systems described herein utilize signal aggregation, together with NLP and machine learning models to better identify all disease subgroups of interest along with more specific boundaries. In signal aggregation (Figure 4C), numerous signals are defined from these multimodal data features using different labeling functions. Because no single, final phenotype is generated from each of these multiple data elements, a large number of useful rules can be created. In fact, the resulting weak labels do not need to be perfect, accounting for the numerous additional dotted ovals in Figure 4C.

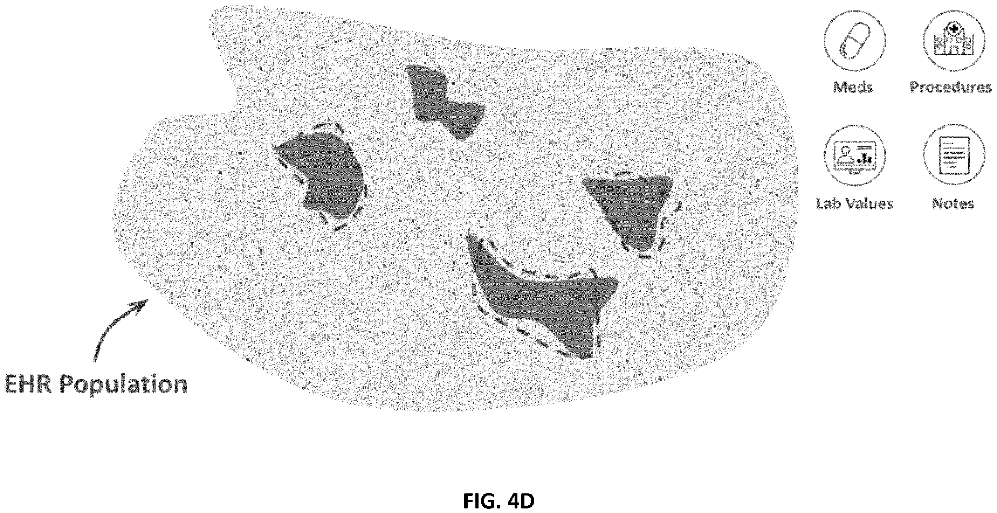

These signals then get aggregated using statistical and technical methods to generate labels for patients which are more custom fit to the actual patient subgroups after the signal aggregation step, as seen by the dotted shapes in Figure 4D that are no longer forced to be rigid circular shapes. As described in further detail herein, signal aggregation represents an opportunity to generate a higher-performing subgroup, which can then be compared to all prior structured phenotypes based on chart review validation metrics to select the best performing model. However, despite signal aggregation, the final cohort may still be improved upon or may still miss some subgroups which were not at all represented in the structured data elements and labeling functions.

In some embodiments, the aggregation is accomplished using a voting method, such as a majority voting method. That is, labeling functions define different segments of the medical records indicating the presence of the disease. Each labeling function is evaluated over a subject's medical record and the result of the evaluation is used as a single vote, aggregated together with the votes provided from every other labeling function evaluated over the subject's medical record. In some embodiments, the aggregation is accomplished by solving for a probabilistic graphing modeling (PGM) model that applies one or more priors, *e.g.,* associated with the performance of an individual labeling function, to the result of the evaluation of each labeling over the subject medical record. Examples of the evaluation of such PGM models is shown, for instance, in **Example 4.** In some embodiments, the aggregation is accomplished using snorkel's probabilistic model to aggregate the result of the evaluation of each labeling over the subject medical record.

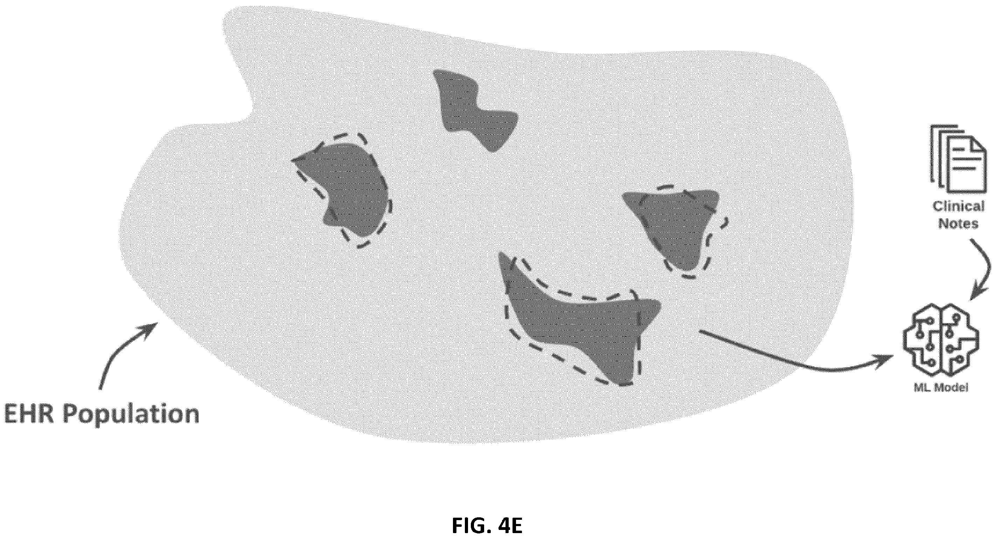

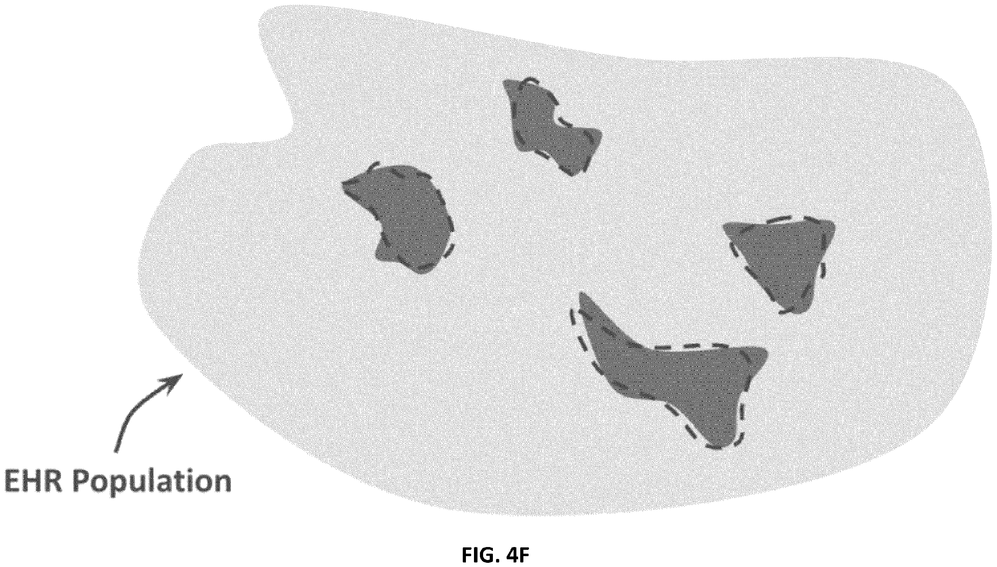

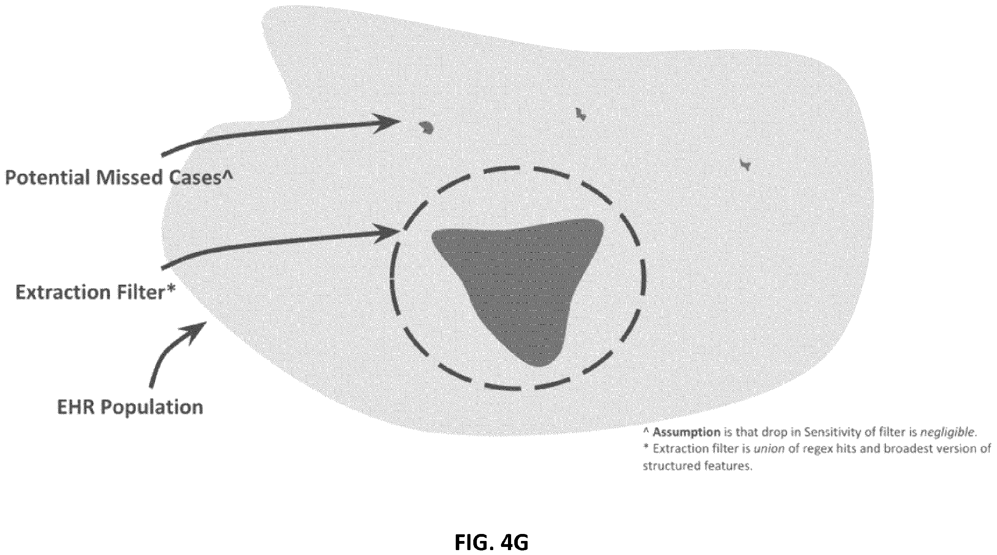

As described above, the dashed shapes in Figure 4 (*e.g.,* structured phenotype 406) are abstract representations of a phenotype for a medical condition. When structured queries are used to identify these phenotypes, the identified cohorts are "rigid" (*e.g.,* as represented by a regular shape such as the oval for structured phenotype 406). However, when more complicated aggregation models are used to identify subjects with the phenotype, the identified signal becomes more accurate, resulting in a more "flexible" identification of subjects (*e.g.,* as represented by the irregular shapes in Figure 4D resulting from aggregation of the labeling functions).

In some embodiments, further improvements on the phenotype are made by training a machine learning model, such as a transformer-based, NLP model off of the aggregate weak labels post-signal aggregation or from the structured phenotype itself, as illustrated in Figure 4E. This machine learning model then may be able to identify signals or learn relationships, *e.g.,* in unstructured clinical note EHR data that can not only allow it to better identify the boundaries between cases and controls for those represented in the structured data elements, but also identify new subgroups such as the top cohort in the figure which was previously not represented by any structured data element or labeling function, as illustrated in Figure 4F.

In order to overcome these challenges, a method was developed that integrates different structured phenotypes identifiable from different modalities of medical data to accurately label medical records. In some embodiments, the method begins by defining a patient population. Examples of considerations that can be used to describe a patient population include (i) what clinical population / entities are being targeted, (ii) what research question and/or predicted task is being addressed, (iii) what is the clinical and/or research use case, and (iv) what patients should be specifically included or excluded, and over what time frame. For example, a target population can be described as (adult and/or adolescent) patients with X medical condition, optionally who (have received / are receiving) Y therapeutic intervention, optionally (with or without) a prior history of Z.

In some embodiments, the methods described herein include generating a plurality of labeling functions for the medical condition. Each respective labeling function in the plurality of labeling functions comprises a corresponding set of one or more criterion that, when satisfied, indicate a presence or an absence of the medical condition. The method also includes assigning, to each respective medical record in a first plurality of medical records, a corresponding label indicating a status of the medical condition by evaluating first information from the respective medical record using an ensemble model comprising the plurality of labeling functions to obtain as output from the ensemble model a prediction for the status of the medical condition, where the evaluating comprises natural language processing of at least a portion of the respective medical record.

In some embodiments, the method also includes, prior to generating the plurality of labeling functions, filtering a second plurality of medical records in accordance with a first set of characteristics for the medical condition, wherein the filtering comprises applying an extraction filter to the second plurality of medical records, to obtain the first plurality of medical records, wherein the first plurality of medical records is a subset of the second plurality of medical records.

In some embodiments, the method also includes, training a classification model for phenotyping subjects with respect to the medical condition using, for each respective medical record in the first plurality of the subset of medical records, (i) the corresponding label as a dependent variable and (ii) second information from the respective medical record as independent variables.

Advantageously, the methods described herein are more robust than commonly used, standard phenotyping techniques, which often rely on a limited number of codes, modalities, or data elements (such as focusing only on diagnostic codes or procedures). For example, many studies, especially in early observational research or less methodologically rigorous studies, use only a single ICD code or a limited number of codes. Such code-based structured phenotypes are overly rigid, with significant tradeoffs between capturing more cases (true positives) and limiting the number of controls (false positives). The methods and systems described herein, create a more flexible model to better characterize difficult and/or rare diseases. In some embodiments, this is realized, at least in part, through the use of weak supervision for model development, as described further below.

### *Example Implementation.*

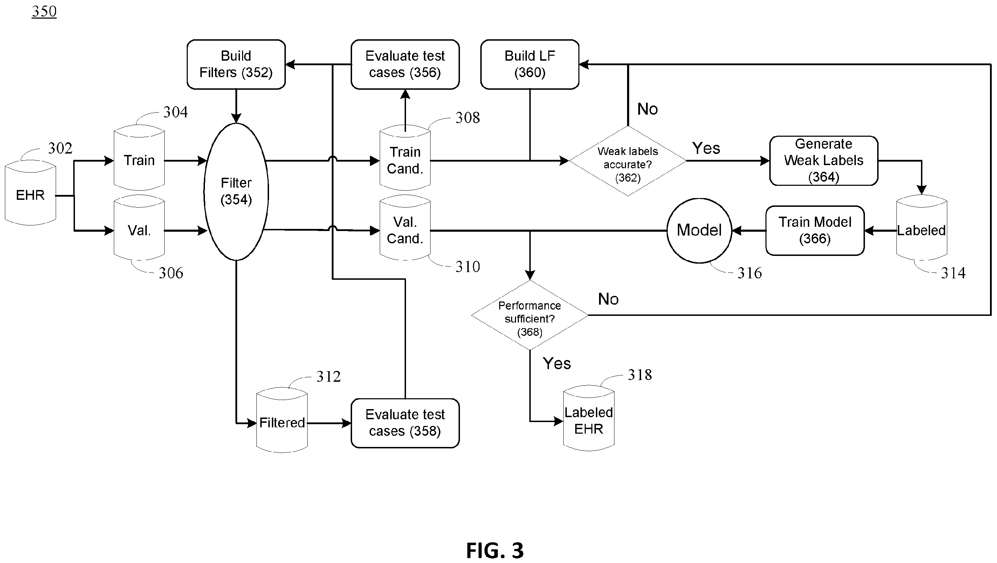

An illustrative implementation 300 of methods for training a model for phenotyping subjects with respect to a medical condition, in accordance with some embodiments of the present disclosure, provided herein will now be described in conjunction with Figure 3. In Figure 3 a plurality of electronic health records (EHRs) 302 are split into a training fold 304 and a validation fold 306. While this split is illustrated prior to filtering (354), label function building (360), and weak labeling (364) in Figure 3, the split into training and validation folds can occur any time prior to model training (366).

As illustrated in Figure 3, implementation 300 includes initial steps of building filters (352) and filtering (354) medical records 302, to remove filtered records 312 thereby generating a subplurality of candidate training records 308 and a subplurality of candidate validation records 310. However, in some implementations, no initial filtering is performed and the weak labeling method is performed using the entirety of the starting plurality of EHRs 302. In some embodiments, building filters (352) includes developing a set of clinical logic to identify a target cohort or outcome of interest. In some embodiments, the set of clinical logic includes one or more first types of conditions that, when satisfied, indicate the presence of a target medical condition (*e.g.,* a diagnosis or clinical outcome). In some embodiments, the set of clinical logic includes one or more second types of conditions that, when satisfied, indicate the absence of the target medical condition. In some embodiments, the set of clinical logic includes both first types of conditions and second types of conditions. In some embodiments, the set of clinical logic only includes first types of conditions.

In some embodiments, filtering is done only using clinical logic including conditions that, when satisfied, indicate the presence of the medical condition. That is, in some embodiments, the filtering only includes inclusion criteria and does not include exclusion criteria.

In some embodiments, one or more conditions used for the clinical logic evaluate text in, or extracted from, an electronic health record. For example, in some embodiments, a list of symptoms and/or relevant mentions of a medical condition that may be included in an unstructured clinical notes section of an EHR are curated. The curated list(s) can then be used to search EHRs (or databases populated from clinical notes sections of EHRs), e.g., by NLP such as regular expression searching, for indications that the subject has the medical condition. In some embodiments, the filter is further refined by building exclusions. In some embodiments, these exclusions are removed from text prior to searching the text for the curated list of indications. In some embodiments, the exclusions are removed after being identified during the searching of the text. For example, when searching for indications of pulmonary hypertension, commonly abbreviated as PH, the term Ph.D. may be excluded from the search criteria.

On goal of this filtering is to refine the subject population down to a reasonable size while maintaining confidence that little to no instances of the medical condition in the starting database are removed by the filter. As such, in some embodiments, the focus of the filtering is to remove only those medical records for which there is a high confidence that they do not include an instance of the target medical condition. As such, in some embodiments, the filter reflects this by casting the widest possible net that still reduces the set of medical records to a reasonable size. As such, it is beneficial to include medical records for which it is unclear whether they include an instance of the medical condition in the filtered subset.

When implemented, the filtering (354) reduces the total number of medical records that need to be processed, thereby reducing the time required to generate weak labels and train phenotyping models, and/or enriches a set of medical records for instances of a target medical condition. In certain embodiments, particularly where there is a low instance of a target medical condition in the population, enriching the training set for instances of such medical condition reduces overfitting and allows the model to better generalize instances of the medical condition. Accordingly, in some embodiments, the methods described herein include a step of filtering a plurality of medical records (*e.g.,* EHRs 302) in accordance with a first set of characteristics (*e.g.,* criteria 52) for the medical condition. The filtering includes applying (354) an extraction filter (*e.g.,* filters 51) to plurality of medical records 302, to obtain a subplurality of medical records (*e.g.,* subplurality of candidate training records 308 and a subplurality of candidate validation records 310).

The filtering also allows for random selection of patients (EHRs) from the filtered population to provide a reasonable number of positive instances of the target medical condition, *e.g.,* at least 25%. These randomly selected EHRs can be independently reviewed for use in evaluating labeling functions, evaluating / validating labeling ensemble models, validating downstream machine learning phenotyping models, etc. That is, the filtering allows for robust validation of the phenotyping models described herein.

As illustrated in Figure 3, in some embodiments, implementation 300 also includes evaluating (356) instances of the subplurality of candidate EHRs that made it through the filtering (354) and optionally refining the filters when it is determined that more EHRs that don't include instances of the medical condition are making it through the filter than desired. For example, one or more filters may be modified and/or added to exclude EHRs when certain exclusion criteria are met, thereby allowing fewer EHRs to pass through the filtering (354). Similarly, implementation 300 includes evaluating (356) instances of the filtered EHRs 312 that were removed from the plurality of EHRs 302 by the filtering (354) and optionally refining the filters when it is determined that EHRs that include instances of the medical condition are being filtered out. For example, one or more filters may be modified and/or added to include EHRs when certain inclusion criteria are met, thereby allowing more EHRs to pass through the filtering (354). In some embodiments, when the filters (*e.g.,* filters 51) are refined following the evaluating (354 and/or 356), the plurality of EHRs 302 are filtered (354) again, to update the set of EHRs (*e.g.,* subplurality of candidate training records 308 and subplurality of candidate validation records 310) used to build and test the model.

In some embodiments, a machine learning model 316, *e.g.,* trained based on the weak labels output from the phenotyping pipeline, is used for evaluating (358) all or a portion of the filtered EHRs 312. That is, in some embodiments, the evaluating (358) and/or refining (352) of filters happens after completion of an initial instance of the phenotyping pipeline 300. In some embodiments, the final model 316 evaluates all or a portion (*e.g.,* at least 10%, at least 20%, at least 30%, at least 40%, at least 50%, at least 60%, at least 70%, at least 80%, at least 90%, or more) of the filtered results 312 to rank filtered results, *e.g.,* using a model score, to identify those filtered records that most likely indicate an instance of the medical condition. In some embodiments, the identified filtered records can be used to update (352) the filters and the pipeline can be run again using the updated filters.

As illustrated in Figure 3, implementation 300 also includes building (360) a set of labeling functions (*e.g.,* labeling functions 61) and evaluating (362) whether application of the set of labeling functions results in accurate labeling of EHRs, *e.g.,* of one or more test cases in the subplurality of candidate training records 308. In some embodiments, test cases of EHRs (*e.g.,* of subplurality of candidate training records 308) are separately reviewed and determined to either indicate the presence of the medical condition or indicate the absence of the medical condition, which is used in the evaluating (362) to determine whether to refine one or more labeling functions (e.g., labeling functions 61) in the set of labeling functions.

As illustrated in Figure 3, building (360) the set of labeling functions is an iterative process. The process includes building phenotypes using multimodal data available in the EHRs, such as diagnosis codes, procedures, problem list, medications, encounters, demographics, orders, etc. This includes chart review for metrics, review, and iteration to continue to develop improved versions of the structured phenotype.

As illustrated in Figure 3, once the set of labeling functions (*e.g.,* labeling functions 61) the combination of multiple sources of data in the set of labeling functions is used for signal aggregation to generate (364) weak labels 47 for the dataset. That is, the labeling functions are applied to the EHRs (*e.g.,* subplurality of candidate training records 308) to label each EHR for model training, generating labeled EHRs 314. This process allows for the generation of phenotypes without the need for manual curation through the use of labeling functions and weak supervision.

As illustrated in Figure 3, implementation 300 also includes training (366) a model 316 using the signal aggregated weak labels 47 as independent variables and data from the EHRs 41 as dependent variables. The model can be evaluated using the subplurality of candidate validation records 310. In some embodiments, the subplurality of candidate validation records 310 are separately reviewed and determined to either indicate the presence of the medical condition or indicate the absence of the medical condition, in order to evaluate the performance of the model. In some embodiments, the subplurality of candidate validation records 310 are labeled using the ensemble model of labeling functions, in order to evaluate the performance of the model. In some embodiments, when the performance of the model 316 is not sufficient, the set of label functions is further iterated (360) to improve the weak labeling 364. When the model performance is found to be sufficient, the results of the model can be used to label EHRs.

***Definitions.***

The terminology used in the present disclosure is for the purpose of describing particular embodiments only and is not intended to be limiting of the invention. As used in the description of the invention and the appended claims, the singular forms "a," "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Furthermore, to the extent that the terms "including," "includes," "having," "has," "with," or variants thereof are used in either the detailed description and/or the claims, such terms are intended to be inclusive in a manner similar to the term "comprising."

As used herein, the term "if" can be construed to mean "when" or "upon" or "in response to determining" or "in response to detecting," depending on the context. Similarly, the phrase "if it is determined" or "if [a stated condition or event] is detected" can be construed to mean "upon determining" or "in response to determining" or "upon detecting [the stated condition or event]" or "in response to detecting [the stated condition or event]," depending on the context.

It will also be understood that, although the terms first, second, etc. are used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject. Furthermore, the terms "subject," "user," and "patient" are used interchangeably herein.

As used herein, the term "subject" refers to any living or non-living human. In some embodiments, a subject is a male or female of any stage (e.g., a man, a woman or a child).

As used herein, the term "mutation" or "variant" refers to a detectable change in the genetic material of one or more cells. In a particular example, one or more mutations can be found in, and can identify, cancer cells (*e.g.,* driver and passenger mutations). A mutation can be transmitted from a parent cell to a daughter cell. A person having skill in the art will appreciate that a genetic mutation (*e.g.,* a driver mutation) in a parent cell can induce additional, different mutations (*e.g.,* passenger mutations) in a daughter cell. A mutation generally occurs in a nucleic acid. In a particular example, a mutation can be a detectable change in one or more deoxyribonucleic acids or fragments thereof. A mutation generally refers to nucleotides that are added, deleted, substituted for, inverted, or transposed to a new position in a nucleic acid. In some embodiments, a variant refers to changes in the primary genome sequence at single or multiple nucleotide positions, e.*g.,* a single nucleotide variant (SNV), a multi-nucleotide variant (MNV), an indel (*e.g.,* an insertion or deletion of nucleotides), a DNA rearrangement (*e.g.,* an inversion or translocation of a portion of a chromosome or chromosomes), a variation in the copy number of a locus (*e.g.,* an exon, gene or a large span of a chromosome) (CNV), a partial or complete change in the ploidy of the cell, as well as in changes in the epigenetic information of a genome, such as altered DNA methylation patterns. A mutation can be a spontaneous mutation or an experimentally induced mutation. A mutation in the sequence of a particular tissue is an example of a "tissue-specific allele." For example, a tumor can have a mutation that results in an allele at a locus that does not occur in normal cells. Another example of a "tissue-specific allele" is a fetal-specific allele that occurs in the fetal tissue, but not the maternal tissue. As used herein, the term "single nucleotide variant" or "SNV" refers to a substitution of one nucleotide to a different nucleotide at a position *(*e.g., site) of a nucleotide sequence, *e.g.,* a sequence read from an individual. A substitution from a first nucleobase X to a second nucleobase Y, in some implementations, is denoted as "X>Y." For example, a cytosine to thymine SNV, in some embodiments, is denoted as "C>T."

As used herein, the term "cancer," "cancerous tissue," or "tumor" refers to an abnormal mass of tissue in which the growth of the mass surpasses and is not coordinated with the growth of normal tissue. A cancer or tumor can be defined as "benign" or "malignant" depending on the following characteristics: degree of cellular differentiation including morphology and functionality, rate of growth, local invasion and metastasis. A "benign" tumor can be well differentiated, have characteristically slower growth than a malignant tumor and remain localized to the site of origin. In addition, in some cases a benign tumor does not have the capacity to infiltrate, invade or metastasize to distant sites. A "malignant" tumor can be poorly differentiated (anaplasia), have characteristically rapid growth accompanied by progressive infiltration, invasion, and destruction of the surrounding tissue. Furthermore, a malignant tumor can have the capacity to metastasize to distant sites. Accordingly, a cancer cell is a cell found within the abnormal mass of tissue whose growth is not coordinated with the growth of normal tissue. Accordingly, a "tumor sample" refers to a biological sample obtained or derived from a tumor of a subject, as described herein. A cancerous tissue can refer to blood cells if the cancer is a hematological (blood) cancer.

As used interchangeably herein, the term "classifier" or "model" refers to a machine learning model or algorithm.

In some embodiments, a model includes an unsupervised learning algorithm. One example of an unsupervised learning algorithm is cluster analysis. In some embodiments, a model includes supervised machine learning. Nonlimiting examples of supervised learning algorithms include, but are not limited to, logistic regression, neural networks, support vector machines, Naive Bayes algorithms, nearest neighbor algorithms, random forest algorithms, decision tree algorithms, boosted trees algorithms, multinomial logistic regression algorithms, linear models, linear regression, Gradient Boosting, mixture models, hidden Markov models, Gaussian NB algorithms, linear discriminant analysis, or any combinations thereof. In some embodiments, a model is a multinomial classifier algorithm. In some embodiments, a model is a 2-stage stochastic gradient descent (SGD) model. In some embodiments, a model is a deep neural network (*e.g.,* a deep-and-wide sample-level model).

*Neural networks.* In some embodiments, the model is a neural network (*e.g.,* a convolutional neural network and/or a residual neural network). Neural network algorithms, also known as artificial neural networks (ANNs), include convolutional and/or residual neural network algorithms (deep learning algorithms). In some embodiments, neural networks are machine learning algorithms that are trained to map an input dataset to an output dataset, where the neural network includes an interconnected group of nodes organized into multiple layers of nodes. For example, in some embodiments, the neural network architecture includes at least an input layer, one or more hidden layers, and an output layer. In some embodiments, the neural network includes any total number of layers, and any number of hidden layers, where the hidden layers function as trainable feature extractors that allow mapping of a set of input data to an output value or set of output values. In some embodiments, a deep learning algorithm comprises a neural network including a plurality of hidden layers, *e.g.,* two or more hidden layers. In some instances, each layer of the neural network includes a number of nodes (or "neurons"). In some embodiments, a node receives input that comes either directly from the input data or the output of nodes in previous layers, and performs a specific operation, *e.g.,* a summation operation. In some embodiments, a connection from an input to a node is associated with a parameter (*e.g.,* a weight and/or weighting factor). In some embodiments, the node sums up the products of all pairs of inputs, $x_i$, and their associated parameters. In some embodiments, the weighted sum is offset with a bias, b. In some embodiments, the output of a node or neuron is gated using a threshold or activation function, f, which, in some instances, is a linear or non-linear function. In some embodiments, the activation function is, for example, a rectified linear unit (ReLU) activation function, a Leaky ReLU activation function, or other function such as a saturating hyperbolic tangent, identity, binary step, logistic, arcTan, softsign, parametric rectified linear unit, exponential linear unit, softPlus, bent identity, softExponential, Sinusoid, Sine, Gaussian, or sigmoid function, or any combination thereof.

In some implementations, the weighting factors, bias values, and threshold values, or other computational parameters of the neural network, are "taught" or "learned" in a training phase using one or more sets of training data. For example, in some implementations, the parameters are trained using the input data from a training dataset and a gradient descent or backward propagation method so that the output value(s) that the ANN computes are consistent with the examples included in the training dataset. In some embodiments, the parameters are obtained from a back propagation neural network training process.

Any of a variety of neural networks are suitable for use in accordance with the present disclosure. Examples include, but are not limited to, feedforward neural networks, radial basis function networks, recurrent neural networks, residual neural networks, convolutional neural networks, residual convolutional neural networks, and the like, or any combination thereof. In some embodiments, the machine learning makes use of a pre-trained and/or transfer-learned ANN or deep learning architecture. In some implementations, convolutional and/or residual neural networks are used, in accordance with the present disclosure.

For instance, a deep neural network model includes an input layer, a plurality of individually parameterized (*e.g.,* weighted) convolutional layers, and an output scorer. The parameters (*e.g.,* weights) of each of the convolutional layers as well as the input layer contribute to the plurality of parameters (*e.g.,* weights) associated with the deep neural network model. In some embodiments, at least 100 parameters, at least 1000 parameters, at least 2000 parameters or at least 5000 parameters are associated with the deep neural network model. As such, deep neural network models require a computer to be used because they cannot be mentally solved. In other words, given an input to the model, the model output needs to be determined using a computer rather than mentally in such embodiments. *See,* for example, Krizhevsky et al., 2012, "Imagenet classification with deep convolutional neural networks," in Advances in Neural Information Processing Systems 2, Pereira, Burges, Bottou, Weinberger, eds., pp. 1097-1105, Curran Associates, Inc.; Zeiler, 2012 "ADADELTA: an adaptive learning rate method," CoRR, vol. abs/1212.5701; and Rumelhart et al., 1988, "Neurocomputing: Foundations of research," ch. Learning Representations by Back-propagating Errors, pp. 696-699, Cambridge, MA, USA: MIT Press, each of which is hereby incorporated by reference.

Neural network algorithms, including convolutional neural network algorithms, suitable for use as models are disclosed in, for example, Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology, each of which is hereby incorporated by reference. Additional example neural networks suitable for use as models are disclosed in Duda et al., 2001, Pattern Classification, Second Edition, John Wiley & Sons, Inc., New York; and Hastie et al., 2001, The Elements of Statistical Learning, Springer-Verlag, New York, each of which is hereby incorporated by reference in its entirety. Additional example neural networks suitable for use as models are also described in Draghici, 2003, Data Analysis Tools for DNA Microarrays, Chapman & Hall/CRC; and Mount, 2001, Bioinformatics: sequence and genome analysis, Cold Spring Harbor Laboratory Press, Cold Spring Harbor, New York, each of which is hereby incorporated by reference in its entirety.

*Support vector machines.* In some embodiments, the model is a support vector machine (SVM). SVM algorithms suitable for use as models are described in, for example, Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914, each of which is hereby incorporated by reference in its entirety. When used for classification, SVMs separate a given set of binary labeled data with a hyper-plane that is maximally distant from the labeled data. For certain cases in which no linear separation is possible, SVMs work in combination with the technique of 'kernels', which automatically realizes a non-linear mapping to a feature space. The hyper-plane found by the SVM in feature space corresponds, in some instances, to a non-linear decision boundary in the input space. In some embodiments, the plurality of parameters (*e.g.,* weights) associated with the SVM define the hyper-plane. In some embodiments, the hyper-plane is defined by at least 10, at least 20, at least 50, or at least 100 parameters and the SVM model requires a computer to calculate because it cannot be mentally solved.

*Naïve Bayes algorithms.* In some embodiments, the model is a Naive Bayes algorithm. Naive Bayes models suitable for use as models are disclosed, for example, in Ng et al., 2002, "On discriminative vs. generative classifiers: A comparison of logistic regression and naive Bayes," Advances in Neural Information Processing Systems, 14, which is hereby incorporated by reference. A Naive Bayes model is any model in a family of "probabilistic models" based on applying Bayes' theorem with strong (naive) independence assumptions between the features. In some embodiments, they are coupled with Kernel density estimation. See, for example, Hastie et al., 2001, The elements of statistical learning: data mining, inference, and prediction, eds. Tibshirani and Friedman, Springer, New York, which is hereby incorporated by reference.

*Nearest neighbor algorithms.* In some embodiments, a model is a nearest neighbor algorithm. In some implementations, nearest neighbor models are memory-based and include no model to be fit. For nearest neighbors, given a query point $x_0$ (a test subject), the k training points $x_{(r)}$, r, ... , k (here the training subjects) closest in distance to $x_0$ are identified and then the point $x_0$ is classified using the k nearest neighbors. In some embodiments, Euclidean distance in feature space is used to determine distance as $d_{(i)} = \|x_{(i)} - x_{(O)}\|$. Typically, when the nearest neighbor algorithm is used, the abundance data used to compute the linear discriminant is standardized to have mean zero and variance 1. In some embodiments, the nearest neighbor rule is refined to address issues of unequal class priors, differential misclassification costs, and feature selection. Many of these refinements involve some form of weighted voting for the neighbors. For more information on nearest neighbor analysis, *see* Duda, Pattern Classification, Second Edition, 2001, John Wiley & Sons, Inc; and Hastie, 2001, The Elements of Statistical Learning, Springer, New York, each of which is hereby incorporated by reference.

A k-nearest neighbor model is a non-parametric machine learning method in which the input consists of the k closest training examples in feature space. The output is a class membership. An object is classified by a plurality vote of its neighbors, with the object being assigned to the class most common among its k nearest neighbors (k is a positive integer, typically small). If k = 1, then the object is simply assigned to the class of that single nearest neighbor. See, Duda et al., 2001, Pattern Classification, Second Edition, John Wiley & Sons, which is hereby incorporated by reference. In some embodiments, the number of distance calculations needed to solve the k-nearest neighbor model is such that a computer is used to solve the model for a given input because it cannot be mentally performed.

*Random forest, decision tree, and boosted tree algorithms.* In some embodiments, the model is a decision tree. Decision trees suitable for use as models are described generally by Duda, 2001, Pattern Classification, John Wiley & Sons, Inc., New York, pp. 395-396, which is hereby incorporated by reference. Tree-based methods partition the feature space into a set of rectangles, and then fit a model (like a constant) in each one. In some embodiments, the decision tree is random forest regression. For example, one specific algorithm is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York, pp. 396-408 and pp. 411-412, which is hereby incorporated by reference. CART, MART, and C4.5 are described in Hastie et al., 2001, The Elements of Statistical Learning, Springer-Verlag, New York, Chapter 9, which is hereby incorporated by reference in its entirety. Random Forests are described in Breiman, 1999, "Random Forests--Random Features," Technical Report 567, Statistics Department, U.C. Berkeley, September 1999, which is hereby incorporated by reference in its entirety. In some embodiments, the decision tree model includes at least 10, at least 20, at least 50, or at least 100 parameters (*e.g.,* weights and/or decisions) and requires a computer to calculate because it cannot be mentally solved.

*Regression.* In some embodiments, the model uses a regression algorithm. In some embodiments, a regression algorithm is any type of regression. For example, in some embodiments, the regression algorithm is logistic regression. In some embodiments, the regression algorithm is logistic regression with lasso, L2 or elastic net regularization. In some embodiments, those extracted features that have a corresponding regression coefficient that fails to satisfy a threshold value are pruned (removed from) consideration. In some embodiments, a generalization of the logistic regression model that handles multicategory responses is used as the model. Logistic regression algorithms are disclosed in Agresti, An Introduction to Categorical Data Analysis, 1996, Chapter 5, pp. 103-144, John Wiley & Son, New York, which is hereby incorporated by reference. In some embodiments, the model makes use of a regression model disclosed in Hastie et al., 2001, The Elements of Statistical Learning, Springer-Verlag, New York. In some embodiments, the logistic regression model includes at least 10, at least 20, at least 50, at least 100, or at least 1000 parameters (*e.g.,* weights) and requires a computer to calculate because it cannot be mentally solved.

*Linear discriminant analysis algorithms.* In some embodiments, linear discriminant analysis (LDA), normal discriminant analysis (NDA), or discriminant function analysis is a generalization of Fisher's linear discriminant, a method used in statistics, pattern recognition, and machine learning to find a linear combination of features that characterizes or separates two or more classes of objects or events. In some embodiments, the resulting combination is used as the model (linear model) in some embodiments of the present disclosure.

*Mixture model and Hidden Markov model.* In some embodiments, the model is a mixture model, such as that described in McLachlan et al., Bioinformatics 18(3):413-422, 2002. In some embodiments, in particular, those embodiments including a temporal component, the model is a hidden Markov model such as described by Schliep et al., 2003, Bioinformatics 19(1):i255-i263.

*Clustering.* In some embodiments, the model is an unsupervised clustering model. In some embodiments, the model is a supervised clustering model. Clustering algorithms suitable for use as models are described, for example, at pages 211-256 of Duda and Hart, Pattern Classification and Scene Analysis, 1973, John Wiley & Sons, Inc., New York, (hereinafter "Duda 1973") which is hereby incorporated by reference in its entirety. As an illustrative example, in some embodiments, the clustering problem is described as one of finding natural groupings in a dataset. To identify natural groupings, two issues are addressed. First, a way to measure similarity (or dissimilarity) between two samples is determined. This metric (*e.g.,* similarity measure) is used to ensure that the samples in one cluster are more like one another than they are to samples in other clusters. Second, a mechanism for partitioning the data into clusters using the similarity measure is determined. One way to begin a clustering investigation is to define a distance function and to compute the matrix of distances between all pairs of samples in the training set. If distance is a good measure of similarity, then the distance between reference entities in the same cluster is significantly less than the distance between the reference entities in different clusters. However, in some implementations, clustering does not use a distance metric. For example, in some embodiments, a nonmetric similarity function $s(x, x')$ is used to compare two vectors x and x'. In some such embodiments, $s(x, x')$ is a symmetric function whose value is large when x and x' are somehow "similar." Once a method for measuring "similarity" or "dissimilarity" between points in a dataset has been selected, clustering uses a criterion function that measures the clustering quality of any partition of the data. Partitions of the dataset that extremize the criterion function are used to cluster the data. Particular exemplary clustering techniques contemplated for use in the present disclosure include, but are not limited to, hierarchical clustering (agglomerative clustering using a nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering includes unsupervised clustering (*e.g.,* with no preconceived number of clusters and/or no predetermination of cluster assignments).

*Ensembles of models and boosting.* In some embodiments, an ensemble (two or more) of models is used. In some embodiments, a boosting technique such as AdaBoost is used in conjunction with many other types of learning algorithms to improve the performance of the model. In this approach, the output of any of the models disclosed herein, or their equivalents, is combined into a weighted sum that represents the final output of the boosted model. In some embodiments, the plurality of outputs from the models is combined using any measure of central tendency known in the art, including but not limited to a mean, median, mode, a weighted mean, weighted median, weighted mode, etc. In some embodiments, the plurality of outputs is combined using a voting method. In some embodiments, a respective model in the ensemble of models is weighted or unweighted.

As used herein, the term "parameter" refers to any coefficient or, similarly, any value of an internal or external element (*e.g.,* a weight and/or a hyperparameter) in an algorithm, model, regressor, and/or classifier that can affect (*e.g.,* modify, tailor, and/or adjust) one or more inputs, outputs, and/or functions in the algorithm, model, regressor and/or classifier. For example, in some embodiments, a parameter refers to any coefficient, weight, and/or hyperparameter that can be used to control, modify, tailor, and/or adjust the behavior, learning, and/or performance of an algorithm, model, regressor, and/or classifier. In some instances, a parameter is used to increase or decrease the influence of an input (*e.g.,* a feature) to an algorithm, model, regressor, and/or classifier. As a nonlimiting example, in some embodiments, a parameter is used to increase or decrease the influence of a node (*e.g.,* of a neural network), where the node includes one or more activation functions. Assignment of parameters to specific inputs, outputs, and/or functions is not limited to any one paradigm for a given algorithm, model, regressor, and/or classifier but can be used in any suitable algorithm, model, regressor, and/or classifier architecture for a desired performance. In some embodiments, a parameter has a fixed value. In some embodiments, a value of a parameter is manually and/or automatically adjustable. In some embodiments, a value of a parameter is modified by a validation and/or training process for an algorithm, model, regressor, and/or classifier (*e.g.,* by error minimization and/or backpropagation methods). In some embodiments, an algorithm, model, regressor, and/or classifier of the present disclosure includes a plurality of parameters. In some embodiments, the plurality of parameters is n parameters, where: $n \geq 2$; $n \geq 5$; $n \geq 10$; $n \geq 25$; $n \geq 40$; $n \geq 50$; $n \geq 75$; $n \geq 100$; $n \geq 125$; $n \geq 150$; $n \geq 200$; $n \geq 225$; $n \geq 250$; $n \geq 350$; $n \geq 500$; $n > 600$; $n \geq 750$; $n \geq 1,000$; $n > 2,000$; $n \geq 4,000$; $n \geq 5,000$; $n \geq 7,500$; $n \geq 10,000$; $n \geq 20,000$; $n \geq 40,000$; $n \geq 75,000$; $n \geq 100,000$; $n \geq 200,000$; $n \geq 500,000$, $n \geq 1 \times 10^6$, $n \geq 5 \times 10^6$, or $n \geq 1 \times 10^7$. As such, the algorithms, models, regressors, and/or classifiers of the present disclosure cannot be mentally performed. In some embodiments n is between 10,000 and $1 \times 10^7$, between 100,000 and $5 \times 10^6$, or between 500,000 and $1 \times 10^6$. In some embodiments, the algorithms, models, regressors, and/or classifier of the present disclosure operate in a k-dimensional space, where k is a positive integer of 5 or greater (*e.g.,* 5, 6, 7, 8, 9, 10, etc.). As such, the algorithms, models, regressors, and/or classifiers of the present disclosure cannot be mentally performed.

As used herein, the term "untrained model" (*e.g.,* "untrained classifier" and/or "untrained neural network") refers to a machine learning model or algorithm, such as a classifier or a neural network, that has not been trained on a target dataset. In some embodiments, "training a model" (*e.g.,* "training a neural network") refers to the process of training an untrained or partially trained model (*e.g.,* "an untrained or partially trained neural network"). Moreover, it will be appreciated that the term "untrained model" does not exclude the possibility that transfer learning techniques are used in such training of the untrained or partially trained model. For instance, Fernandes et al., 2017, "Transfer Learning with Partial Observability Applied to Cervical Cancer Screening," Pattern Recognition and Image Analysis: 8th Iberian Conference Proceedings, 243-250, which is hereby incorporated by reference, provides non-limiting examples of such transfer learning. In instances where transfer learning is used, the untrained model described above is provided with additional data over and beyond that of the primary training dataset. Typically, this additional data is in the form of parameters (*e.g.,* coefficients, weights, and/or hyperparameters) that were learned from another, auxiliary training dataset. Moreover, while a description of a single auxiliary training dataset has been disclosed, it will be appreciated that there is no limit on the number of auxiliary training datasets that can be used to complement the primary training dataset in training the untrained model in the present disclosure. For instance, in some embodiments, two or more auxiliary training datasets, three or more auxiliary training datasets, four or more auxiliary training datasets or five or more auxiliary training datasets are used to complement the primary training dataset through transfer learning, where each such auxiliary dataset is different than the primary training dataset. Any manner of transfer learning is used, in some such embodiments. For instance, consider the case where there is a first auxiliary training dataset and a second auxiliary training dataset in addition to the primary training dataset. In such a case, the parameters learned from the first auxiliary training dataset (by application of a first model to the first auxiliary training dataset) are applied to the second auxiliary training dataset using transfer learning techniques (*e.g.,* a second model that is the same or different from the first model), which in turn results in a trained intermediate model whose parameters are then applied to the primary training dataset and this, in conjunction with the primary training dataset itself, is applied to the untrained model. Alternatively, in another example embodiment, a first set of parameters learned from the first auxiliary training dataset (by application of a first model to the first auxiliary training dataset) and a second set of parameters learned from the second auxiliary training dataset (by application of a second model that is the same or different from the first model to the second auxiliary training dataset) are each individually applied to a separate instance of the primary training dataset (*e.g.,* by separate independent matrix multiplications) and both such applications of the parameters to separate instances of the primary training dataset in conjunction with the primary training dataset itself (or some reduced form of the primary training dataset such as principal components or regression coefficients learned from the primary training set) are then applied to the untrained model in order to train the untrained model.

Several aspects are described herein with reference to example applications for illustration. It should be understood that numerous specific details, relationships, and methods are set forth to provide a full understanding of the features described herein. One having ordinary skill in the relevant art, however, will readily recognize that the features described herein can be practiced without one or more of the specific details or with other methods. The features described herein are not limited by the illustrated ordering of acts or events, as some acts can occur in different orders and/or concurrently with other acts or events. Furthermore, not all illustrated acts or events are required to implement a methodology in accordance with the features described herein.

Reference is made herein to embodiments, examples of which are illustrated in the accompanying drawings. In the present disclosure, numerous specific details are set forth in order to provide a thorough understanding of the present disclosure. However, it will be apparent to one of ordinary skill in the art that the present disclosure can be practiced without these specific details. In other instances, well-known methods, procedures, components, circuits, and networks have not been described in detail so as not to unnecessarily obscure aspects of the embodiments.

*Example System Embodiments.*

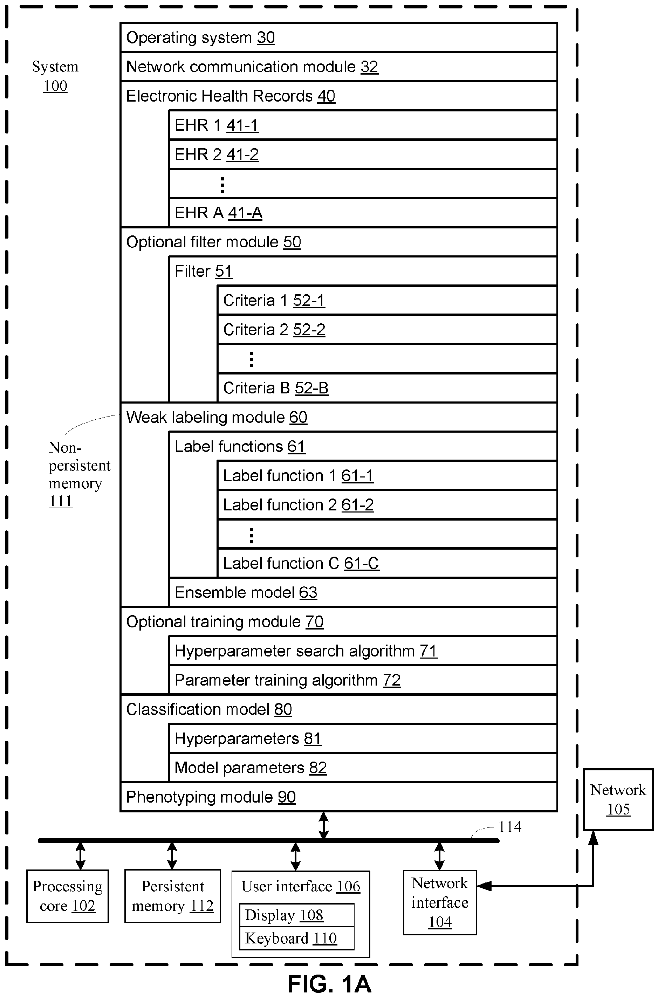

Now that an overview of some aspects of the present disclosure and some definitions used in the present disclosure have been provided, details of an exemplary system for training a model for phenotyping subjects with respect to a medical condition (*e.g.,* a cardiovascular condition, a neurological condition, a cancer, etc.) are now described in conjunction with FIGS. 1A-1D. FIGS. 1A-1D collectively illustrate the topology of an example system for training a model for phenotyping subjects with respect to a medical condition, in accordance with some embodiments of the present disclosure. Advantageously, the example system illustrated in FIGS. 1A-1D improves upon conventional methods for training a model for phenotyping subjects with respect to a medical condition by aggregating label functions that interrogate multimodal EHR data to generate weak labels for model training.

FIGS. 1A-1D collectively illustrate a block diagram illustrating a system in accordance with some implementations. The device 100 in some implementations includes one or more processing units CPU(s) 102 (also referred to as processors), one or more network interfaces 104, a user interface 106, *e.g.,* including a display 108 and/or an input 110 (*e.g.,* a mouse, touchpad, keyboard, *etc.*)*,* a non-persistent memory 111, a persistent memory 112, and one or more communication buses 114 for interconnecting these components. The one or more communication buses 114 optionally include circuitry (sometimes called a chipset) that interconnects and controls communications between system components. The non-persistent memory 111 typically includes high-speed random-access memory, such as DRAM, SRAM, DDR RAM, ROM, EEPROM, flash memory, whereas the persistent memory 112 typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices. The persistent memory 112 optionally includes one or more storage devices remotely located from the CPU(s) 102. The persistent memory 112, and the non-volatile memory device(s) within the non-persistent memory 112, comprise non-transitory computer readable storage medium. In some implementations, the non-persistent memory 111 or alternatively the non-transitory computer readable storage medium stores the following programs, modules and data structures, or a subset thereof, sometimes in conjunction with the persistent memory 112:

- an operating system 30, which includes procedures for handling various basic system services and for performing hardware dependent tasks;
- a network communication module (or instructions) 30 for connecting the system 100 with other devices and/or a communication network 105;
- an electronic health record store 40 that stores electronic health records 41 (*e.g.,* electronic health records 41-1, ... 41-A) for subjects, which include one or more data types, such as structured clinical data 42, unstructured clinical data 43, prescribed medications 44, medical procedures 45, and laboratory results 46, as well as optional weak labels 47 and/or classifications 48 generated using the methods described herein;
- an optional filter module 50 for building and/or applying filter 51 (*e.g.,* criteria 52-1, ... 52-B) to electronic health records 41 as an optional step for filtering out electronic health records 41 that are not associated with the medical condition of interest prior to assigning weak labels, thereby enriching the set of electronic records for those associated with the medical condition of interest, by interrogating the electronic health records 41 against inclusion and/or exclusion criterion 52 related to characteristics 53;
- weak labeling module 60 for building and/or applying label functions 61 (*e.g.,* label functions 61-1,... 61-C) to electronic health records 41 to generate labels for training a classification model 80 by interrogating the electronic health records 41 against criterion 62 (*e.g.,* criterion 62-1-1, ... 62-1-G for label function 1 61-1) and combining the output of individual label functions 61 using an ensemble model 63;
- an optional training module 70 for training a classification model 80 to predict instances of a medical condition using information (*e.g.,* one or more data types such as structured clinical data 42, unstructured clinical data 43, prescribed medications 44, medical procedures 45, and laboratory results 46) from an electronic health record (*e.g.,* electronic health records 41) and weak labels 47 as variables, the training module optionally includes a hyperparameter search algorithm 71 and a parameter optimization algorithm 72;
- an optional classifications model 80 (*e.g.*, as trained using optional training module 70) having a plurality of hyperparameters 81 and model parameters 82; and
- an optional phenotyping module 90 for predicting a phenotype for a medical condition by inputting information from an electronic medical record (*e.g.,* from an EHR store 40) into a classification model 80.

Although FIGS. 1A-1D depict various components of a "system 100," the figures are intended more as a functional description of the various features that, in some embodiments, are present in computer systems than as a structural schematic of the implementations described herein. In practice, in some implementations, items shown separately are combined and some items are separated. Moreover, although FIGS. 1A-1D depict certain data and modules in non-persistent memory 111, in some embodiments, some or all of these data and modules are in persistent memory 112. For example, in various implementations, one or more of the above identified elements are stored in one or more of the previously mentioned memory devices and correspond to a set of instructions for performing a function described above. The above identified modules, data, or programs (*e.g.,* sets of instructions) need not be implemented as separate software programs, procedures, datasets, or modules, and thus various subsets of these modules and data, in some embodiments, are combined or otherwise re-arranged in various implementations.

In some implementations, the non-persistent memory 111 optionally stores a subset of the modules and data structures identified above. Furthermore, in some embodiments, the memory stores additional modules and data structures not described above. In some embodiments, one or more of the above-identified elements is stored in a computer system, other than that of system 100, that is addressable by system 100 so that system 100 retrieves all or a portion of such data when needed.

For purposes of illustration in FIG. 1A, system 100 is represented as a single computer that includes all of the functionality for training a model for phenotyping subjects with respect to a medical condition. However, while a single machine is illustrated, the term "system" shall also be taken to include any collection of machines that individually or jointly execute a set (or multiple sets) of instructions to perform any one or more of the methodologies discussed herein.

For example, in some embodiments, system 100 includes one or more computers. In some embodiments, the functionality for training a model for phenotyping subjects with respect to a medical condition is spread across any number of networked computers and/or resides on each of several networked computers and/or is hosted on one or more virtual machines at a remote location accessible across the communications network 105. For example, different portions of the various modules and data stores illustrated in FIGS. 1A-1D can be stored and/or executed on the various instances of a processing device and/or processing server/database in a distributed computing environment.

In some embodiments, the system operates in the capacity of a server or a client machine in a client-server network environment, as a peer machine in a peer-to-peer (or distributed) network environment, or as a server or a client machine in a cloud computing infrastructure or environment. In some embodiments, the system is a personal computer (PC), a tablet PC, a set-top box (STB), a Personal Digital Assistant (PDA), a cellular telephone, a web appliance, a server, a network router, a switch or bridge, or any machine capable of executing a set of instructions (sequential or otherwise) that specify actions to be taken by that machine.

In another implementation, the system comprises a virtual machine that includes a module for executing instructions for performing any one or more of the methodologies disclosed herein. In computing, a virtual machine (VM) is an emulation of a computer system that is based on computer architectures and provides functionality of a physical computer. Some such implementations involve specialized hardware, software, or a combination of hardware and software.

One of skill in the art will appreciate that any of a wide array of different computer topologies are used for the application and all such topologies are within the scope of the present disclosure.

In some embodiments, system 100 further includes a clinical trials module that evaluates one or more electronic health record 41 with a classification 48 indicating the presence of a medical condition to determine whether a subject is eligible for inclusion in a clinical trial for treatment of the medical condition, *e.g.,* a clinical trial that is currently recruiting patients, a clinical trial that has not yet begun recruiting patients, and/or an ongoing clinical trial that may recruit additional patients in the future. In some embodiments, a clinical trial module evaluates one or more electronic health record 41 with a classification 48 indicating the presence of a medical condition to determine whether the results of a clinical trial are relevant for a subject, e.g., the results of an ongoing clinical trial and/or the results of a completed clinical trial. For instance, in some embodiments, system 100 queries a database, *e.g.,* a look-up-table ("LUT") of clinical trials, *e.g.,* active and/or completed clinical trials, related to the medical condition and compares information in the one or more electronic health record 41 with inclusion criteria for the clinical trials, stored in the database, to identify subjects meeting or nearly meeting clinical trial inclusion criteria. In some embodiments, a record of matching clinical trials, *e.g.,* those clinical trials that a subject is or is nearly eligible for, and/or those trials that a subject would have been or nearly would have been eligible for, are further stored in system 100 and/or subject data store 120.

In some embodiments, system 100 further includes a reporting module that evaluates one or more classifications 48 and generates a report for a medical practitioner or medical organization (*e.g.,* a hospital, clinic, healthcare provider, or medical insurance company) identifying one or more undiagnosed subjects that likely have a medical condition. In some embodiments, the report includes one or more clinical recommendation for further evaluation and/or treatment of an identified subject. In some embodiments, the report includes one or more treatment recommendations for an identified subject. In some embodiments, the report includes one or more clinical trials that may be relevant for an identified patient, *e.g.,* as identified using a clinical trial module as described above.

In some embodiments, system 100 includes a model training module that includes instructions for training one or more untrained or partially trained models based on feature data from a training dataset. In some embodiments, system 100 also includes a database of training data (*e.g.,* a plurality of training electronic health records) for use in training the one or more models. In other embodiments, the model training module accesses a remote storage device hosting training data. In some embodiments, the training data includes a set of training features, including but not limited to, multimodal data extracted from an electronic health record (*e.g.,* structured clinical data 42, unstructured clinical data 43, medications, 44, medical procedures, 45, and/or laboratory results 46) as illustrated in FIGS. 1A-1B.

In some embodiments, each of the one or more data stores and/or modules are communicatively coupled to a data bus 114 to transfer data between each data store or module for processing and/or storage. In some alternative embodiments, each of the one or more data stores and/or modules are communicatively coupled to each other for independent communication without sharing the data bus.

Further details on systems and exemplary embodiments of modules and feature collections are discussed in PCT Application PCT/US19/69149, titled "A METHOD AND PROCESS FOR PREDICTING AND ANALYZING PATIENT COHORT RESPONSE, PROGRESSION, AND SURVIVAL," filed December 31, 2019, the content of which is incorporated herein by reference, in its entirety, for all purposes.

### Example Embodiments

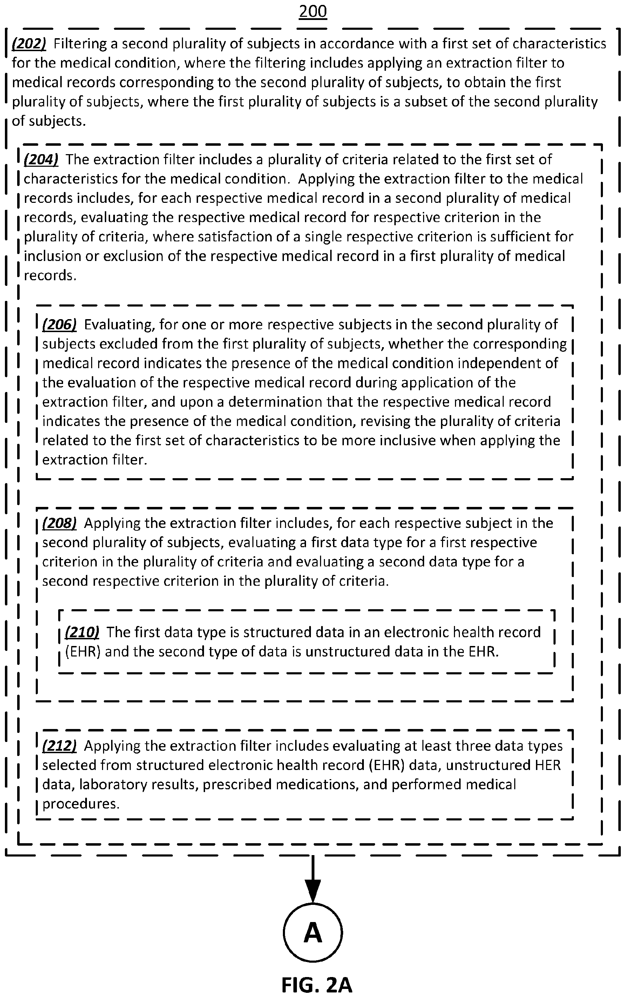

Now that details of a system 100 for training a model for phenotyping subjects with respect to a medical condition have been disclosed, details regarding processes and features of the system, in accordance with various embodiments of the present disclosure, are provided below. Specifically, example processes are described below with reference to FIGS. 2A-2H. In some embodiments, such processes and features of the system are carried out by modules 50, 60, 70, and/or 90, as illustrated in FIGS. 1A-1D. Referring to these methods, in some embodiments, the systems described herein (*e.g.,* system 100) include instructions for training a model for phenotyping subjects with respect to a medical condition that are improved compared to conventional methods for training a model for phenotyping subjects with respect to a medical condition.

In one aspect, the disclosure provides a method 200 for training a model for phenotyping subjects with respect to a medical condition. In some embodiments, all or a portion of the method is performed at a computer system including at least one processor and a memory storing at least one program having instructions for execution by the at least one processor.

In some embodiments, one or more electronic health records are preprocessed prior to analysis using the methods and systems described herein. For example, in some embodiments, an electronic health record (EHR) is obtained from an image of a health record, such as a scanned image of a physical (*e.g.,* paper and/or handwritten) health record document. In some embodiments, the EHR is obtained in an image file format, such as a PDF. In some such embodiments, the method includes analyzing the EHR, using a text recognition process, to convert the image to computer readable text. For instance, in some embodiments, the method includes, prior to the receiving the first electronic health record, receiving one or more images of text corresponding to the first electronic health record and converting the one or more images of text to a computer-readable text format using text recognition. In some embodiments, the text recognition is optical character recognition (OCR). In some embodiments, the OCR converts the image to raw text.

Methods for text recognition are well known in the art, including but not limited to sliding window classification, Connected Component Analysis (CCA), bounding box regression-based methods, segmentation-based methods, and/or combinations thereof. For example, sliding window classification utilizes convolutional classifiers to detect characters in an image using a multi-scale sliding window. CCA-based methods operate by segmenting pixels in an image having consistent local characteristics such as color, edge, texture, and/or stroke width into characters. Alternatively, or additionally, text-line based methods operate by initially identifying lines of text and partitioning the identified text lines into smaller components such as words and letters. Generally, bounding box and segmentation-based methods operate by detecting text at the single-word level, for instance, using bounding boxes that isolate regions of text from the local background, with optional filtering, cleaning, and recognition post-processing. See, for example, Keerthana et al., 2020, "Text Detection and Recognition: A Review," IRJET Vol 7(8), 2156-2169, which is hereby incorporated herein by reference in its entirety.

In some embodiments, the subdividing is performed by identifying one or more regular expressions in a corresponding section. Generally, regular expressions refer to a sequence of characters that specifies a search pattern in text. For instance, in an example embodiment, the subdividing subdivides a respective section into a plurality of sentences using language pattern recognition to search for the ends of sentences, as specified by a corresponding regular expression.

For example, in some embodiments, regular expression filtering is used to split the first EHR into a plurality of sentences. An example of regular expression syntax that can be used to split raw text into sentences is "r'\s{2,}|(?<!\w\.\w.)(?<![A-Z][a-z]\.)(?<=\.|\?)\s'." In some embodiments, regular expression filtering is used to subdivide each respective section, in the plurality of sections, into a corresponding plurality of text spans on either side of one or more punctuation marks. For instance, in some implementations, text spans are obtained by subdividing a string of text (*e.g.,* a section) at a period, a question mark, an exclamation point, a comma, a colon, a semicolon, a dash, a hyphen, a bracket, a parenthesis, an apostrophe, a quotation mark, and/or an ellipsis. In some embodiments, particular punctuation marks are excluded from being identified as text span (*e.g.,* sentence) boundaries. For example, in some implementations, the period at the end of the abbreviation 'Dr.' for doctor is excluded (*e.g.,* "dr. XX"). Examples of regular expression syntax useful for excluding identification of particular punctuation as text span (*e.g.,* sentence) boundaries are found, for example, in Section 3.2.2. of Rokach L. et al., Information Retrieval Journal, 11(6):499-538 (2008), the content of which is incorporated herein by reference, in its entirety, for all purposes.

In some embodiments, relevant portions of an EHR are extracted and/or indexed prior to data analysis. For example, in some embodiments, structured diagnosis codes from a plurality of EHR are extracted into a table or other data structure, to facilitate identification of diagnosis codes indicating the presence or absence of a target medical condition.

In some embodiments, an EHR is broken into separate episodes. For example, an EHR can be broken into a first episode related to the diagnosis and/or treatment of a first medical condition and a second episode related to the diagnosis and/or treatment of a second medical condition. For example, a first episode (*e.g.,* relating to a PH diagnosis and/or treatment) may span more than one clinical visit and/or intervention, *e.g.,* including one or more diagnostic clinical visits, one or more therapeutic visits, one or more follow-up visits, *etc.,* all relating to a first medical condition (*e.g.,* PH). Likewise, a second episode (*e.g.,* relating to a cancer diagnosis and/or treatment) may span more than one clinical visit and/or intervention, *e.g.,* including one or more diagnostic clinical visits, one or more therapeutic visits, one or more follow-up visits, *etc.,* all relating to a second medical condition (*e.g.,* cancer). Different episodes within a single EHR may be temporally overlapping or not.

In some embodiments, clinical notes for an EHR are partitioned into separate episodes. For example, in some embodiments, an encounter can be defined as an interaction between a patient and a healthcare provider that results in the logging of clinical notes into an EHR system. In some embodiments, an episode includes a cluster of encounters representing a single hospital stay. That is, a single hospital stay can be logged as multiple encounters. In Some embodiments, episode boundaries are determined using one-dimensional clustering (*e.g.,* kernel density estimation (KDE)) on an encounter date. In some embodiments, notes between boundaries are aggregated together.

In some embodiments, clinical notes are segmented in order to identify separate episodes. On example method for segmenting clinical notes includes splitting unstructured clinical data by tokenizing unstructured clinical data to obtain a plurality of tokens and segmenting the plurality of tokens to obtain a plurality of segments, *e.g.,* where each respective segment in the plurality of segments has approximately a same number of tokens. In some embodiments, relevant segments can be further enriched by ranking respective segments in the plurality of segments based on values of tokens within each respective segment, and removing one or more respective segments from the plurality of segments based on the ranking, thereby generating the corresponding plurality of snippets for an episodic record.

In some embodiments, the phenotyping process begins with exploratory data analysis (EDA), optionally supplemented by discussion with clinical domain experts and literature review of relevant clinical research, informatics or technical papers, and/or clinical guidelines for the target medical condition. In some embodiments, exploratory data analysis includes characterizing all relevant structured data elements in a dataset, for example, diagnosis codes, procedures, medications, orders, labs, measurements, imaging studies, devices, *etc.* In some embodiments, EDA also includes characterizing data elements with respect to how they pertain to relevant criteria for diagnosing the target medical condition and/ or exploring relationships between these data elements, such as identifying whether gaps exist such as a discrepancy between patients with relevant diagnosis codes as compared to gold standard diagnostic metrics.

In some embodiments, the method includes use of a filter to extract a subpopulation of medical records of interest, to generate effective, unbiased samples for chart review and phenotype development. In some embodiments, the filter is iteratively developed, *e.g.,* based on domain expertise from clinicians, literature review of any and all codes or criteria used in papers, and/or the findings of our initial data exploration. In some embodiments, this filter includes the union of all potentially relevant signals for the target medical condition. In some embodiments, this filter includes substring matches of clinical notes to maximize the coverage of the medical condition in the starting plurality of EHRs.

As an example, consider phenotyping EHRs for pulmonary hypertension (PH). Because the prevalence of PH in the general public can be estimated at around 1%, one goal of the filtering is to shrink down the relevant PH population from the larger general population to a subpopulation of interest with a higher prevalence, *e.g.,* greater than 2.5%, greater than 5%, greater than 10%, etc., without sacrificing sensitivity by removing any significant proportion of the diseased population. Returning to our EHR representation analogy described above with reference to Figure 4, this subpopulation of interest is represented by the dotted circle created by the extraction filter in Figure 4G, with few or no missed cases of patients with true disease outside the circle. This higher prevalence of PH in the selected subpopulation improves the random, unbiased sampling of this population of interest, maintaining enough true positive patients with disease, even in a random sample of 100, to generate fairly reliable metrics from chart reviews and manual abstraction. Otherwise, random sampling in the general population would be expected to yield only 1 or 2 (or no) patients with true disease out of a random sampling of 100 EHRs, therefore requiring either an impractically large number of chart reviews (likely thousands) or a biased sample which could not be used across multiple stages and iterations of phenotype development.

In some embodiments, the same sets of unbiased samples and chart review results (ground truth labels) from this extracted subpopulation of interest can be used as training, validation, and test sets across multiple different iterations of phenotypes, including structured, post-signal aggregation, and NLP model-generated phenotypes, and allows for effective comparison between different versions and different types of models. In contrast, a targeted, biased sample would be relevant only for a specific phenotype, such as choosing a random sample of positive patients from a structured phenotype and could not be used to evaluate future versions of the phenotype because the sampling was anchored on the structured phenotype only. These targeted samples are useful to closely evaluate a specific phenotype, such as for reviewing a final phenotype in each stage of the process, providing greater confidence in the PPV metric, and can be representative of how effective it would be for the downstream task of labeling ECGs. However, for development, it quickly becomes resource-intensive to evaluate every iteration of a phenotype, while metrics cannot be compared across iterations or types, such as when comparing structured vs NLP.

The post-filter random samples can be used throughout the phenotype development pipeline, with the training set being used for structured phenotype development, iterative improvement including false positive/negative review, and building labeling functions and ML models, while the validation set remains separate and unseen, used only for evaluation purposes.

Accordingly, referring to block 202, in some embodiments, the method includes filtering a second plurality of subjects in accordance with a first set of characteristics for the medical condition, where the filtering comprises applying an extraction filter to medical records corresponding to the second plurality of subjects, to obtain the first plurality of subjects, wherein the first plurality of subjects is a subset of the second plurality of subjects. For example, as illustrated in Figure 3, the EHR records 302 are filtered (354) to remove filtered records 312 and pass training records 308 and validation records 310 along to the next step of the process.

In some embodiments, the medical records (e.g., EHR) used for the methods described herein are first split to form at least a training subset and a validation subset. In some embodiments, the plurality of medical records are also split to include a test subset. An example of this splitting is to assign 80% of the medical records to a training set, 10% of the medical records to a validation set, and 10% of the medical records to a test set. However, other splits of the medical records can be used. For example, as illustrated in Figure 3, the EHR records 302 are split into a training subset 304 and a validation subset 306 prior to the filter step (354).

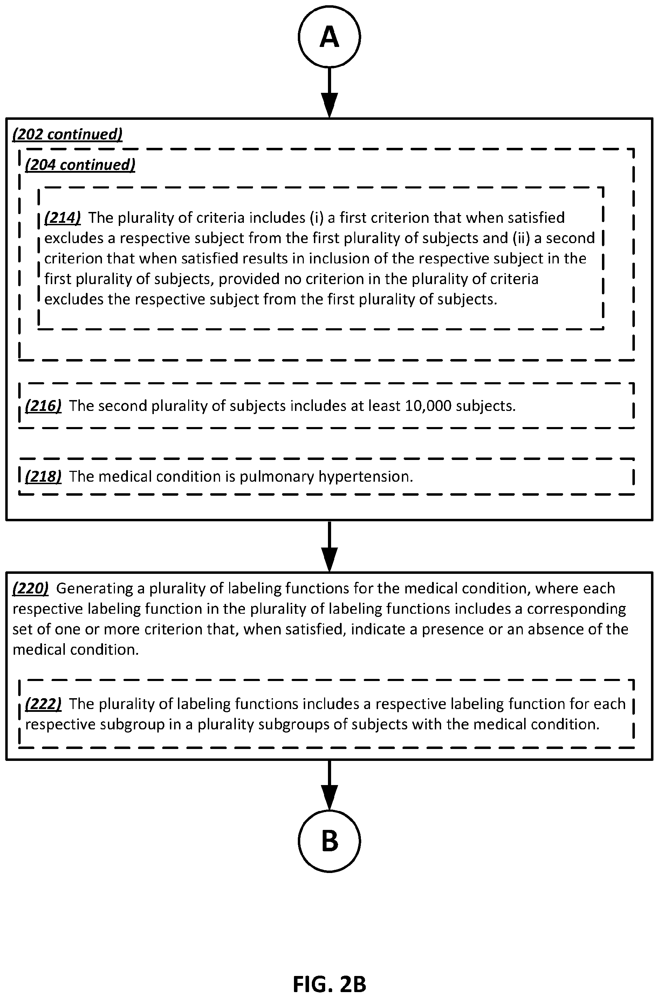

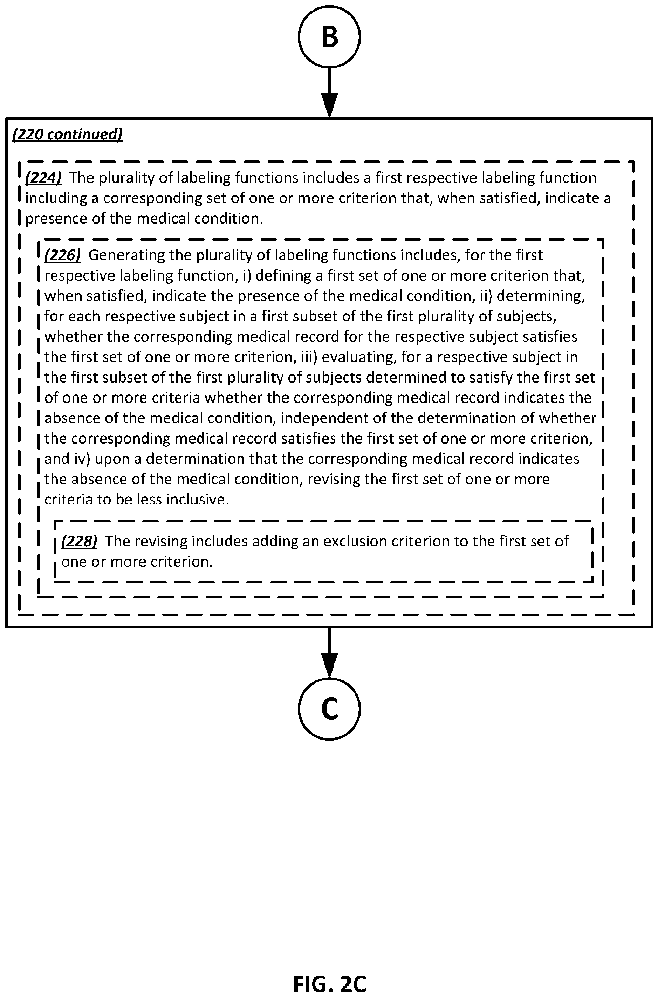

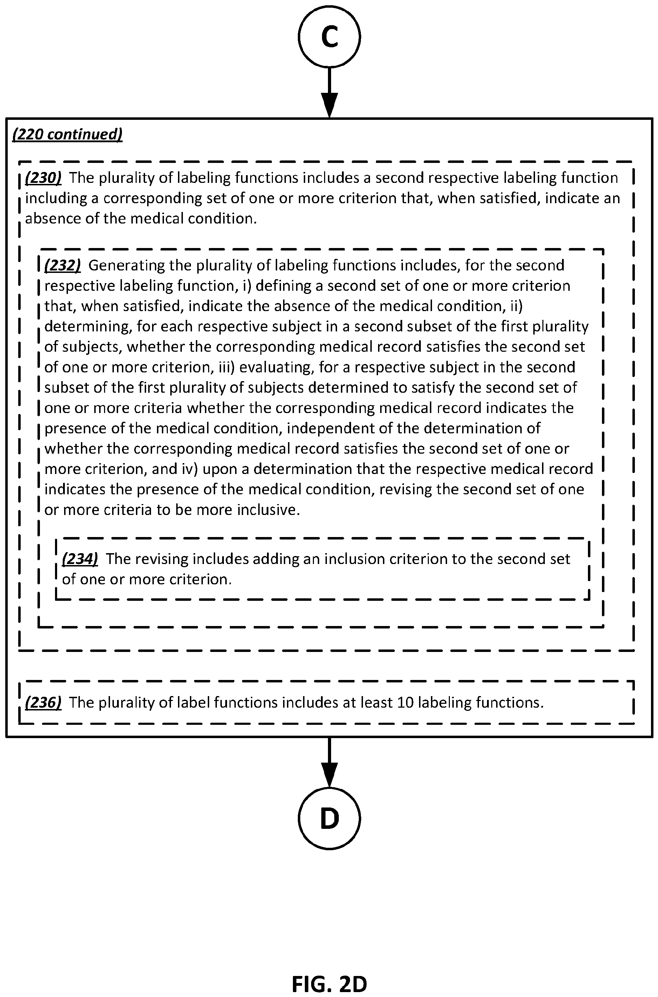

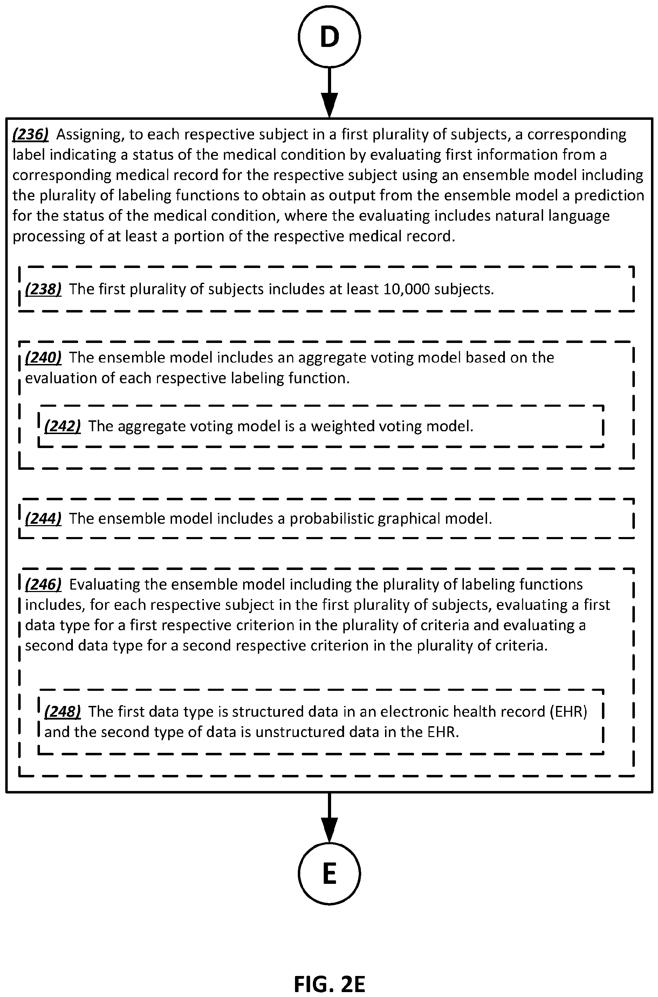

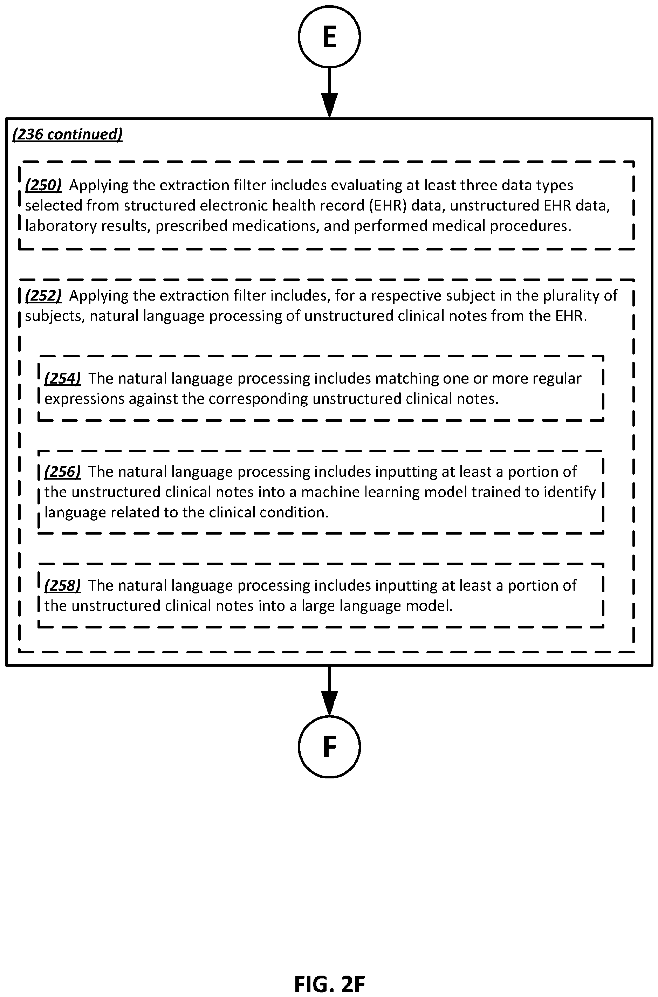

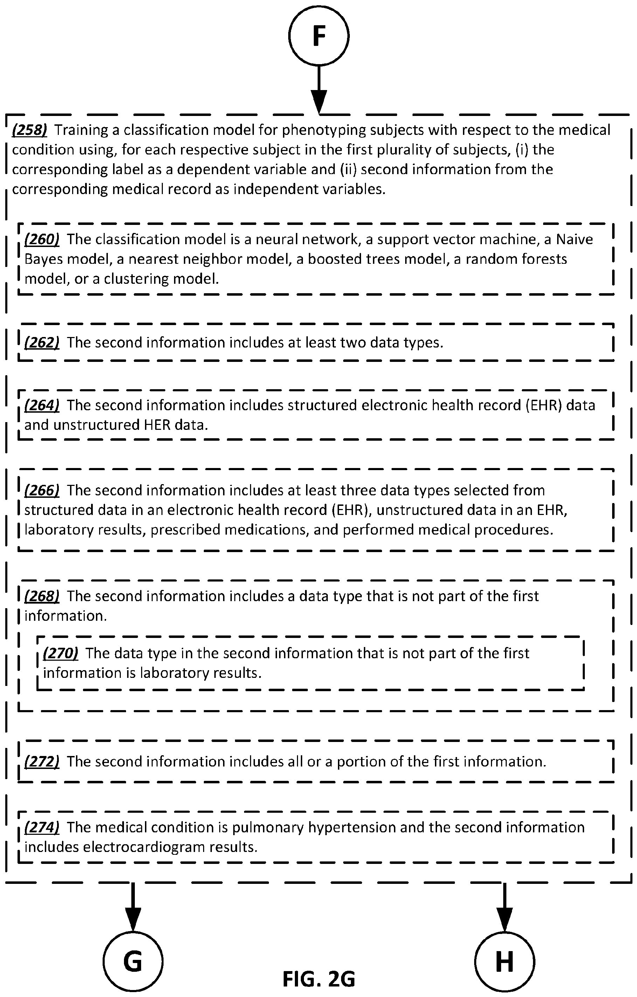

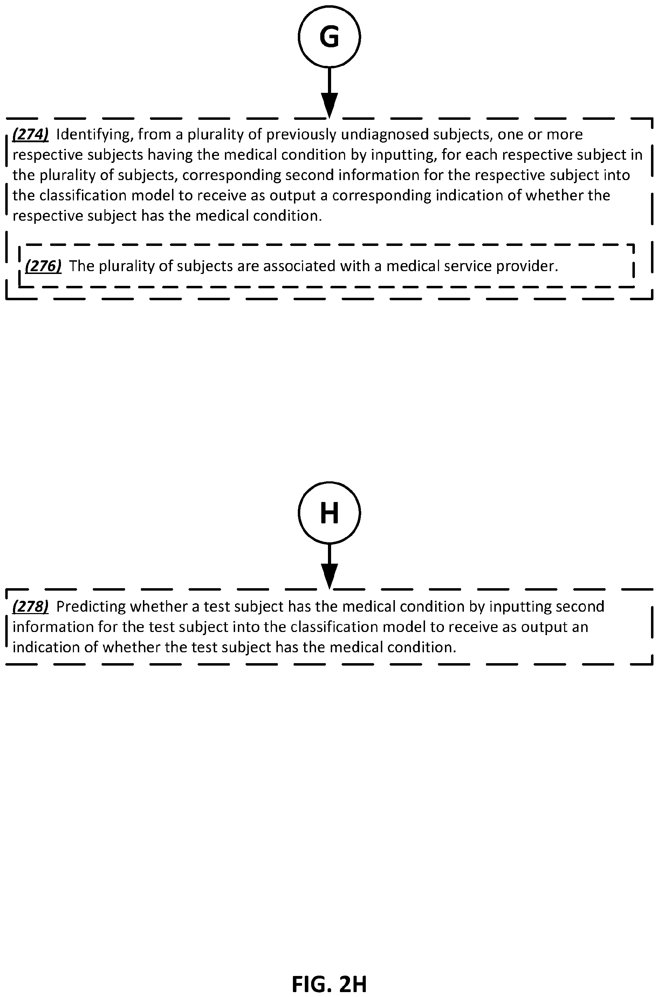

In some embodiments, the medical records used for the methods described herein are not split into training, validation, and/or test subsets prior to filtering. In some embodiments, medical records are split into training, validation, and/or test subsets after the initial filtering, e.g., to ensure a desired ratio of split records. That is, if the medical records are split x : y : z (training : validation : test) prior to an initial filtering step, e.g., filtering (202) in Figure 2 or the filter step (354) in Figure 3, and the filtering removes a higher percentage of records from one set, the split will no longer be x : y : z. With reference to the example workflow in Figure 3, in some embodiments, the EHR records 302 are filtered (354) before being split into training records 308 and validation records 310 (e.g., the prefiltered training record subset 304 and validation record subset 306 are not formed).

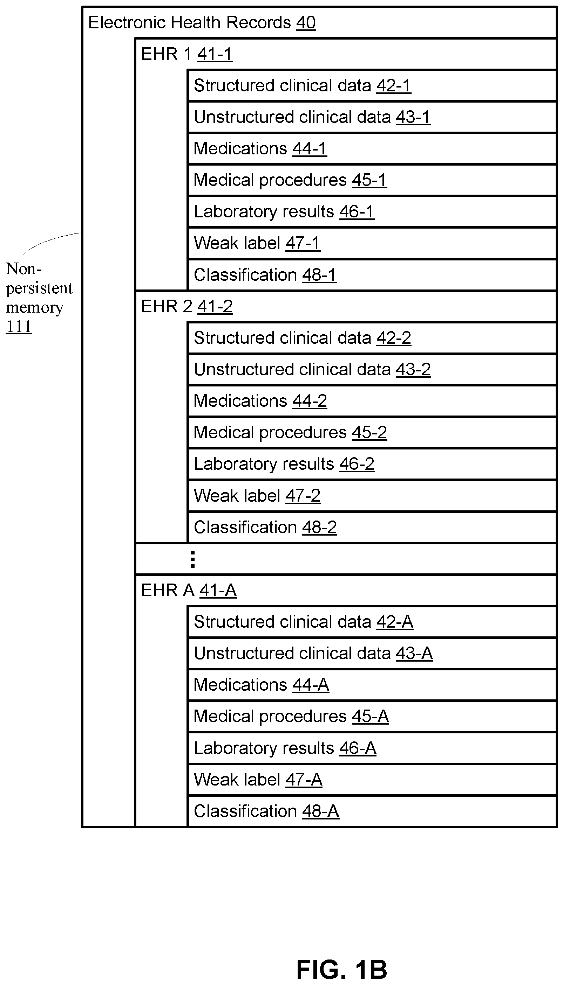

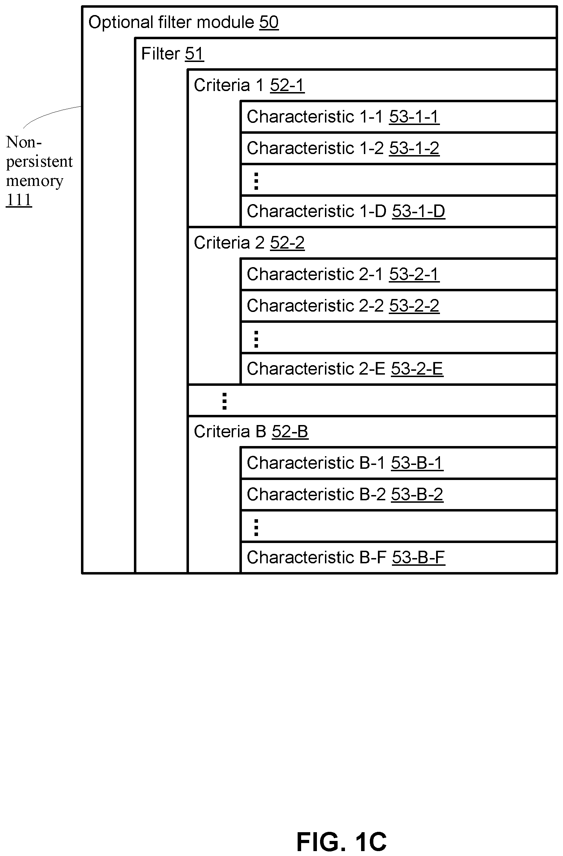

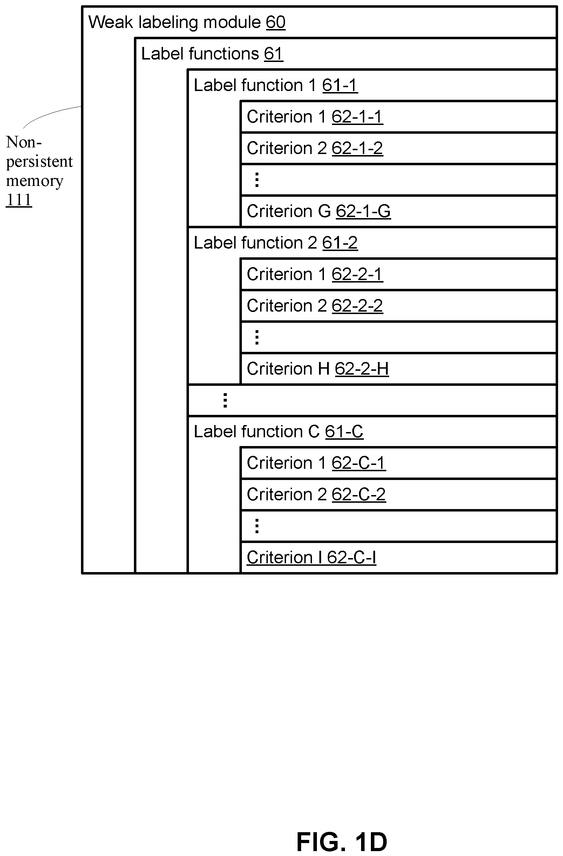

Referring to block 204, in some embodiments, the extraction filter includes a plurality of criteria (e.g., criteria 52 as illustrated in Figure 1C) related to the first set of characteristics for the medical condition (e.g., characteristics 53 as illustrated in Figure 1C). Applying the extraction filter to the plurality of medical records (e.g., medical records 41 in Figure 1, EHRs 302 in Figure 3, and/or training subplurality 304 and validation subplurality 306 in figure 3) includes, for each respective medical record 41 in the plurality of medical records, evaluating the respective medical record for respective criterion 52 in the plurality of criteria, where satisfaction of a single respective criterion is sufficient for inclusion or exclusion of the respective medical record in the subplurality of medical records (e.g., subplurality of training records 308 or subplurality of validation records 310 in figure 3).

In some embodiments, filtered medical records, e.g., either or both records that pass through the filter and records that are removed by the filter, are independently reviewed to assess the performance of the filtering step. For example, referring to Figure 3, in some embodiments, a sample of medical records 41 in the training subplurality of medical records 308 are independently reviewed (356) to determine whether records 41 that are unrelated to PH are passing through the filter 51. In some embodiments, where records 41 that have passed through the filtering step (354) are identified as unrelated to PH, the filters are refined (352) so as to exclude such records during filtering. For example, when the target medical disorder is pulmonary hypertension (commonly abbreviated as PH), a mention of "Ph.D." in a medical record might satisfy a criteria 52 such that the record is allowed to pass through the filter. By identifying such record in the independent review, the filter 51 can be modified to exclude "Ph.D." from meeting the inclusion criteria. Similarly, referring again to Figure 3, in some embodiments, a sample of medical records 41 in the filtered subplurality of medical records 312 are independently reviewed (358) to determine whether records 41 that are related to PH are being filtered by the filter 51. In some embodiments, where records 41 that have been filtered during the filtering step (354) are identified as related to PH, the filters are refined (352) so as to include such records during filtering. For example, when the target medical disorder is PH and a medical record includes a right heart catheterization (RHC) mean pulmonary artery pressure of greater than 20 mm Hg but does not include mention of PH sufficient to satisfy any criteria 52, the filter 51 can be modified to include a criteria 52 satisfied by a mean pulmonary artery pressure of greater than 20 mm Hg.

In some embodiments, this review is performed on de-identified data which can include, for example, unstructured clinical notes and/or structured data fields from the EHRs. In some embodiments, all chart reviews are blinded so that reviewers do not know the phenotype or model label and seek only to ascertain clinical ground truth for the medical diagnosis and/or index a date of diagnosis. In some embodiments, review of one or more record is performed multiple times and a consensus on the labeling is used as the ground truth for the record. In some embodiments, the independent review happens before the filtering step. For example, referring to Figure 3, in some embodiments, a sampling of medical records 41 in the subplurality of training records 304 are independently reviewed prior to the filtering step (354). In some embodiments, the independent review happens after the filtering step. For example, referring again to Figure 3, in some embodiments, a sampling of medical records 41 in the subplurality of training records 308 and/or filtered records 312 are independently reviewed after the filtering step (354).

In some embodiments, at least 10 medical records are independently reviewed. In some embodiments, at least 20 medical records, at least 25 medical records, at least 30 medical records, at least 40 medical records, at least 50 medical records, at least 75 medical records, at least 100 medical records, at least 150 medical records, at least 200 medical records, at least 250 medical records, at least 300 medical records, at least 400 medical records, at least 500 medical records, at least 750 medical records, at least 1000 medical records, at least 2500 medical records, at least 5000 medical records, at least 10,000 medical records, or more medical records are independently reviewed.

In some embodiments, no more than 50,000 medical records are independently reviewed. In some embodiments, no more than 25,000 medical records, no more than 10,000 medical records, no more than 7500 medical records, no more than 5000 medical records, no more than 2500 medical records, no more than 1000 medical records, no more than 750 medical records, no more than 500 medical records, no more than 250 medical records, no more than 100 medical records, or fewer medical records are independently reviewed.

In some embodiments, from 10 to 10,000 medical records are independently reviewed. In some embodiments, from 10 to 7500 medical records, from 10 to 5000 medical records, from 10 to 2500 medical records, from 10 to 1000 medical records, from 10 to 750 medical records, from 10 to 500 medical records, from 10 to 250 medical records, from 10 to 100 medical records, from 50 to 10,000 medical records, from 50 to 7500 medical records, from 50 to 5000 medical records, from 50 to 2500 medical records, from 50 to 1000 medical records, from 50 to 750 medical records, from 50 to 500 medical records, from 50 to 250 medical records, from 50 to 100 medical records, from 250 to 10,000 medical records, from 250 to 7500 medical records, from 250 to 5000 medical records, from 250 to 2500 medical records, from 250 to 1000 medical records, from 250 to 750 medical records, from 250 to 500 medical records, from 500 to 10,000 medical records, from 500 to 7500 medical records, from 500 to 5000 medical records, from 500 to 2500 medical records, from 500 to 1000 medical records, from 1000 to 10,000 medical records, from 1000 to 7500 medical records, from 1000 to 5000 medical records, or from 1000 to 2500 medical records are independently reviewed.

Accordingly, referring to block 206, in some embodiments, the method includes evaluating, for one or more respective subject in the second plurality of subjects (*e.g.,* medical records 302 illustrated in Figure 3) excluded from the first plurality of subjects (*e.g.,* excluded from subplurality of training records 308 or subplurality of validation records 310 and included in filtered records 312 as illustrated in Figure 3), whether the corresponding medical record indicates the presence of the medical condition (*e.g.,* PH) independent of the evaluation of the respective medical record during application of the extraction filter (*e.g.,* filtering 354 illustrated in Figure 3), and upon a determination that the respective medical record 41 indicates the presence of the medical condition, revising (*e.g.,* building filters (352) illustrated in Figure 3) the plurality of criteria 52 related to the first set of characteristics 53 to be more inclusive when applying the extraction filter.

Referring to block 208, in some embodiments, applying the extraction filter (*e.g.,* filtering 354 illustrated in Figure 3) includes, for each respective subject in the second plurality of subjects (*e.g.,* medical records 302 illustrated in Figure 3), evaluating a first data type (*e.g.,* one of structured clinical data 42, unstructured clinical data 43, medications 44, medical procedures 45, or laboratory results 46) for a first respective criterion 52-i in the plurality of criteria and evaluating a second data type (*e.g.,* a different one of structured clinical data 42, unstructured clinical data 43, medications 44, medical procedures 45, or laboratory results 46) for a second respective criterion 52-j in the plurality of criteria.

Referring to block 210, in some embodiments, the first data type is structured data (*e.g.,* structured clinical data 42) in an electronic health record (EHR) and the second type of data is unstructured data in the EHR (*e.g.,* unstructured clinical data 43). In some embodiments, the structured data is a medical billing code or diagnosis code and the unstructured data is a clinical note in the EHR.

Referring to block 212, in some embodiments, applying the extraction filter (*e.g.,* filtering 354 illustrated in Figure 3) includes evaluating at least three data types selected from structured electronic health record (EHR) data 42, unstructured EHR data 43, laboratory results 46, prescribed medications 44, and performed medical procedures 45. In some embodiments, applying the extraction filter includes evaluating structured electronic health record (EHR) data 42, unstructured EHR data 43, and laboratory results 46. In some embodiments, applying the extraction filter includes evaluating structured electronic health record (EHR) data 42, unstructured EHR data 43, and prescribed medications 44. In some embodiments, applying the extraction filter includes evaluating structured electronic health record (EHR) data 42, unstructured EHR data 43, and performed medical procedures 45. In some embodiments, applying the extraction filter includes evaluating structured electronic health record (EHR) data 42, laboratory results 46, and prescribed medications 44. In some embodiments, applying the extraction filter includes evaluating structured electronic health record (EHR) data 42, laboratory results 46, and performed medical procedures 45. In some embodiments, applying the extraction filter includes evaluating structured electronic health record (EHR) data 42, prescribed medications 44, and performed medical procedures 45. In some embodiments, applying the extraction filter includes evaluating unstructured EHR data 43, laboratory results 46, and prescribed medications 44. In some embodiments, applying the extraction filter includes evaluating unstructured EHR data 43, laboratory results 46, and performed medical procedures 45. In some embodiments, applying the extraction filter includes evaluating unstructured EHR data 43, prescribed medications 44, and performed medical procedures 45. In some embodiments, applying the extraction filter includes evaluating laboratory results 46, prescribed medications 44, and performed medical procedures 45.

In some embodiments, applying the extraction filter (*e.g.*, filtering 354 illustrated in Figure 3) includes evaluating at least four data types selected from structured electronic health record (EHR) data 42, unstructured EHR data 43, laboratory results 46, prescribed medications 44, and performed medical procedures 45. In some embodiments, applying the extraction filter includes evaluating unstructured EHR data 43, laboratory results 46, prescribed medications 44, and performed medical procedures 45. In some embodiments, applying the extraction filter includes evaluating structured electronic health record (EHR) data 42, laboratory results 46, prescribed medications 44, and performed medical procedures 45. In some embodiments, applying the extraction filter includes evaluating structured electronic health record (EHR) data 42, unstructured EHR data 43, prescribed medications 44, and performed medical procedures 45. In some embodiments, applying the extraction filter includes evaluating structured electronic health record (EHR) data 42, unstructured EHR data 43, laboratory results 46, and performed medical procedures 45. In some embodiments, applying the extraction filter includes evaluating structured electronic health record (EHR) data 42, unstructured EHR data 43, laboratory results 46, and prescribed medications 44.

In some embodiments, applying the extraction filter (*e.g.,* filtering 354 illustrated in Figure 3) includes evaluating at least structured electronic health record (EHR) data 42, unstructured EHR data 43, laboratory results 46, prescribed medications 44, and performed medical procedures 45.

In some embodiments, medications indicating a likelihood of a particular medical condition can be identified from a relevant compendium of targeted therapies. For example, with respect to medication that indicate a likelihood that a patient has cancer, or a particular type of cancer, can be found in National Cancer Institute, "List of Targeted Therapy Drugs Approved for Specific Types of Cancer," updated October 31, 2022, available on the Internet at cancer.gov/about-cancer/treatment/types/targeted-therapies/approved-drug-list, which is hereby incorporated herein by reference in its entirety.

In some embodiments, a medication is identified from one or more medication databases. For instance, the Translational Medicine Ontology (TMO). See, for example, Luciano et al., "The Translational Medicine Ontology and Knowledge Base: driving personalized medicine by bridging the gap between bench and bedside," Journal of Biomedical Semantics, 2011;2(2):S1, which is hereby incorporated herein by reference in its entirety. In some implementations, a medication is identified from the National Cancer Institute Thesaurus (NCIt). See, for example, "NCIthesaurus," Version 22.11d (Release date: 2022-11-28), available on the Internet at ncithesaurus.nci.nih.gov/ncitbrowser, which is hereby incorporated herein by reference in its entirety. Suitable examples of medication databases contemplated for use in the present disclosure further include, but are not limited to, CHM: CheMBL; DrugB: Drug-Bank; ODB: Ontario database; THIN: The Health Improvement Network; TCM: Traditional Chinese Medicine; TBDB: Tuberculosis Database; TTD: Therapeutic Target database; PDTD: Potential Drug-Target Database; TDR: Tropical Diseases Research; HIVRT: HIV Drug Resistance Database; TCMSP: Traditional Chinese Medicine Platform; SCYP: Super Cytochrome P450; DHUB: Drug Repurposing Hub; DSDB: Drug Signatures Database; PROM: Promiscuous; DRAR: Drug Repurposing Adverse Reaction; DMAP: Drug-Protein connectivity MAP; CMAP: Complement Map database; DMC: Drug Map Central; SIDER: Side Effect Resource; KSRPO: A platform for drug Repositioning; NNFIN: Network-based similarity finder; DSRV: Drug survival database; CHSP: anti-Cancer Herbs database for System Pharmacology; D2G: Drug to Gene; GSDB: Gene Set Database; SBIOS: Swiss BIOisostere; DTOM: Drug Target interactome database; DPTH: Drug Pathway database; DTW: Drug Target Web; DNET: Drug-disease Network database; SUT: SuperTarget database; DTC: Drug Target Commons; and/or KEGG: Kyoto Encyclopedia of Genes and Genomes. See, for example, Masoudi-Sobhanzadeh et al., "Drug databases and their contributions to drug repurposing," Genomics. 2020;112(2):1087-1095, which is hereby incorporated herein by reference in its entirety.

In some embodiments, a criterion related to the plurality of characteristics for the medical condition is one or more words, medical codes, or phrases that were, at least partially, automatically curated, *e.g.,* using an electronic medical glossary, medical dictionary, or the like. In some embodiments, a criterion is identified using natural language processing, *e.g.,* by training a machine learning model to identify electronic health records for patients affected by a health entity based on structured and/or unstructured clinical data and then performing post *hoc* model interpretability analysis to determine words and/or phrases providing predictive power to the model, *e.g.,* using a technique such as Local Interpretable Model-Agnostic Explanations (LIME), SHapley Additive exPlanations (SHAP), or the like. Similarly, NLP can be used to identify words and/or phrases that are significantly more prevalent in EHRs of patients affected by the medical condition than in EHRs of patients that are not affected by the medical condition.

In some embodiments, a criterion is evaluated using a string-matching algorithm. Generally, a string-matching algorithm (*e.g.,* a string-search algorithm), performs a function in which one or more sub-strings are identified within a larger string. The function further identifies the position at which the sub-string is located within the larger string. A string includes, but is not limited to, a sequence of text (*e.g.,* an EHR, a section, a text span, or a portion thereof). String-matching is further described in Bulus et al., 2017, "Comparison of String-Matching Algorithms in Web Documents," UniTech 2017, which is hereby incorporated herein by reference in its entirety. In some embodiments, the string-matching algorithm includes a naive string search, a finite-state-automaton-based search, a Rabin-Karp algorithm, a Knuth-Morris-Pratt algorithm, a Boyer-Moore string-search algorithm, a two-way string-matching algorithm, or a backward non-deterministic directed acyclic word graph matching algorithm using a computer system comprising a processor coupled to a non-transitory memory. In some embodiments, the string matching is exact string matching. That is, in some embodiments, the algorithm must find an exact match of a predefined string from the criteria (*e.g.,* a curated list of words, medical codes, and/or phrases) in the EHR in order to satisfy the criteria. In some embodiments, fuzzy matching logic is used in order to allow for some mismatch between an expression in the EHR and an expression in the curated list of words, medical codes, and/or phrases, *e.g.,* by applying a threshold value for a string distance between the expression in the EHR and the expression in the curated list. Methods for determining string distance, such as Levenshtein distance, Hamming distance, Jaro-Winkler distance, and the like, are known in the art. In some embodiments, wild cards are used to account for different forms and/or conjugations of a word when matching to a curated list, *e.g.,* where the curated list includes one or more Regular Expressions (RegEx). In some embodiments, the threshold value for the string distance is from 0 (*e.g.,* exact match) to 1 (*e.g.,* most permissive). For example, in some embodiments, the threshold value for the string distance is at least 0.1, at least 0.2, at least 0.3, at least 0.4, at least 0.5, at least 0.6, at least 0.7, or at least 0.8. In some embodiments, the threshold value for the string distance is no more than 0.99, no more than 0.95, no more than 0.9, no more than 0.8, no more than 0.7, no more than 0.6, no more than 0.5, no more than 0.4, no more than 0.3, or no more than 0.2. In some embodiments, the threshold value for the string distance is from 0.1 to 0.6, from 0.1 to 0.4, from 0.2 to 0.8, from 0.4 to 0.95, or from 0.3 to 0.7. In some embodiments, the threshold value for the string distance falls within another range from 0 to 1.

In some embodiments, a criterion related to the plurality of characteristics for the medical condition is one or more latent variables learned by a model. For example, in some embodiments, a natural language processing model is trained to distinguish between EHRs from subjects having a particular medical condition and EHRs from subjects that don't have the medical condition. Then, rather than trying to match expressions in an EHR to expressions in a curated list of words, medical codes, and/or phrases, part or all of the EHR is scored using the model to provide an indication (*e.g.,* a probability, likelihood, score, classification, *etc.*) of whether the EHR contains an expression that indicates the presence of the medical condition.

Any one of a number of clustering techniques can be used, examples of which include, but are not limited to, hierarchical clustering, k-means clustering, and density-based clustering. In some embodiments, the clustering algorithm is density-based spatial clustering of applications with noise (DBSCAN). In some embodiments, a hierarchical density-based clustering algorithm is used (referred to as HDBSCAN, *see, e.g.,* Campello et al., 2015, "Hierarchical density estimates for data clustering, visualization, and outlier detection," ACM Trans Knowl Disc Data, 10(1), 5). In another embodiment, a community detection-based cluster algorithm is used, such as Louvain clustering (*see, e.g.,* Blondel et al., 2008, "Fast unfolding of communities in large networks," J stat mech: theor exp, 2008(10), P10008). In yet another embodiment, Leiden clustering is used. *See, e.g.,* Traag et al., (2019), "From Louvain to Leiden: guaranteeing well-connected communities," Sci Rep 9:5233, doi: 10.1038/s41598-019-41695-z. In still another embodiment, a diffusion path algorithm is used. In some embodiments, the clustering algorithm comprises a pre-trained language model and/or an embedding model. In some embodiments, the clustering algorithm further comprises applying a dimensionality reduction technique to data from the EHR. In some implementations, each portion in a set of portions of the EHR is represented as a vector, such as a dense representation.

In some implementations, the language model is a pre-trained language model. In some implementations, the language model is a masked language modeling (MLM) model. In some implementations, the language model is BERT. See, for example, Devlin et al., 2018, "BERT: Pre-training of Deep Bidirectional Transformers for Language Understanding," arXiv:1810.04805v2). In some embodiments, the corresponding evaluation metric is a perplexity metric. Generally, perplexity refers to a measure of how well a language model predicts a sample, such as an EHR that is indicative of the presence or absence of a medical condition. For instance, in some embodiments, perplexity is determined as the inverse probability of the test set indicating the presence or absence of the medical condition, normalized by the number of words evaluated in the EHR. A lower perplexity indicates a higher probability, such that the model is better able to predict EHRs that indicate the presence or absence of a medical condition. In some embodiments, for each respective EHR in the plurality of EHRs, the respective scoring representation is a perplexity metric, and the method includes filtering the plurality of EHRs to remove each respective EHR that fails to satisfy an evaluation threshold (*e.g.,* removing EHRs with high perplexity scores). For example, in some embodiments, the respective scoring representation is a perplexity metric, and the evaluation threshold is no more than 200, no more than 100, no more than 90, no more than 80, no more than 70, no more than 60, no more than 50, no more than 40, no more than 30, no more than 20, no more than 10, no more than 5, no more than 3, or no more than 2, or less. In some embodiments, the respective scoring representation is a perplexity metric, and the evaluation threshold is at least 1, at least 2, at least 5, at least 10, at least 20, at least 50, or at least 100. In some embodiments, the respective scoring representation is a perplexity metric, and the evaluation threshold is from 1 to 5, from 2 to 10, from 1 to 3, from 2 to 50, from 5 to 100, or from 80 to 200. In some embodiments, the evaluation threshold falls within another range starting no lower than 1 and ending no higher than 200.

In some embodiments, any one or more of a variety of dimensionality reduction techniques is used. Examples include, but are not limited to, principal component analysis (PCA), non-negative matrix factorization (NMF), linear discriminant analysis (LDA), diffusion maps, or network (*e.g.,* neural network) techniques such as an autoencoder.

In some embodiments, the dimension reduction is a principal components algorithm, a random projection algorithm, an independent component analysis algorithm, a feature selection method, a factor analysis algorithm, Sammon mapping, curvilinear components analysis, a stochastic neighbor embedding (SNE) algorithm, an Isomap algorithm, a maximum variance unfolding algorithm, a locally linear embedding algorithm, a t-SNE algorithm, a non-negative matrix factorization algorithm, a kernel principal component analysis algorithm, a graph-based kernel principal component analysis algorithm, a linear discriminant analysis algorithm, a generalized discriminant analysis algorithm, a uniform manifold approximation and projection (UMAP) algorithm, a LargeVis algorithm, a Laplacian Eigenmap algorithm, or a Fisher's linear discriminant analysis algorithm. See, for example, Fodor, 2002, "A survey of dimension reduction techniques," Center for Applied Scientific Computing, Lawrence Livermore National, Technical Report UCRL-ID-148494; Cunningham, 2007, "Dimension Reduction," University College Dublin, Technical Report UCD-CSI-2007-7, Zahorian et al., 2011, "Nonlinear Dimensionality Reduction Methods for Use with Automatic Speech Recognition," Speech Technologies. doi:10.5772/16863. ISBN 978-953-307-996-7; and Lakshmi et al., 2016, "2016 IEEE 6th International Conference on Advanced Computing (IACC)," pp. 31-34. doi:10.1109/IACC.2016.16, ISBN 978-1-4673-8286-1, the contents of which are hereby incorporated by reference, in their entireties, for all purposes.

In some embodiments, the dimension reduction is a principal component analysis (PCA) algorithm, and each respective extracted dimension reduction component comprises a respective principal component derived by the PCA. In such embodiments, the number of principal components in the plurality of principal components can be limited to a threshold number of principal components calculated by the PCA algorithm. In some embodiments, the threshold number of principal components is, for example, at least 2, at least 3, at least 5, at least 10, at least 20, at least 50, at least 100, at least 1000, at least 1500, or any other number. In some embodiments, the threshold number of principal components is no more than 2000, no more than 1000, no more than 100, no more than 50, or no more than 10. In some embodiments, the threshold number of principal components is from 2 to 10, from 5 to 20, from 3 to 50, from 20 to 100, or from 100 to 2000. In some embodiments, the threshold number of principal components falls within another range starting no lower than 2 and ending no higher than 2000.

In some embodiments, the dimension reduction includes manifold learning using the respective scoring representation for each respective EHR in the plurality of EHRs. Generally, manifold learning is used to describe the low-dimensional structure of high-dimensional data by determining maximal variations in a dataset. Examples include, but are not limited to, force-directed layout(*see, e.g.,* Fruchterman, T. M., & Reingold, E. M. (1991). Graph drawing by force-directed placement. Software: Practice and experience, 21(11), 1129-1164) (*e.g.,* Force Atlas 2), t-distributed stochastic neighbor embedding (t-SNE), locally linear embedding (*see, e.g.,* Roweis, S. T., & Saul, L. K. (2000). Nonlinear dimensionality reduction by locally linear embedding. Science, 290(5500), 2323-2326), local linear isometric mapping (ISOMAP; *see, e.g.,* Tenenbaum, J. B., De Silva, V., & Langford, J. C. (2000). A global geometric framework for nonlinear dimensionality reduction. Science, 290(5500), 2319-2323), kernel PCA, graph-based kernel PCA, Potential of Heat-Diffusion for Affinity Based Trajectory Embedding (PHATE), generalized discriminant analysis (GDA), Uniform Manifold Approximation and Projection (UMAP), and/or kernel discriminant analysis.

In some embodiments, the dimension reduction includes discriminant analysis. Generally, force-directed layouts are useful in various particular embodiments because of their ability to identify new, lower dimensions that encode non-linear aspects of the underlying data which arise from underlying relationships between data elements. In some implementations, force directed layouts use physics-based models as mechanisms for determining a reduced dimensionality that best represents the data, make few assumptions about the structure of the data, and do not impose a de-noising approach. Manifold learning is further described, for example, in Wang et al., 2004, "Adaptive Manifold Learning," Advances in Neural Information Processing Systems 17, the content of which is hereby incorporated by reference, in its entirety, for all purposes.

Referring to block 214, in some embodiments, the plurality of criteria includes (i) a first criterion that when satisfied excludes a respective subject from the first plurality of subjects and (ii) a second criterion that when satisfied results in inclusion of the respective subject in the first plurality of subjects, provided no criterion in the plurality of criteria excludes the respective subject from the first plurality of subjects. For example, in some embodiments, for a medical condition that shares many symptoms with another medical condition, identification of a diagnosis code for the other medical condition in the EHR can be used as an exclusion criteria, while identification of a diagnosis code for the target condition can be used as an inclusion criteria.

In some embodiments, an initial structured phenotype for the medical condition is developed based, for example, on a combination of clinical domain expertise, literature and guideline review, along with data exploration. In some embodiments, the criteria and logic for this phenotype is discussed and agreed upon across a multi-disciplinary team of clinicians, informaticians, and data scientists. In some embodiments, this initial phenotype is then evaluated on the training and validation set chart reviews to assess performance. Review of results includes unblinded chart review of false positives and false negatives from the training set only, where the validation set is not used to avoid overfitting the model, to assess how the phenotype definitions and queries might be improved. In some embodiments, the structured phenotype is then revised to generate V2 and continue this cycle of evaluation and iteration until there are no further revisions that would clearly improve the phenotype.

Referring to block 216, in some embodiments, the second plurality of subjects comprises at least 10,000 subjects (e.g., subplurality of training records 308 as illustrated in Figure 3). In some embodiments, the second plurality of subjects includes at least 2500 subjects, at least 5000 subjects, at least 10,000 subjects, at least 25,000 subjects, at least 50,000 subjects, at least 100,000 subjects, at least 250,000 subjects, at least 500,000 subjects, at least 1 million subjects, at least 2.5 million subjects, at least 5 million subjects, at least 10 million subjects, at least 25 million subjects, at least 50 million subjects, at least 100 million subjects, at least 250 million subjects, at least 500 million subjects, at least 1 billion subjects, or more subjects.

In some embodiments, the second plurality of subjects includes no more than 1 billion subjects, no more than 500 million subjects, no more than 250 million subjects, no more than 100 million subjects, no more than 50 million subjects, no more than 25 million subjects, no more than 10 million subjects, no more than 5 million subjects, no more than 2.5 million subjects, no more than 1 million subjects, no more than 500,000 subjects, no more than 250,000 subjects, no more than 100,000 subjects, no more than 50,000 subjects, no more than 25,000 subjects, no more than 10,000 subjects, no more than 5000 subjects, no more than 2500 subjects, no more than 1000 subjects, or fewer subjects.

In some embodiments, the second plurality of subjects includes from 500 to 1 billion subjects, from 1000 to 1 billion subjects, from 5000 to 1 billion subjects, from 10,000 to 1 billion subjects, from 50,000 to 1 billion subjects, from 100,000 to 1 billion subjects, from 500,000 to 1 billion subjects, from 1 million to 1 billion subjects, from 5 million to 1 billion subjects, from 10 million to 1 billion subjects, from 50 million to 1 billion subjects, from 100 million to 1 billion subjects, or from 500 million to 1 billion subjects. In some embodiments, the second plurality of subjects includes from 500 to 250 million subjects, from 1000 to 250 million subjects, from 5000 to 250 million subjects, from 10,000 to 250 million subjects, from 50,000 to 250 million subjects, from 100,000 to 250 million subjects, from 500,000 to 250 million subjects, from 1 million to 250 million subjects, from 5 million to 250 million subjects, from 10 million to 250 million subjects, from 50 million to 250 million subjects, or from 100 million to 250 million subjects. In some embodiments, the second plurality of subjects includes from 500 to 10 million subjects, from 1000 to 10 million subjects, from 5000 to 10 million subjects, from 10,000 to 10 million subjects, from 50,000 to 10 million subjects, from 100,000 to 10 million subjects, from 500,000 to 10 million subjects, from 1 million to 10 million subjects, or from 5 million to 10 million subjects. In some embodiments, the second plurality of subjects includes from 500 to 1 million subjects, from 1000 to 1 million subjects, from 5000 to 1 million subjects, from 10,000 to 1 million subjects, from 50,000 to 1 million subjects, from 100,000 to 1 million subjects, or from 500,000 to 1 million subjects. In some embodiments, the second plurality of subjects includes from 500 to 250,000 subjects, from 1000 to 250,000 subjects, from 5000 to 250,000 subjects, from 10,000 to 250,000 subjects, from 50,000 to 250,000 subjects, or from 100,000 to 250,000 subjects. In some embodiments, the second plurality of subjects includes from 500 to 100,000 subjects, from 1000 to 100,000 subjects, from 5000 to 100,000 subjects, from 10,000 to 100,000 subjects, or from 50,000 to 100,000 subjects.

Referring to block 218, in some embodiments, the medical condition is pulmonary hypertension (PH). PH is high blood pressure affecting the right side of the heart and arteries in the lungs. In one type of PH, pulmonary arterial hypertension (PAH), high blood pressure causes the arteries in the lungs to constrict, reducing oxygen levels in the blood. It has been estimated the PH affects approximately 1% of the global population. For more information on PH see, for example, Mandras SA, et al., "Pulmonary Hypertension: A Brief Guide for Clinicians" Mayo Clin Proc., 95(9):1978-88 (2020), which is hereby incorporated herein by reference in its entirety.

In some embodiments, the medical condition is cancer. In some embodiments, the medical condition is a particular type of cancer and/or a particular cancer stage. Examples of cancer include, but are not limited to, carcinoma, lymphoma, blastoma, glioblastoma, sarcoma, and leukemia. In some embodiments, non-limiting cancers include breast cancer, squamous cell cancer, lung cancer (including small-cell lung cancer, non-small cell lung cancer (NSCLC), adenocarcinoma of the lung, and squamous carcinoma of the lung (e.g., squamous NSCLC)), various types of head and neck cancer (e.g., HNSC), cancer of the peritoneum, hepatocellular cancer, gastric or stomach cancer (including gastrointestinal cancer), pancreatic cancer, ovarian cancer, cervical cancer, liver cancer, bladder cancer, hepatoma, colon cancer, colorectal cancer, endometrial or uterine carcinoma, salivary gland carcinoma, kidney or renal cancer, liver cancer, prostate cancer, vulval cancer, thyroid cancer, and hepatic carcinoma. In some embodiments, non-limiting cancers further include B-cell lymphoma (including low grade/follicular non-Hodgkin's lymphoma (NHL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, and Waldenstrom's Macroglobulinemia), chronic lymphocytic leukemia (CLL), acute lymphoblastic leukemia (ALL), hairy cell leukemia, chronic myeloblastic leukemia, and post-transplant lymphoproliferative disorder (PTLD). In some embodiments, non-limiting cancers further include abnormal vascular proliferation associated with phakomatoses, edema (such as that associated with brain tumors), and/or Meigs' syndrome.

As would be well understood by one of ordinary skill, the term "cancer" for use with methods and systems of the present disclosure is not limited only to primary forms of cancer, but also involves cancer subtypes. Some such cancer subtypes are listed above but also include breast cancer subtypes such as Luminal A (hormone receptor (HR)+/human epidermal growth factor receptor (HER2)-); Luminal B (HR+/HER2+); Triple-negative or (HR-/HER2-) and HER2 positive. Other cancer subtypes include the various lung cancers listed above and prostate cancer subtypes involving changes in E26 transformation specific genes (ETS; specifically ERG, ETV1/4, and FLI1 genes) and subsets defined by mutations in FOXA1, SPOP, and IDH1 genes.

In some embodiments, a goal of method 200 is to identify instances of the medical condition by capturing a diverse set of different multimodal signals indicating the presence of the medical condition. In some embodiments, each of these signals are expressed by an individual 1-class classifier, referred to herein as a "label function." A label function is a simple set of rules meant to pattern match for a particular signal indicating the presence or absence of the medical condition. In some embodiments, when the set of rules (criteria) for a particular labeling function is satisfied, the labeling function votes for a predetermined class, *e.g.,* class 0, indicating the absence of the medical condition or no finding of the medical condition (*e.g.,* if the absence of the medical condition cannot be positively confirmed) or class 1 indicating the presence of the medical condition. In some embodiments, when the set of criteria for a particular labeling function is not satisfied, the labeling function abstains from voting, i.e., evaluation of the particular labeling function does not contribute to the final determination of whether the medical record indicates the presence or does not indicate the presence of the medical condition. In some embodiments, the labeling function can only either vote for a predetermined class, *e.g.,* a class corresponding to the presence of the medical condition or a class corresponding to the absence of a medical condition, or the absence of an indication of the medical condition, or abstain from voting. These rules can include any number of sets of conditions. For instance, with respect to pulmonary hypertension, a criteria might include a regular expression, such as "pulm.{0,10}hypertension" matched against text data; the presence of a code indicating pulmonary hypertension in a structured field; a lab value satisfying a threshold that indicates pulmonary hypertension; *etc.*

Accordingly, referring to block 220, in some embodiments method 200 includes generating a plurality of labeling functions 61 (*e.g.,* illustrated as step 360 in Figure 3) for the medical condition, where each respective labeling function in the plurality of labeling functions includes a corresponding set of one or more criterion 62 that, when satisfied, indicate a presence or an absence of the medical condition.

Referring to block 222, in some embodiments, the plurality of labeling functions includes a respective labeling function 61 for each respective subgroup in a plurality subgroups of subjects with the medical condition. For example, as illustrated in Figure 4B, different subgroups 404 of subjects having a medical condition will have different indications of the medical condition in their EHR. Different labeling functions 406 can be used to identify different subgroups 404 of these subjects having the medical condition. For example, subgroup 404-1 can be identified as having the medical condition based on information in the unstructured clinical notes portion of their EHR. Accordingly, a labeling function 406-1 having one or more criterion 62 that can be satisfied by information stored within unstructured clinical notes can be used to identify all or a portion of subgroup 404-1, but not patients in subgroup 404-2, which have indications of the medical condition within laboratory results stored in their EHR. Accordingly, one or more labeling functions (*e.g.,* labeling functions 406-2 and 406-3) can be generated to identify subjects having the medical condition based on laboratory results. Likewise, one or more labeling functions (*e.g.,* LF 406-4) can be generated to identify subjects 404-3 having the medical condition based on medications that have been recommended or prescribed to the subject.

Referring to block 224, in some embodiments, the plurality of labeling functions includes a first respective labeling function 61 including a corresponding set of one or more criterion 62 that, when satisfied, indicate a presence of the medical condition. For example, a criterion satisfied by identification of a medical diagnostic or billing code for the medical condition.

Referring to block 226, in some embodiments, generating the plurality of labeling functions includes, for the first respective labeling function 61, i) defining a first set of one or more criterion 62 that, when satisfied, indicate the presence of the medical condition, ii) determining, for each respective subject in a first subset of the first plurality of subjects, whether the corresponding medical record for the respective subject satisfies the first set of one or more criterion (*e.g.,* evaluating (362) the first respective labeling function over a subset of the plurality of training candidates 308 as illustrated in Figure 3), iii) evaluating, for a respective subject in the first subset of the first plurality of subjects determined to satisfy the first set of one or more criteria whether the corresponding medical record indicates the absence of the medical condition, independent of the determination of whether the corresponding medical record satisfies the first set of one or more criterion, and iv) upon a determination that the corresponding medical record indicates the absence of the medical condition, revising the first set of one or more criteria to be less inclusive (*e.g.,* returning to step 360 of building label functions when the labeling function has misidentified a sample EHR as indicating the presence of the medical condition, as illustrated in Figure 3). Referring to block 228, in some embodiments, the revising includes adding an exclusion criterion to the first set of one or more criterion. For example, when the target medical disorder is pulmonary hypertension (commonly abbreviated as PH), a mention of "Ph.D." in a medical record might satisfy a criteria 62 such that the record is identified as indicating the presence of PH. By identifying such record, the labeling function 61 having the criterion 62 satisfied by "Ph.D." can be modified to exclude "Ph.D." from meeting inclusion criteria.

Referring to block 230, in some embodiments, the plurality of labeling functions 61 includes a second respective labeling function including a corresponding set of one or more criterion 62 that, when satisfied, indicate an absence of the medical condition. For example, a criterion satisfied by identification of a medical diagnostic or billing code for another medical condition that shares one or more symptoms with target medical condition.

Referring to block 232, in some embodiments, generating the plurality of labeling functions includes, for the second respective labeling function 61, i) defining a second set of one or more criterion 62 that, when satisfied, indicate the absence of the medical condition, ii) determining, for each respective subject in a second subset of the first plurality of subjects, whether the corresponding medical record satisfies the second set of one or more criterion (*e.g.,* evaluating (362) the second respective labeling function over a subset of the plurality of training candidates 308 as illustrated in Figure 3), iii) evaluating, for a respective subject in the second subset of the first plurality of subjects determined to satisfy the second set of one or more criteria whether the corresponding medical record indicates the presence of the medical condition, independent of the determination of whether the respective medical record satisfies the second set of one or more criterion, and iv) upon a determination that the respective medical record indicates the presence of the medical condition, revising the second set of one or more criteria to be more inclusive (*e.g.,* returning to step 360 of building label functions when the labeling function has misidentified a sample EHR as indicating the absence of the medical condition, as illustrated in Figure 3). Referring to block 234, in some embodiments, the revising includes adding an inclusion criterion to the second set of one or more criterion. For example, when the target medical disorder is pulmonary hypertension (commonly abbreviated as PH), an EHR containing a medical diagnostic or billing code for heart disease might satisfy an exclusion criteria 62 such that the record is misidentified as indicating the absence of PH, even when laboratory results show the subject has a mean pulmonary artery pressure of greater than 20 mm Hg, the labeling function 61 can be modified to include an inclusion criteria 62 satisfied by a mean pulmonary artery pressure of greater than 20 mm Hg.

Referring to block 236, in some embodiments, the plurality of label functions includes at least 10 labeling functions. In some embodiments, the plurality of labeling functions includes at least 3 labeling functions, at least 4 labeling functions, at least 5 labeling functions, at least 6 labeling functions, at least 7 labeling functions, at least 8 labeling functions, at least 9 labeling functions, at least 10 labeling functions, at least 15 labeling functions, at least 20 labeling functions, at least 25 labeling functions, at least 30 labeling functions, at least 40 labeling functions, at least 50 labeling functions, or more labeling functions.

In some embodiments, the dimension reduction includes discriminant analysis. Generally, force-directed layouts are useful in various particular embodiments because of their ability to identify new, lower dimensions that encode non-linear aspects of the underlying data which arise from underlying relationships between data elements. In some implementations, force directed layouts use physics-based models as mechanisms for determining a reduced dimensionality that best represents the data, make few assumptions about the structure of the data, and do not impose a de-noising approach. Manifold learning is further described, for example, in Wang et al., 2004, "Adaptive Manifold Learning," Advances in Neural Information Processing Systems 17, the content of which is hereby incorporated by reference, in its entirety, for all purposes.

Referring to block 236, method 200 also includes assigning, to each respective subject in a first plurality of subjects medical record in a first plurality of medical records (*e.g.,* subplurality of training records 308 as illustrated in Figure 3), a corresponding label 47 indicating a status of the medical condition by evaluating first information (*e.g.,* one or more of structured electronic health record (EHR) data 42, unstructured EHR data 43, laboratory results 46, prescribed medications 44, and performed medical procedures 45) from a corresponding medical record for the respective subject using an ensemble model including the plurality of labeling functions 61 to obtain as output from the ensemble model a prediction 47 for the status of the medical condition, where the evaluating includes natural language processing of at least a portion of the respective medical record.

In some embodiments, a criterion 62 is evaluated using a string-matching algorithm. Generally, a string-matching algorithm (*e.g.,* a string-search algorithm), performs a function in which one or more sub-strings are identified within a larger string. The function further identifies the position at which the sub-string is located within the larger string. A string includes, but is not limited to, a sequence of text (*e.g.,* an EHR, a section, a text span, or a portion thereof). String-matching is further described in Bulus et al., 2017, "Comparison of String-Matching Algorithms in Web Documents," UniTech 2017, which is hereby incorporated herein by reference in its entirety. In some embodiments, the string-matching algorithm includes a naive string search, a finite-state-automaton-based search, a Rabin-Karp algorithm, a Knuth-Morris-Pratt algorithm, a Boyer-Moore string-search algorithm, a two-way string-matching algorithm, or a backward non-deterministic directed acyclic word graph matching algorithm using a computer system comprising a processor coupled to a non-transitory memory. In some embodiments, the string matching is exact string matching. That is, in some embodiments, the algorithm must find an exact match of a predefined string from the criteria (*e.g.,* a curated list of words, medical codes, and/or phrases) in the EHR in order to satisfy the criteria. In some embodiments, fuzzy matching logic is used in order to allow for some mismatch between an expression in the EHR and an expression in the curated list of words, medical codes, and/or phrases, e.g., by applying a threshold value for a string distance between the expression in the EHR and the expression in the curated list. Methods for determining string distance, such as Levenshtein distance, Hamming distance, Jaro-Winkler distance, and the like, are known in the art. In some embodiments, wild cards are used to account for different forms and/or conjugations of a word when matching to a curated list, *e.g.,* where the curated list includes one or more Regular Expressions (RegEx). In some embodiments, the threshold value for the string distance is from 0 (*e.g.,* exact match) to 1 (*e.g.,* most permissive). For example, in some embodiments, the threshold value for the string distance is at least 0.1, at least 0.2, at least 0.3, at least 0.4, at least 0.5, at least 0.6, at least 0.7, or at least 0.8. In some embodiments, the threshold value for the string distance is no more than 0.99, no more than 0.95, no more than 0.9, no more than 0.8, no more than 0.7, no more than 0.6, no more than 0.5, no more than 0.4, no more than 0.3, or no more than 0.2. In some embodiments, the threshold value for the string distance is from 0.1 to 0.6, from 0.1 to 0.4, from 0.2 to 0.8, from 0.4 to 0.95, or from 0.3 to 0.7. In some embodiments, the threshold value for the string distance falls within another range from 0 to 1.

In some embodiments, a criterion 62 is evaluated by searching for one or more latent variables learned by a model. For example, in some embodiments, a natural language processing model is trained to distinguish between EHRs from subjects having a particular medical condition and EHRs from subjects that don't have the medical condition. Then, rather than trying to match expressions in an EHR to expressions in a curated list of words, medical codes, and/or phrases, part or all of the EHR is scored using the model to provide an indication of whether the EHR contains an expression that indicates the presence of the medical condition.

Any one of a number of clustering techniques can be used, examples of which include, but are not limited to, hierarchical clustering, k-means clustering, and density-based clustering. In some embodiments, the clustering algorithm is density-based spatial clustering of applications with noise (DBSCAN). In some embodiments, a hierarchical density-based clustering algorithm is used (referred to as HDBSCAN, *see, e.g.,* Campello et al., 2015, "Hierarchical density estimates for data clustering, visualization, and outlier detection," ACM Trans Knowl Disc Data, 10(1), 5). In another embodiment, a community detection-based cluster algorithm is used, such as Louvain clustering (*see, e.g.,* Blondel et al., 2008, "Fast unfolding of communities in large networks," J stat mech: theor exp, 2008(10), P10008). In yet another embodiment, Leiden clustering is used. *See, e.g.,* Traag et al., (2019), "From Louvain to Leiden: guaranteeing well-connected communities," Sci Rep 9:5233, doi: 10.1038/s41598-019-41695-z. In still another embodiment, a diffusion path algorithm is used. In some embodiments, the clustering algorithm comprises a pre-trained language model and/or an embedding model. In some embodiments, the clustering algorithm further comprises applying a dimensionality reduction technique to data from the EHR. In some implementations, each portion in a set of portions of the EHR is represented as a vector, such as a dense representation.

In some implementations, the language model is a pre-trained language model. In some implementations, the language model is a masked language modeling (MLM) model. In some implementations, the language model is BERT. See, for example, Devlin et al., 2018, "BERT: Pre-training of Deep Bidirectional Transformers for Language Understanding," arXiv:1810.04805v2). In some embodiments, the corresponding evaluation metric is a perplexity metric. Generally, perplexity refers to a measure of how well a language model predicts a sample, such as an EHR that is indicative of the presence or absence of a medical condition. For instance, in some embodiments, perplexity is determined as the inverse probability of the test set indicating the presence or absence of the medical condition, normalized by the number of words evaluated in the EHR. A lower perplexity indicates a higher probability, such that the model is better able to predict EHRs that indicate the presence or absence of a medical condition. In some embodiments, for each respective EHR in the plurality of EHRs, the respective scoring representation is a perplexity metric, and the method includes filtering the plurality of EHRs to remove each respective EHR that fails to satisfy an evaluation threshold (*e.g.,* removing EHRs with high perplexity scores). For example, in some embodiments, the respective scoring representation is a perplexity metric, and the evaluation threshold is no more than 200, no more than 100, no more than 90, no more than 80, no more than 70, no more than 60, no more than 50, no more than 40, no more than 30, no more than 20, no more than 10, no more than 5, no more than 3, or no more than 2, or less. In some embodiments, the respective scoring representation is a perplexity metric, and the evaluation threshold is at least 1, at least 2, at least 5, at least 10, at least 20, at least 50, or at least 100. In some embodiments, the respective scoring representation is a perplexity metric, and the evaluation threshold is from 1 to 5, from 2 to 10, from 1 to 3, from 2 to 50, from 5 to 100, or from 80 to 200. In some embodiments, the evaluation threshold falls within another range starting no lower than 1 and ending no higher than 200.

In some embodiments, any one or more of a variety of dimensionality reduction techniques is used. Examples include, but are not limited to, principal component analysis (PCA), non-negative matrix factorization (NMF), linear discriminant analysis (LDA), diffusion maps, or network (*e.g.,* neural network) techniques such as an autoencoder.

In some embodiments, the dimension reduction is a principal components algorithm, a random projection algorithm, an independent component analysis algorithm, a feature selection method, a factor analysis algorithm, Sammon mapping, curvilinear components analysis, a stochastic neighbor embedding (SNE) algorithm, an Isomap algorithm, a maximum variance unfolding algorithm, a locally linear embedding algorithm, a t-SNE algorithm, a non-negative matrix factorization algorithm, a kernel principal component analysis algorithm, a graph-based kernel principal component analysis algorithm, a linear discriminant analysis algorithm, a generalized discriminant analysis algorithm, a uniform manifold approximation and projection (UMAP) algorithm, a LargeVis algorithm, a Laplacian Eigenmap algorithm, or a Fisher's linear discriminant analysis algorithm. See, for example, Fodor, 2002, "A survey of dimension reduction techniques," Center for Applied Scientific Computing, Lawrence Livermore National, Technical Report UCRL-ID-148494; Cunningham, 2007, "Dimension Reduction," University College Dublin, Technical Report UCD-CSI-2007-7, Zahorian et al., 2011, "Nonlinear Dimensionality Reduction Methods for Use with Automatic Speech Recognition," Speech Technologies. doi:10.5772/16863. ISBN 978-953-307-996-7; and Lakshmi et al., 2016, "2016 IEEE 6th International Conference on Advanced Computing (IACC)," pp. 31-34. doi:10.1109/IACC.2016.16, ISBN 978-1-4673-8286-1, the contents of which are hereby incorporated by reference, in their entireties, for all purposes.

In some embodiments, the dimension reduction is a principal component analysis (PCA) algorithm, and each respective extracted dimension reduction component comprises a respective principal component derived by the PCA. In such embodiments, the number of principal components in the plurality of principal components can be limited to a threshold number of principal components calculated by the PCA algorithm. In some embodiments, the threshold number of principal components is, for example, at least 2, at least 3, at least 5, at least 10, at least 20, at least 50, at least 100, at least 1000, at least 1500, or any other number. In some embodiments, the threshold number of principal components is no more than 2000, no more than 1000, no more than 100, no more than 50, or no more than 10. In some embodiments, the threshold number of principal components is from 2 to 10, from 5 to 20, from 3 to 50, from 20 to 100, or from 100 to 2000. In some embodiments, the threshold number of principal components falls within another range starting no lower than 2 and ending no higher than 2000.

In some embodiments, the dimension reduction includes manifold learning using the respective scoring representation for each respective EHR in the plurality of EHRs. Generally, manifold learning is used to describe the low-dimensional structure of high-dimensional data by determining maximal variations in a dataset. Examples include, but are not limited to, force-directed layout(*see, e.g.,* Fruchterman, T. M., & Reingold, E. M. (1991). Graph drawing by force-directed placement. Software: Practice and experience, 21(11), 1129-1164) (*e.g.,* Force Atlas 2), t-distributed stochastic neighbor embedding (t-SNE), locally linear embedding (*see, e.g.,* Roweis, S. T., & Saul, L. K. (2000). Nonlinear dimensionality reduction by locally linear embedding. Science, 290(5500), 2323-2326), local linear isometric mapping (ISOMAP; *see, e.g.,* Tenenbaum, J. B., De Silva, V., & Langford, J. C. (2000). A global geometric framework for nonlinear dimensionality reduction. Science, 290(5500), 2319-2323), kernel PCA, graph-based kernel PCA, Potential of Heat-Diffusion for Affinity Based Trajectory Embedding (PHATE), generalized discriminant analysis (GDA), Uniform Manifold Approximation and Projection (UMAP), and/or kernel discriminant analysis.

Referring to block 238, in some embodiments, the first plurality of subjects includes at least 500 subjects. In some embodiments, the first plurality of subjects includes at least 1000 subjects, at least 2500 subjects, at least 5000 subjects, at least 10,000 subjects, at least 25,000 subjects, at least 50,000 subjects, at least 100,000 subjects, at least 250,000 subjects, at least 500,000 subjects, at least 1 million subjects, at least 2.5 million subjects, at least 5 million subjects, at least 10 million subjects, at least 25 million subjects, at least 50 million subjects, at least 100 million subjects, at least 250 million subjects, at least 500 million subjects, at least 1 billion subjects, or more subjects.

In some embodiments, the first plurality of subjects includes no more than 1 billion subjects, no more than 500 million subjects, no more than 250 million subjects, no more than 100 million subjects, no more than 50 million subjects, no more than 25 million subjects, no more than 10 million subjects, no more than 5 million subjects, no more than 2.5 million subjects, no more than 1 million subjects, no more than 500,000 subjects, no more than 250,000 subjects, no more than 100,000 subjects, no more than 50,000 subjects, no more than 25,000 subjects, no more than 10,000 subjects, no more than 5000 subjects, no more than 2500 subjects, no more than 1000 subjects, or fewer subjects.

In some embodiments, the first plurality of subjects includes from 500 to 1 billion subjects, from 1000 to 1 billion subjects, from 5000 to 1 billion subjects, from 10,000 to 1 billion subjects, from 50,000 to 1 billion subjects, from 100,000 to 1 billion subjects, from 500,000 to 1 billion subjects, from 1 million to 1 billion subjects, from 5 million to 1 billion subjects, from 10 million to 1 billion subjects, from 50 million to 1 billion subjects, from 100 million to 1 billion subjects, or from 500 million to 1 billion subjects. In some embodiments, the first plurality of subjects includes from 500 to 250 million subjects, from 1000 to 250 million subjects, from 5000 to 250 million subjects, from 10,000 to 250 million subjects, from 50,000 to 250 million subjects, from 100,000 to 250 million subjects, from 500,000 to 250 million subjects, from 1 million to 250 million subjects, from 5 million to 250 million subjects, from 10 million to 250 million subjects, from 50 million to 250 million subjects, or from 100 million to 250 million subjects. In some embodiments, the first plurality of subjects includes from 500 to 10 million subjects, from 1000 to 10 million subjects, from 5000 to 10 million subjects, from 10,000 to 10 million subjects, from 50,000 to 10 million subjects, from 100,000 to 10 million subjects, from 500,000 to 10 million subjects, from 1 million to 10 million subjects, or from 5 million to 10 million subjects. In some embodiments, the first plurality of subjects includes from 500 to 1 million subjects, from 1000 to 1 million subjects, from 5000 to 1 million subjects, from 10,000 to 1 million subjects, from 50,000 to 1 million subjects, from 100,000 to 1 million subjects, or from 500,000 to 1 million subjects. In some embodiments, the first plurality of subjects includes from 500 to 250,000 subjects, from 1000 to 250,000 subjects, from 5000 to 250,000 subjects, from 10,000 to 250,000 subjects, from 50,000 to 250,000 subjects, or from 100,000 to 250,000 subjects. In some embodiments, the first plurality of subjects includes from 500 to 100,000 subjects, from 1000 to 100,000 subjects, from 5000 to 100,000 subjects, from 10,000 to 100,000 subjects, or from 50,000 to 100,000 subjects.

In some embodiments, the corresponding prediction 47 is a probability that the medical record indicates the presence of the medical condition. In some embodiments, the corresponding prediction 47 is a binary indication (e.g., yes/no and/or 0/1) that the medical record indicates the presence of the medical condition.

Each individual label function 61 will capture a different, optionally multimodal feature correlated with the target medical condition. For example, referring again to Figure 4B, labeling function 406-1 captures features within unstructured clinical notes that indicate the presence of the medical condition while labeling function 406-4 captures features related to medications recommended or prescribed to the subject that indicate the presence of the medical condition. Consequently, no single label function will entirely capture all cases, nor will they be perfectly accurate, which means the label functions can be thought of as weak labels. These label functions can also contradict each other on any given data point. While the structured phenotyping approach relies on Subject Matter Expert (SME) input to consolidate these potentially conflicting views, there is a challenge in stitching together all these different features, particularly as the number of labeling function increases. For example, without careful sampling practices in place, the final structured phenotype can be overfit to a particular dataset after several iterations of fine-tuning.

In some embodiments, a majority voting model is used to aggregate the signals from the plurality of label functions. In majority voting, each label function casts a vote on the label of a given datapoint and the majority winner is accepted as the official label. Yet, a simple majority vote fails to account for information pertaining to perceived label function accuracy and confidence when weighing these votes. However, several approaches are known to account for these attributes. For example, the Snorkel Labeling Package, described in Ratner A. et al., "Snorkel: Rapid Training Data Creation with Weak Supervision," PVLDB, 11(3) (2017), which is incorporated herein by reference in its entirety, is an aggregation approach that often can provide a small premium over majority voting.

Accordingly, referring to block 240, in some embodiments, the ensemble model includes an aggregate voting model based on the evaluation of each respective labeling function. Referring to block 242, in some embodiments, the aggregate voting model is a weighted voting model.

Referring to block 244, in some embodiments, the ensemble model includes a probabilistic graphical model (PGM). In some embodiments, the PGM is a generalized case of a majority voting model, in which each labeling function is assigned an independent prior. Examples of such PGMs are provided in Example 4. For a review of PGM models see, for example, Koller and Friedman, "Probabilistic Graphical Models" Massachusetts: MIT Press (2009), ISBN 978-0-262-01319-2, which is hereby incorporated herein by reference in its entirety.

In some embodiments, the PGM can be solved for as:

$$P(l = 1|\lambda_0,\ldots,\lambda_K) = \frac{\alpha_\rho \ \prod_j \omega(s_j)}{\alpha_\rho \ \prod_j \omega(s_j) + \beta_\rho \ \prod_j \omega(\overline{s_j})},$$

where:

$l_{i \in (0,N)}$ is a binary variable indicating whether a subject does or does not have the medical disorder,

$\lambda_{ij}$ is binary voting variable for labeling function $j$ of $K$ independent labeling functions,

$s_j = P(\lambda_j = 1|l = 1)$ is a true positive rate for labeling function $j$,

$\overline{s}_j = P(\lambda_j = 1|l = 0)$ is a false positive rate for labeling function $j$,

$\alpha_\rho$ is an alpha parameter of a beta distribution $\rho \sim Beta(\alpha_\rho, \beta_\rho)$ defining a prior, and

$\beta_\rho$ is a beta parameter of the beta distribution $\rho \sim Beta(\alpha_\rho, \beta_\rho)$ defining a prior.

Referring to block 246, in some embodiments, evaluating the ensemble model including the plurality of labeling functions includes, for each respective subject in the first plurality of subjects, evaluating a first data type for a first respective criterion in the plurality of criteria and evaluating a second data type for a second respective criterion in the plurality of criteria.

Referring to block 248, in some embodiments, the first data type is structured data in an electronic health record (EHR) and the second type of data is unstructured data in the EHR. In some embodiments, the structured data is a medical billing code or diagnosis code, and the unstructured data is a clinical note in the EHR.

Referring to block 250, in some embodiments, applying the extraction filter includes evaluating at least three data types selected from structured electronic health record (EHR) data 42, unstructured EHR data 43, laboratory results 46, prescribed medications 44, and performed medical procedures 45. In some embodiments, applying the extraction filter includes evaluating structured electronic health record (EHR) data 42, unstructured EHR data 43, and laboratory results 46. In some embodiments, applying the extraction filter includes evaluating structured electronic health record (EHR) data 42, unstructured EHR data 43, and prescribed medications 44. In some embodiments, applying the extraction filter includes evaluating structured electronic health record (EHR) data 42, unstructured EHR data 43, and performed medical procedures 45. In some embodiments, applying the extraction filter includes evaluating structured electronic health record (EHR) data 42, laboratory results 46, and prescribed medications 44. In some embodiments, applying the extraction filter includes evaluating structured electronic health record (EHR) data 42, laboratory results 46, and performed medical procedures 45. In some embodiments, applying the extraction filter includes evaluating structured electronic health record (EHR) data 42, prescribed medications 44, and performed medical procedures 45. In some embodiments, applying the extraction filter includes evaluating unstructured EHR data 43, laboratory results 46, and prescribed medications 44. In some embodiments, applying the extraction filter includes evaluating unstructured EHR data 43, laboratory results 46, and performed medical procedures 45. In some embodiments, applying the extraction filter includes evaluating unstructured EHR data 43, prescribed medications 44, and performed medical procedures 45. In some embodiments, applying the extraction filter includes evaluating laboratory results 46, prescribed medications 44, and performed medical procedures 45.

In some embodiments, applying the extraction filter (*e.g.,* filtering 354 illustrated in Figure 3) includes evaluating at least four data types selected from structured electronic health record (EHR) data 42, unstructured EHR data 43, laboratory results 46, prescribed medications 44, and performed medical procedures 45. In some embodiments, applying the extraction filter includes evaluating unstructured EHR data 43, laboratory results 46, prescribed medications 44, and performed medical procedures 45. In some embodiments, applying the extraction filter includes evaluating structured electronic health record (EHR) data 42, laboratory results 46, prescribed medications 44, and performed medical procedures 45. In some embodiments, applying the extraction filter includes evaluating structured electronic health record (EHR) data 42, unstructured EHR data 43, prescribed medications 44, and performed medical procedures 45. In some embodiments, applying the extraction filter includes evaluating structured electronic health record (EHR) data 42, unstructured EHR data 43, laboratory results 46, and performed medical procedures 45. In some embodiments, applying the extraction filter includes evaluating structured electronic health record (EHR) data 42, unstructured EHR data 43, laboratory results 46, and prescribed medications 44.

In some embodiments, applying the extraction filter (*e.g.,* filtering 354 illustrated in Figure 3) includes evaluating at least structured electronic health record (EHR) data 42, unstructured EHR data 43, laboratory results 46, prescribed medications 44, and performed medical procedures 45.

Referring to block 252, in some embodiments, applying the extraction filter includes, for a respective subject in the plurality of subjects, natural language processing of unstructured clinical notes from the EHR. Referring to block 254, in some embodiments, the natural language processing includes matching one or more regular expressions against the corresponding unstructured clinical notes. Referring to block 256, in some embodiments, the natural language processing includes inputting at least a portion of the unstructured clinical notes into a machine learning model trained to identify language related to the clinical condition. Referring to block 258, in some embodiments, the natural language processing includes inputting at least a portion of the unstructured clinical notes into a large language model.

While decent signal can be achieved through the aggregation of label functions, they tend to produce a limited set of labeled training data, i.e., having limited coverage. In some embodiments, to expand further the set of labels in the training dataset, weak supervision techniques are employed to train a machine learning model, *e.g.,* a natural language processing (NLP), Transformer-based, deep learning phenotype model. In some embodiments, this is an NLP model that learns to extract the same information found in the multimodal aggregated signals by looking exclusively at the clinical notes. Training of this model allows for matching of the results of the aggregated signal and phenotyping models yet produce significantly more coverage. For example, returning to Figure 4, while aggregation of the labeling functions in Figure 4C can lead to identification of various subpopulations of subjects with a medical condition, other subpopulations can be missed, as illustrated in Figure 4D. By training a machine learning model to identify instances of the medical condition using the weak labels generated by aggregating the plurality of labeling functions, these other subpopulations can be identified as illustrated in Figure 4E.

Accordingly referring to block 258, in some embodiments, method 200 also includes training a classification model for phenotyping subjects with respect to the medical condition using, for each respective subject in the first plurality of subjects, (i) the corresponding label as a dependent variable and (ii) second information from the corresponding medical record as independent variables.

Referring to block 260, in some embodiments, the classification model is a neural network, a support vector machine, a Naive Bayes model, a nearest neighbor model, a boosted trees model, a random forests model, or a clustering model.

Referring to block 262, in some embodiments, the second information includes at least two data types. Referring to block 264, in some embodiments, the second information includes structured electronic health record (EHR) data and unstructured EHR data.

Referring to block 266, in some embodiments, the second information includes at least three data types selected from structured electronic health record (EHR) data 42, unstructured EHR data 43, laboratory results 46, prescribed medications 44, and performed medical procedures 45. In some embodiments, the second information includes structured electronic health record (EHR) data 42, unstructured EHR data 43, and laboratory results 46. In some embodiments, the second information includes structured electronic health record (EHR) data 42, unstructured EHR data 43, and prescribed medications 44. In some embodiments, the second information includes structured electronic health record (EHR) data 42, unstructured EHR data 43, and performed medical procedures 45. In some embodiments, the second information includes structured electronic health record (EHR) data 42, laboratory results 46, and prescribed medications 44. In some embodiments, the second information includes structured electronic health record (EHR) data 42, laboratory results 46, and performed medical procedures 45. In some embodiments, the second information includes structured electronic health record (EHR) data 42, prescribed medications 44, and performed medical procedures 45. In some embodiments, the second information includes unstructured EHR data 43, laboratory results 46, and prescribed medications 44. In some embodiments, the second information includes unstructured EHR data 43, laboratory results 46, and performed medical procedures 45. In some embodiments, the second information includes unstructured EHR data 43, prescribed medications 44, and performed medical procedures 45. In some embodiments, the second information includes laboratory results 46, prescribed medications 44, and performed medical procedures 45.

In some embodiments, the second information includes at least four data types selected from structured electronic health record (EHR) data 42, unstructured EHR data 43, laboratory results 46, prescribed medications 44, and performed medical procedures 45. In some embodiments, the second information includes unstructured EHR data 43, laboratory results 46, prescribed medications 44, and performed medical procedures 45. In some embodiments, the second information includes structured electronic health record (EHR) data 42, laboratory results 46, prescribed medications 44, and performed medical procedures 45. In some embodiments, the second information includes structured electronic health record (EHR) data 42, unstructured EHR data 43, prescribed medications 44, and performed medical procedures 45. In some embodiments, the second information includes structured electronic health record (EHR) data 42, unstructured EHR data 43, laboratory results 46, and performed medical procedures 45. In some embodiments, the second information includes structured electronic health record (EHR) data 42, unstructured EHR data 43, laboratory results 46, and prescribed medications 44.

In some embodiments, the second information includes at least structured electronic health record (EHR) data 42, unstructured EHR data 43, laboratory results 46, prescribed medications 44, and performed medical procedures 45.

In some embodiments, the second information includes at least one of structured electronic health record (EHR) data 42, unstructured EHR data 43, laboratory results 46, prescribed medications 44, and performed medical procedures 45. In some embodiments, the second information includes at least laboratory results 46.

Referring to block 268, in some embodiments, the second information includes a data type that is not part of the first information. Referring to block 270, in some embodiments, the data type in the second information that is not part of the first information is laboratory results.

Referring to block 272, in some embodiments, the second information includes all or a portion of the first information.

Referring to block 274, in some embodiments, the medical condition is pulmonary hypertension and the second information includes electrocardiogram results.

Referring to block 274, in some embodiments, method 200 also includes identifying, from a plurality of previously undiagnosed subjects, one or more respective subjects having the medical condition by inputting, for each respective subject in the plurality of subjects, corresponding second information for the respective subject into the classification model to receive as output a corresponding indication of whether the respective subject has the medical condition.

Referring to block 276, in some embodiments, the plurality of subjects are associated with a medical service provider. For example, in some embodiments, the plurality of subjects are all associated with a medical insurance company. In some embodiments, the plurality of subjects are all associated with a medical provider (*e.g.,* a hospital or clinic) or chain of medical providers (*e.g.,* a hospital or clinic group).

Referring to block 278, in some embodiments, method 200 also includes predicting whether a test subject has the medical condition by inputting second information for the test subject into the classification model to receive as output an indication of whether the test subject has the medical condition.

In some embodiments, the methods described herein also include a step of generating a report for one or more phenotyped subject that includes the identified phenotype for the subject (*e.g.,* the presence or absence of PH). In some embodiments, the report further includes information about clinical trials for which the subject is eligible, therapies that are specific to the subject's medical condition, and/or possible therapeutic adverse effects associated with the specific characteristics of the subject's medical condition, *e.g.,* the subject's genetic variations, epigenetic abnormalities, associated oncogenic pathogenic infections, and/or pathology abnormalities, or other characteristics of the subject's sample and/or clinical records.

In some implementations, the method further includes using an identified phenotype to determine an eligibility of a subject for enrollment in a clinical trial.

In one aspect, the disclosure provides methods, systems, and non-transitory computer readable media for training a model for phenotyping subjects with respect to a medical condition. The method is performed at a computer system that includes one or more processors and memory. The method includes generating a plurality of labeling functions for the medical condition, where each respective labeling function in the plurality of labeling functions comprises a corresponding set of one or more criterion that, when satisfied, indicate a presence of the medical condition. The method includes assigning, to each respective subject in a first plurality of subjects, a corresponding label indicating a status of the medical condition by evaluating first information from a corresponding medical record for the respective subject using an ensemble model comprising the plurality of labeling functions to obtain as output from the ensemble model a prediction for the status of the medical condition, wherein the evaluating comprises natural language processing of at least a portion of the respective medical record.

## Digital and Laboratory Health Care Platform

In some embodiments, the methods and systems described herein are utilized in combination with, or as part of, a digital and laboratory health care platform that is generally targeted to medical care and research. It should be understood that many uses of the methods and systems described above, in combination with such a platform, are possible. One example of such a platform is described in U.S. Patent Publication No. 2021/0090694, titled "Data Based Cancer Research and Treatment Systems and Methods," and published March 25, 2021, the content of which is incorporated herein by reference, in its entirety, for all purposes.

For example, an implementation of one or more embodiments of the methods and systems as described above includes microservices constituting a digital and laboratory health care platform supporting analysis of electrocardiograms (ECGs) to provide clinical support for personalized cardiovascular care. Embodiments include a single microservice for executing and delivering analysis of ECGs to clinical support for personalized cardiovascular care and/or include a plurality of microservices each having a particular role, which together implement one or more of the embodiments above. In one example, a first microservice executes ECG analysis in order to deliver features to a second microservice for curating clinical support for personalized cardiovascular care based on the identified features. Similarly, the second microservice executes therapeutic analysis of the curated clinical support to deliver recommended therapeutic modalities, according to various embodiments described herein.

Where embodiments above are executed in one or more micro-services with or as part of a digital and laboratory health care platform, one or more of such micro-services, in some implementations, are part of an order management system that orchestrates the sequence of events as needed at the appropriate time and in the appropriate order necessary to instantiate embodiments above. A microservices-based order management system is disclosed, for example, in U.S. Patent Publication No. 2020/80365232, titled "Adaptive Order Fulfillment and Tracking Methods and Systems," and published November 19, 2020, the content of which is incorporated herein by reference, in its entirety, for all purposes.

For example, continuing with the above first and second microservices, an order management system, in some embodiments, notifies the first microservice that an order for curating clinical support for personalized cardiovascular care has been received and is ready for processing. In some embodiments, the first microservice executes and notifies the order management system once the delivery of ECG features for the patient is ready for the second microservice. Furthermore, the order management system identifies that execution parameters (prerequisites) for the second microservice are satisfied, including that the first microservice has completed, and notifies the second microservice that it may continue processing the order to curate clinical support for personalized cardiovascular care, according to various embodiments described herein.

Where the digital and laboratory health care platform further includes a genetic analyzer system, the genetic analyzer system, in some embodiments, includes targeted panels and/or sequencing probes. An example of a targeted panel is disclosed, for example, in U.S. Patent Publication No. 2021/0090694, titled "Data Based Cancer Research and Treatment Systems and Methods," and published March 25, 2021, which is incorporated herein by reference and in its entirety for all purposes. An example of a targeted panel for sequencing cell-free (cf) DNA and determining various characteristics of a specimen based on the sequencing is disclosed, for example, in U.S. Patent Application No. 17/179,086, titled "Methods And Systems For Dynamic Variant Thresholding In A Liquid Biopsy Assay," and filed February 18, 2021; U.S. Patent Application No. 17/179,267, titled "Estimation Of Circulating Tumor Fraction Using Off-Target Reads Of Targeted-Panel Sequencing," and filed February 18, 2021; and U.S. Patent Application No. 17/179,279, titled "Methods And Systems For Refining Copy Number Variation In A Liquid Biopsy Assay," and filed February 18, 2021, each of which is incorporated herein by reference and in its entirety for all purposes. In one example, targeted panels enable the delivery of next generation sequencing results for providing clinical support for personalized cancer therapy, according to various embodiments described herein. An example of the design of next-generation sequencing probes is disclosed, for example, in U.S. Patent Publication No. 2021/0115511, titled "Systems and Methods for Next Generation Sequencing Uniform Probe Design," and published June 22, 2021; and U.S. Patent Application No. 17/323,986, titled "Systems and Methods for Next Generation Sequencing Uniform Probe Design," and filed May 18, 2021, each of which is incorporated herein by reference and in its entirety for all purposes.

Where the digital and laboratory health care platform further includes an epigenetic analyzer system, the epigenetic analyzer system, in some embodiments, analyzes specimens to determine their epigenetic characteristics and further uses that information for monitoring a patient over time. An example of an epigenetic analyzer system is disclosed, for example, in U.S. Patent Application No. 17/352,231, titled "Molecular Response and Progression Detection From Circulating Cell Free DNA," and filed June 18, 2021, which is incorporated herein by reference and in its entirety for all purposes.

Where the digital and laboratory health care platform further includes a bioinformatics pipeline, the methods and systems described above, in some embodiments, are utilized after completion or substantial completion of the systems and methods utilized in the bioinformatics pipeline. As one example, the bioinformatics pipeline receives next-generation genetic sequencing results and returns a set of binary files, such as one or more BAM files, reflecting nucleic acid (e.g., cfDNA, DNA and/or RNA) read counts aligned to a reference genome. The methods and systems described above, in some embodiments, are utilized, for example, to ingest the cfDNA, DNA and/or RNA read counts and produce genomic features as a result.

When the digital and laboratory health care platform further includes an RNA data normalizer, in some embodiments, any RNA read counts are normalized before processing embodiments as described above. An example of an RNA data normalizer is disclosed, for example, in Publication No. 2020/0098448, titled "Methods of Normalizing and Correcting RNA Expression Data," and published March 26, 2020, which is incorporated herein by reference and in its entirety for all purposes.

When the digital and laboratory health care platform further includes a genetic data deconvolver, in some embodiments, any system and method for deconvoluting is utilized for analyzing genetic data associated with a specimen having two or more biological components to determine the contribution of each component to the genetic data and/or determine what genetic data would be associated with any component of the specimen if it were purified. An example of a genetic data deconvolver is disclosed, for example, in U.S. Patent Publication No. 2020/0210852, published July 2, 2020, and PCT/US19/69161, filed December 31, 2019, both titled "Transcriptome Deconvolution of Metastatic Tissue Samples"; and U.S. Patent Application No. 17/074,984, titled "Calculating Cell-type RNA Profiles for Diagnosis and Treatment," and filed October 20, 2020, the content of each of which is incorporated herein by reference, in its entirety, for all purposes.

When the digital and laboratory health care platform further includes an automated RNA expression caller, in some embodiments, RNA expression levels are adjusted to be expressed as a value relative to a reference expression level, which is often done in order to prepare multiple RNA expression data sets for analysis to avoid artifacts caused when the data sets have differences because they have not been generated by using the same methods, equipment, and/or reagents. An example of an automated RNA expression caller is disclosed, for example, in U.S. Patent No. 11,043,283, titled "Systems and Methods for Automating RNA Expression Calls in a Cancer Prediction Pipeline," and issued June 22, 2021, which is incorporated herein by reference and in its entirety for all purposes.

In some embodiments, RNA expression levels are adjusted to be expressed as a value relative to a reference expression level. Furthermore, multiple RNA expression data sets, in some embodiments, are adjusted, prepared, and/or combined for analysis and/or adjusted to avoid artifacts caused when the data sets have differences because they have not been generated by using the same methods, equipment, and/or reagents. An example of RNA data set adjustment, preparation, and/or combination is disclosed, for example, in U.S. Patent Application No. 17/405,025, titled "Systems and Methods for Homogenization of Disparate Datasets," and filed August 18, 2021, which is hereby incorporated herein by reference in its entirety.

The digital and laboratory health care platform, in some embodiments, further includes one or more insight engines to deliver information, characteristics, or determinations related to a disease state that can be based on genetic and/or clinical data associated with a patient and/or specimen. Exemplary insight engines include, without limitation, a tumor of unknown origin engine, a human leukocyte antigen (HLA) loss of homozygosity (LOH) engine, a tumor mutational burden engine, a PD-L1 status engine, a homologous recombination deficiency engine, a cellular pathway activation report engine, an immune infiltration engine, a microsatellite instability engine, a pathogen infection status engine, a T cell receptor or B cell receptor profiling engine, a line of therapy engine, a metastatic prediction engine, and/or an IO progression risk prediction engine, and so forth. An example tumor of unknown origin engine is disclosed, for example, in U.S. Patent Application No. 15/930,234, titled "Systems and Methods for Multi-Label Cancer Classification," and filed May 12, 2020, which is incorporated herein by reference and in its entirety for all purposes. An example of an HLA LOH engine is disclosed, for example, in U.S. Patent No. 11,081,210, titled "Detection of Human Leukocyte Antigen Class I Loss of Heterozygosity in Solid Tumor Types by NGS DNA Sequencing," and issued August 3, 2021, which is incorporated herein by reference and in its entirety for all purposes. An additional example of an HLA LOH engine is disclosed, for example, in U.S. Patent App. No. 17/304,940, titled "Detection of Human Leukocyte Antigen Loss of Heterozygosity," and filed June 28, 2021, which is incorporated herein by reference and in its entirety for all purposes. An example of a tumor mutational burden (TMB) engine is disclosed, for example, in U.S. Patent Publication No. 2020/0258601, titled "Targeted-Panel Tumor Mutational Burden Calculation Systems and Methods," and published August 13, 2020, which is incorporated herein by reference and in its entirety for all purposes. An example of a PD-L1 status engine is disclosed, for example, in U.S. Patent Publication No. 2020/0395097, titled "A Pan-Cancer Model to Predict The PD-L1 Status of a Cancer Cell Sample Using RNA Expression Data and Other Patient Data," and published December 17, 2020, which is incorporated herein by reference and in its entirety for all purposes. An additional example of a PD-L1 status engine is disclosed, for example, in U.S. Patent No. 10,957,041, titled "Determining Biomarkers from Histopathology Slide Images," issued March 23, 2021, which is incorporated herein by reference and in its entirety for all purposes. An example of a homologous recombination deficiency engine is disclosed, for example, in U.S. Patent No. 10,975,445, titled "An Integrative Machine-Learning Framework to Predict Homologous Recombination Deficiency," and issued April 13, 2021, which is incorporated herein by reference and in its entirety for all purposes. An additional example of a homologous recombination deficiency engine is disclosed, for example, in U.S. Patent App. No. 17/492,518, titled "Systems and Methods for Predicting Homologous Recombination Deficiency Status of a Specimen," filed October 1, 2021, which is incorporated herein by reference and in its entirety for all purposes. An example of a cellular pathway activation report engine is disclosed, for example, in U.S. Patent Publication No. 2021/0057042, titled "Systems And Methods For Detecting Cellular Pathway Dysregulation In Cancer Specimens," and published February 25, 2021, which is incorporated herein by reference and in its entirety for all purposes. An example of an immune infiltration engine is disclosed, for example, in U.S. Patent Publication No. 2020/0075169, titled "A Multi-Modal Approach to Predicting Immune infiltration Based on Integrated RNA Expression and Imaging Features," and published March 5, 2020, which is incorporated herein by reference and in its entirety for all purposes. An example of an MSI engine is disclosed, for example, in U.S. Patent Publication No. 2020/0118644, titled "Microsatellite Instability Determination System and Related Methods," and published April 16, 2020, which is incorporated herein by reference and in its entirety for all purposes. An additional example of an MSI engine is disclosed, for example, in U.S. Patent Publication No. 2021/0098078, titled "Systems and Methods for Detecting Microsatellite Instability of a Cancer Using a Liquid Biopsy," and published April 1, 2021, which is incorporated herein by reference and in its entirety for all purposes. An example of a pathogen infection status engine is disclosed, for example, in U.S. Patent No. 11,043,304, titled "Systems And Methods For Using Sequencing Data For Pathogen Detection," and issued June 22, 2021, which is incorporated herein by reference and in its entirety for all purposes. Another example of a pathogen infection status engine is disclosed, for example, in PCT/US21/18619, titled "Systems And Methods For Detecting Viral DNA From Sequencing," and filed February 18, 2021, which is incorporated herein by reference and in its entirety for all purposes. An example of a T cell receptor or B cell receptor profiling engine is disclosed, for example, in U.S. Patent Application No. 17/302,030, titled "TCR/BCR Profiling Using Enrichment with Pools of Capture Probes," and filed April 21, 2021, which is incorporated herein by reference and in its entirety for all purposes. An example of a line of therapy engine is disclosed, for example, in U.S. Patent Publication No. 2021/0057071, titled "Unsupervised Learning And Prediction Of Lines Of Therapy From High-Dimensional Longitudinal Medications Data," and published February 25, 2021, which is incorporated herein by reference and in its entirety for all purposes. An example of a metastatic prediction engine is disclosed, for example, in U.S. Patent No. 11,145,416, titled "Predicting likelihood and site of metastasis from patient records," and issued October 12, 2021, which is incorporated herein by reference and in its entirety for all purposes. An example of an IO progression risk prediction engine is disclosed, for example, in U.S. Patent Application No. 17/455,876, titled "Determination of Cytotoxic Gene Signature and Associated Systems and Methods For Response Prediction and Treatment," and filed November 19, 2021, which is incorporated herein by reference and in its entirety for all purposes.

In some embodiments, any data generated by the systems and methods and/or the digital and laboratory health care platform is downloadable by the user. In one example, the data is downloaded as a CSV file comprising clinical and/or molecular data associated with tests, data structuring, and/or other services ordered by the user. In various embodiments, this is accomplished by aggregating clinical data in a system backend and making it available via a portal. This data, in some embodiments, includes not only variants and RNA expression data, but also data associated with immunotherapy markers such as MSI and TMB, as well as RNA fusions.

When the digital and laboratory health care platform further includes a device comprising a microphone and speaker for receiving audible queries or instructions from a user and delivering answers or other information, in some embodiments, the methods and systems described above are utilized to add data to a database the device can access. An example of such a device is disclosed, for example, in U.S. Patent Publication No. 2020/0335102, titled "Collaborative Artificial Intelligence Method And System," and published October 22, 2020, which is incorporated herein by reference and in its entirety for all purposes.

In some embodiments, when the digital and laboratory health care platform further includes a mobile application for ingesting patient records, including genomic sequencing records and/or results even if they were not generated by the same digital and laboratory health care platform, the methods and systems described above are utilized to receive ingested patient records. An example of such a mobile application is disclosed, for example, in U.S. Patent No. 10,395,772, titled "Mobile Supplementation, Extraction, And Analysis Of Health Records," and issued August 27, 2019, which is incorporated herein by reference and in its entirety for all purposes. Another example of such a mobile application is disclosed, for example, in U.S. Patent No. 10,902,952, titled "Mobile Supplementation, Extraction, And Analysis Of Health Records," and issued January 26, 2021, which is incorporated herein by reference and in its entirety for all purposes. Another example of such a mobile application is disclosed, for example, in U.S. Patent Publication No. 2021/0151192, titled "Mobile Supplementation, Extraction, And Analysis Of Health Records," and filed May 20, 2021, which is incorporated herein by reference and in its entirety for all purposes.

When the digital and laboratory health care platform further includes a report generation engine, in some embodiments, the methods and systems described above are utilized to create a summary report of a patient's ECG results and/or genetic profile and the results of one or more insight engines for presentation to a physician. For instance, where the digital and laboratory health care platform is used for providing clinical support for personalized cancer therapy, in some embodiments, the report provides, to the physician, information about the extent to which the specimen that was sequenced contained tumor or normal tissue from a first organ, a second organ, a third organ, and so forth. For example, the report provides, in some implementations, a genetic profile for each of the tissue types, tumors, or organs in the specimen. In some embodiments, the genetic profile represents genetic sequences present in the tissue type, tumor, or organ and includes variants, expression levels, information about gene products, or other information that could be derived from genetic analysis of a tissue, tumor, or organ.

In some embodiments, the report includes therapies and/or clinical trials matched based on a portion or all of the ECG, electronic health record, genetic profile, or insight engine findings and summaries. For example, in some embodiments, the therapies are matched according to the systems and methods disclosed in U.S. Patent Application No. 17/546,049, titled "Artificial Intelligence Driven Therapy Curation and Prioritization," filed December 9, 2021, which is incorporated herein by reference and in its entirety for all purposes. For example, the clinical trials are matched, in some embodiments, according to the systems and methods disclosed in U.S. Patent Publication No. 2020/0381087, titled "Systems and Methods of Clinical Trial Evaluation," published December 3, 2020, which is incorporated herein by reference and in its entirety for all purposes.

In some embodiments, the report includes a comparison of the results (for example, molecular and/or clinical patient data) to a database of results from many specimens. Example methods and systems for comparing results to a database of results are disclosed in U.S. Patent Publication No. 2020/0135303 titled "User Interface, System, And Method For Cohort Analysis" and published April 30, 2020, and U.S. Patent Publication No. 2020/0211716 titled "A Method and Process for Predicting and Analyzing Patient Cohort Response, Progression and Survival," and published July 2, 2020, each of which is incorporated herein by reference and in its entirety for all purposes. The information, in some embodiments, is used, sometimes in conjunction with similar information from additional specimens and/or clinical response information, to match therapies likely to be successful in treating a patient, discover biomarkers or design a clinical trial.

When the digital and laboratory health care platform further includes organoids developed in connection with the platform (for example, from the patient specimen), in some embodiments, the methods and systems are used to further evaluate genetic sequencing data derived from an organoid and/or the organoid sensitivity, especially to therapies matched based on a portion or all of the information determined by the systems and methods, including predicted cancer type(s), likely tumor origin(s), etc. In some embodiments, these therapies are tested on the organoid, derivatives of that organoid, and/or similar organoids to determine an organoid's sensitivity to those therapies. In some embodiments, any of the results are included in a report. If the organoid is associated with a patient specimen, in some embodiments, any of the results are included in a report associated with that patient and/or delivered to the patient or patient's physician or clinician. In various examples, organoids are cultured and tested according to the systems and methods disclosed in U.S. Patent Publication No. 2021/0155989, titled "Tumor Organoid Culture Compositions, Systems, and Methods," published May 27, 2021; PCT/US20/56930, titled "Systems and Methods for Predicting Therapeutic Sensitivity," filed October 22, 2020; U.S. Patent Publication No. 2021/0172931, titled "Large Scale Organoid Analysis," published June 10, 2021; PCT/US2020/063619, titled "Systems and Methods for High Throughput Drug Screening," filed December 7, 2020 and U.S. Patent Application No. 17/301,975, titled "Artificial Fluorescent Image Systems and Methods," filed April 20, 2021, each of which are incorporated herein by reference and in their entirety for all purposes. In one example, the drug sensitivity assays are especially informative if the systems and methods return results that match with a variety of therapies, or multiple results (for example, multiple equally or similarly likely cancer types or tumor origins), each matching with at least one therapy.

When the digital and laboratory health care platform further includes application of one or more of the above in combination with or as part of a medical device or a laboratory developed test that is generally targeted to medical care and research, in some embodiments, such laboratory developed test or medical device results are enhanced and personalized through the use of artificial intelligence. An example of laboratory developed tests, especially those that can be enhanced by artificial intelligence, is disclosed, for example, in U.S. Patent Publication No. 2021/0118559, titled "Artificial Intelligence Assisted Precision Medicine Enhancements to Standardized Laboratory Diagnostic Testing," and published April 22, 2021, which is incorporated herein by reference and in its entirety for all purposes.

It should be understood that the examples given above are illustrative and do not limit the uses of the systems and methods described herein in combination with a digital and laboratory health care platform.

In some embodiments, the results of the bioinformatics pipeline are provided for report generation. Report generation includes, in some embodiments, variant science analysis, including the interpretation of variants (including somatic and germline variants as applicable) for pathogenic and biological significance. In some embodiments, the variant science analysis also estimates microsatellite instability (MSI) or tumor mutational burden. Targeted treatments are, in some embodiments, identified based on gene, variant, and cancer type, for further consideration and review by the ordering physician. In some aspects, clinical trials are, in some embodiments, identified for which the patient may be eligible, based on mutations, cancer type, and/or clinical history. In some embodiments, subsequent validation occurs, after which the report may be finalized for sign-out and delivery. In some embodiments, a first or second report includes additional data provided through a clinical dataflow 202, such as patient progress notes, pathology reports, imaging reports, and other relevant documents. Such clinical data is ingested, reviewed, and abstracted based on a predefined set of curation rules. The clinical data is then populated into the patient's clinical history timeline for report generation.

Further details on clinical report generation are disclosed in US Patent Application No. 16/789,363 (PCT/US20/180002), filed February 12, 2020, the content of which is incorporated herein by reference, in its entirety, for all purposes.

**EXAMPLES.**

***Example 1 - Evaluation of the Method for training a model to identify patients with undiagnosed pulmonary hypertension.***

Pulmonary hypertension (PH) is a progressive, debilitating disease for which there are increasing treatment options. Data-driven techniques to find patients eligible for therapy may help optimize population-level therapeutic benefits. This examples seeks to determine whether patients with PH could be accurately identified using structured electronic health record (EHR) data.

Briefly, using a randomly sampled cohort of patients with PH-relevant structured data or unstructured note mentions from a large EHR dataset of over 2 million patients, patients were identified with clinically diagnosed PH using blinded, two-clinician manual review of de-identified patient records. The availability of structured data elements was then characterized, including any occurrence of a PH diagnosis code in any encounter or problem list, any order or medication reconciliation of a PH medication (treprostinil, epoprostenol, iloprost, selexipag, riociguat, ambrisentan, bosentan, macitentan, sildenafil, tadalafil, and their corresponding brand names), or any right heart catheterization (RHC) with mean PA pressure greater than 20 mm Hg. Also characterized were how many patients had echocardiography measurements of tricuspid regurgitation max velocity of greater than 3.4 m/s or max pressure gradient of greater than 46 mm Hg.

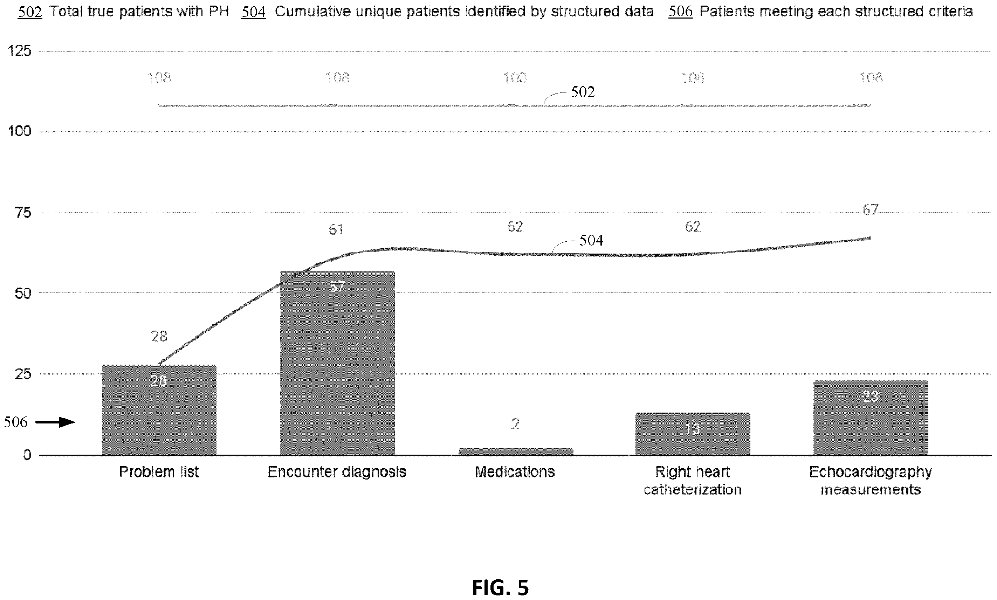

In total 108 patients with clinically diagnosed PH were identified, of which 43% did not have any structured data elements suggestive of PH. Only 26% of patients had a PH code on their problem list, 53% had a PH encounter diagnosis code, 2% were on a PH medication, and 12% had an RHC finding consistent with PH (Figure 1). Adding echocardiography measurements, less commonly available as a structured field, resulted in the identification of 5 additional patients. In total, 38% of patients did not have any EHR data suggestive of PH yet could be identified as having clinically diagnosed PH using note text. This data is shown in Figure 5.

Structured data elements, such as those available in EHR data, only identify a subset of patients with true pulmonary hypertension. Unstructured data such as clinical notes may be needed to better identify cohorts of patients with PH.

***Example 2 - Method for phenotyping electronic health records with respect to pulmonary hypertension.***

To extend the analysis performed in Example 1, it was suggested that a plurality of labeling functions could be developed to automate weak labeling of medical records, in order to ultimately train a machine learning model to identify patients with undiagnosed pulmonary hypertension (PH) based on electrocardiogram (ECG) data. This will help to better identify patients with pulmonary hypertension because deep learning models can learn signals from the ECG based on pathophysiologic effects that humans cannot detect. However, a large number of ECG data sets are needed in order to train such a model without a priori knowledge of what ECG signals indicate pulmonary hypertension. While electronic health records for patients with available ECG data can be used to label such ECG data for model training, manual evaluation of such medical records is time consuming and subjective. To address this challenge, high-quality labels were generated for downstream ECG model training using aggregated labeling functions reflecting structured and/or NLP/ML phenotypes.

As a first step, exploratory analysis of an electronic health record database for incidence of PH was performed. First, the database was searched for records of right heart catheterization (RHC) procedures over the past 10 years. RHC is a diagnostic procedure used to measure pulmonary artery pressures and thus evaluate whether a patient has pulmonary hypertension or not. Within this data, PH was defined as an elevated mean pulmonary artery pressure (mPAP) of at least 25 mmHg at rest. Unique patients meeting this criteria were identified. Next, the database was searched for records of PH diagnosis using International Classification of Diseases (ICD) medical codes ICD-9 416 (chronic pulmonary heart disease) and ICD-10 127 (primary pulmonary hypertension). Tens of thousands of unique patients meeting this criteria were identified. Of the thousands of patients with records indicating an elevated mPAP of at least 25 mmHg at rest, more than a third of them did not have a diagnosis for PH in their medical record. Of the tens of thousands of patients with PH diagnosis codes in their medical records, more than 90% of them did not have elevated mPAP test results of at least 25 mmHg at rest in their medical records. Thus, phenotyping medical records based on either of these indications of PH would result in a significant number of false negatives.

Further, exploration of the EHRs for the thousands of patients with elevated mPAP of at least 25 mmHg at rest revealed a broad array of other cardiovascular disease (CVD) symptoms and diagnoses, as shown in Table 1 below.

**Table 1** - CVD symptoms and diagnoses in medical records of undiagnosed PH patients with elevated mPAP of at least 25 mmHg at rest.

| Diagnosis | |
| --- | --- |
| Atherosclerotic heart disease of native coronary artery without angina pectoris | |
| Hyperlipidemia, unspecified | |
| Essential (primary) hypertension | |
| HTN, goal below 140/90 | |
| Unspecified essential hypertension | |
| Acute kidney failure, unspecified | |
| Other and unspecified hyperlipidemia | |
| HTN, goal below 130/80 | |
| MyCode Research Other*N2006G0258 | |
| Personal history of nicotine dependence | |
| Long term (current) use of anticoagulants | |
| Need for prophylactic vaccination and inoculation against influenza | |
| Coronary atherosclerosis of native coronary artery | |
| Anticoagulation management encounter | |
| Coronary artery disease involving native coronary artery of native heart without angina pectoris | |
| Cough | |
| Acute posthemorrhagic anemia | |
| Shortness of breath | |
| Special screening for malignant neoplasms, colon | |
| Need for pneumococcal vaccination | |
| Auto Orders Authorization | |
| Dyslipidemia, goal to be determined | |
| Other specified pre-operative examination | |
| Type 2 diabetes mellitus without complications | |
| Dyslipidemia, goal LDL below 70 | |

(continued)

| Diagnosis | |
|---|---|
| HTN, goal to be determined | |
| DM type 2, goal A1c below 7 | |
| Dyslipidemia, goal LDL below 100 | |
| Anemia | |

As a first step, the plurality of medical records in the database were split into training (80%), validation (10%), and test (10%) sets. Each set was then filtered in accordance with a first set of characteristics for PH. The filtering included applying an extraction filter to the medical records, to obtain a subplurality of medical records. The extraction filter evaluated each medical record for a plurality of inclusion criteria and a plurality exclusion criteria. When the extraction filter did not identify an exclusion criteria and identified at least one inclusion criteria, the medical record was selected for inclusion in the subplurality of records. When the extraction filter identified at least one exclusion criteria and/or did not identify at least one inclusion criteria, the medical record was excluded from the subplurality of records. The inclusion criteria included (i) a medical code for a PH diagnosis (e.g., ICD-9 416 or ICD-10 127), (ii) a prescription for a medication used to treat PH, (iii) an RHC procedure code, (iv) an echocardiogram result consistent with PH (e.g., a tricuspid regurgitation (TR) maximum velocity of greater than 3.4 m/s or a tricuspid regurgitation peak gradient (TRPG) of greater than 46 mmHg), (v) a mention of PH within an echocardiograph report, and (vi) PH mention within unstructured clinical notes (*e.g.,* regex identification of a PH term such as 'pulmonary hypertension', 'pulm HTN', 'PAH', 'pHTN', 'PHT', or 'pulmonary arterial hypertension,' of regex identification of a broad term such as 'PH', 'PASP', 'PAP', 'pulmonary artery', 'right ventricular pressure', or 'septal flattening', excluding non-relevant terms such as 'PhD', 'PH urine', 'PH arterial', *etc*.). The exclusion criteria included (i) patient age of 18 years or less, and (ii) no completed medical provider encounter (*e.g.,* inpatient, outpatient, or telemedicine). The filtering reduced the total of number of patient records by at least a factor of 10, from millions to hundreds of thousands.

Development of the filter criteria was an iterative process based on consultations with clinicians, review of literature on PH, data analysis, and review of medical records excluded by the filter in earlier versions of the filter. The goal was to minimize the loss of sensitivity due to the exclusion of PH patients from the subplurality of records, while increasing the prevalence of PH within the subplurality of records, to enable unbiased sampling and metrics. As a starting point, inclusion criteria (i) a medical code for a PH diagnosis (*e.g.,* ICD-9 416 or ICD-10 127), (ii) a prescription for a medication used to treat PH, and a subset of (vi) PH mention within unstructured clinical notes were used to filter the dataset, along with the exclusion criteria. The addition of inclusion criteria (iv) an echocardiogram result consistent with PH, added patients. Addition of inclusion criteria (v) a mention of PH within an echocardiograph report, added patients. Addition of inclusion criteria (iii) an RHC procedure code, added patients. Finally, expansion of inclusion criteria (vi) PH mention within unstructured clinical notes to include the terms 'PHCOPD', 'CTEPH', and 'PHILD' added patients. The patient population included by the filter criteria could be further expanded by running a natural language processing (NLP) and/or machine learning model on excluded patients to identify those most likely to have PH.

Next, a series of structured phenotypes for PH were developed that each indicate the presence of PH, based on consultations with clinicians, review of literature on PH, data analysis. The first version of structured phenotypes included:

- 2+ encounter diagnoses (completed encounters with provider only, not lab visits or imaging encounters or phone calls) of PH ICD diagnosis codes, [OR]

- 1 problem list occurrence (excluding problem list entries which were entered and removed on the same day) of PH diagnosis codes, [OR]

- RHC mean pulmonary artery pressure of greater than 20 mm Hg, [OR]

- PH medication use in medication orders or medication recommendations for completed encounter, provided that sildenafil was only included when used or recommended TID (three times daily) and not on demand (PRN) or 'before' use and that tadalafil was only included at 20 mg or 40 mg doses.

100 training samples were independently reviewed by two clinicians who discussed and reached consensus on criteria to use to identify PH and agreed that no reinterpretation of the clinical judgment should be made during the review. After reviewing ten initial medical records together, the reviewers independently reviewed overlapping records to allow for concordance to be assessed between the phenotyping. There was concordance between the reviewers on 51 of 52 overlapping cases. The discordant case was reviewed and adjudicated together by the reviewers. Additionally, the reviewers identified when instances of PH were initially diagnosed.

All of the training samples were then phenotyped by computer review using the first version of the structured phenotypes, shown above. In this review, identification of a single structured phenotype was sufficient to phenotype a medical record as PH. Tens of thousands of medical records were phenotyped as PH. Dates associated with relevant clinical phenotypes were used to predict an initial diagnosis data. Briefly, a tiered system of evidence was used to find the first relevant date. For each subject identified as having the medical condition, evidence types (relevant diagnosis codes, note mention, medications, *etc*.) are sorted into tiers. The first evidence date is selected as the initial diagnosis date in cascading fashion, *e.g.,* if no tier 1 evidence is available, then look for the first tier 2 evidence date, otherwise tier 3 evidence, etc. For example, tier 1 evidence may be right-heart catheterization data, since it is most robust. Whereas tier 2 evidence may be echo mentions and tier 3 may be note mentions, etc.

Concordance of this automated phenotyping with clinical review was evaluated using the 100 training samples that had been independently reviewed. The results showed high specificity but low sensitivity and F1 score.

Medical records corresponding to false positives and false negatives, using clinician review as ground truth, were reviewed to determine why the structured phenotypes mislabeled these records.

One false positive was mislabeled because the chart indicated sildenafil was being recommended for TID use but was actually being used for treatment of erectile dysfunction. Another false positive was mislabeled because the epoprostenol reported on medicine orders was being used for hypoxia/ARDS. Another false positive was mislabeled because, while the patient had a past medical history of ventricular septal defect and mitral valve prolapse, there was no indication that the patient had experienced PH, even on echocardiograms.

Of the false negatives, approximately half would have been correctly labeled if the criteria was lowered to a single clinical encounter, approximately one-third of which would have been correctly labeled if only the inpatient criteria was lowered to 1 clinical encounter and the outpatient criteria was maintained at 2 encounters. Almost all of the false negative medical records had a mention of PH in the unstructured clinical notes.

It was found on chart review that approximately 37.5% of patients phenotyped with PH did not have any codes or medications to suggest PH but did have evidence in the clinical notes. This translated into a prevalence in the initial training set of 33% (95% CI: 24%-43%), even using a conservative definition of true positive. Assuming that no further positive cases were present in the records filtered out at the first stage, this results in an estimate of 2.43% PH in the general population, which significantly exceeds the literature estimate of about 1% occurrence in the general population.

To try and improve the labeling, the structured phenotypes were adjusted by (i) reducing the inpatient criteria to 1 clinical encounter, while maintaining the outpatient criteria at two clinical encounters (adding patients), (ii) removing epoprostenol from the medical orders criteria (removing patients), and (iii) removing erectile dysfunction medication from the medicine recommendation criteria (removing patients).

Version 2 of the structured phenotypes was then used to label the training set of medical records. Again, identification of a single structured phenotype was sufficient to phenotype a medical record as PH. Tens of thousands of medical records were phenotyped as PH. Dates associated with relevant clinical phenotypes were used to predict an initial diagnosis data. Concordance of this automated phenotyping with clinical review was evaluated using the training samples that had been independently reviewed. The results showed slightly higher sensitivity and F1 scores than F1, but both were still low.

The V1 and V2 structured phenotypes were then used to phenotype approximately 200 medical records in the validation set, using the same criteria that the presence of a single structured phenotype was sufficient to label the medical record as PH. Independent clinician review of the approximately 200 validation samples was performed as described above for the test set. Concordance of the automated phenotyping with clinical review was evaluated using the independently reviewed validation samples. The statistics for this phenotyping, using the clinical-reviewed phenotypes as ground truths, shown in Table 2 below, maintained high specificity and had slightly higher sensitivity and F1 scores.

Finally, the independently reviewed validation set of medical records were evaluated using a probabilistic graphical modeling model of a plurality of labeling functions. Briefly, the results obtained from evaluating each structured phenotype, either indicating PH or not, were aggregated using the PGM model, as described further in **Example 4.** Concordance of the automated phenotyping using the aggregated labeling functions with clinical review was evaluated against the results of the independent review of the validation samples described above. The statistics for this phenotyping, using the clinical-reviewed phenotypes as ground truths, as shown in Table 2 below, significantly improve sensitivity and F1 score, while maintaining high specificity.

**Table 2** - Concordance of automated review of validation medical records using the v1 structured phenotypes, v2 structured phenotypes, or aggregated v1 structured phenotypes with clinician review.

|  | PPV | NPV | Sensitivity | Specificity | F1 |
|---|---|---|---|---|---|
| **Structured V1** | 0.92 | 0.98 | 0.45 | 0.99 | 0.60 |

(continued)

| | PPV | NPV | Sensitivity | Specificity | F1 |
|---|---|---|---|---|---|
| Structured V2 | 0.91 | 0.98 | 0.54 | 0.99 | 0.68 |
| Aggregated V1 | 0.80 | 0.99 | 0.78 | 0.99 | 0.79 |

### Example 3 - Method for training a model to identify patients with undiagnosed pulmonary hypertension.

Using the weak phenotyped labels generated in **Example 3,** a neural network model was trained to identify pulmonary hypertension from ECG data. Briefly, the assigned labels were used as dependent variables and electrocardiogram (ECG) data was used as independent variables, to predict whether a patient has PH or not. Standard procedures for backpropagation were used during training.

The labels (PH or no PH) assigned to each medical record in **Example 2** can be further used to train a model based on data in the medical records. For example, in one embodiment, a machine learning model is trained by using the assigned labels as dependent variables and electrocardiogram (ECG) data as independent variables, to predict whether a patient has PH or not.

In a related embodiment, a machine learning model is trained by using the assigned labels as dependent variables and unstructured text from clinical notes in the medical record to predict whether a patient has PH or not.

In yet other embodiments, the assigned labels are used as dependent variables and any combination of multimodal data from an electronic health record can be used to train a model for predicting whether a patent has PH or not. Examples of multimodal data that can be used includes, without limitation, structured data in an electronic health record (EHR), unstructured data in an EHR, laboratory results, prescribed and/or recommended medications, and performed medical procedures.

### Example 4 - Probabilistic graphical modeling approach to weak supervision - majority vote simplification.

#### Goal

Solving the general probabilistic graphical modeling (PGM) model for the weak supervision problem is challenging, requiring simplifications and approximations to estimate in reasonable computational time. Conceptually, this Example starts from a simple version of this PGM, in order to build up more complex models from this work.

#### Problem Statement

It is assumed the general population has a prevalence of a given medical disorder (*e.g.*, PH), and that access to an EHR system comprising $N$ samples from this population is available. Each sample either does or does not have the medical disorder, as denoted by a binary variable $l_{i \in (0,N)}$. As described above, there are K independent labeling functions (LFs), which attempt to classify each sample as having the medical disorder or not through a binary voting variable $\lambda_{ij}$.

Importantly, these LFs are not expected to be perfect. Instead, each LF will perform differently, as defined by a true positive rate $s_j = P(\lambda_j = 1 | l = 1)$ and a false positive rate $\bar{s}_j = P(\lambda_j = 1 | l = 0)$. In fact, some LFs will attempt to classify label samples and will therefore vote when they think a sample does not have the disease. For these LFs, then, the value of $\bar{s}_j$ should be higher than $s_j$. It does not need to be explicitly specified which class a given LF is voting for, since it will be inferred as a latent variable, but it can be implied through the choice of priors for $s_j$ and $\bar{s}_j$.

#### Variables Being Solved For

Instead of handling the general problem of using all votes data across the entire sample to inform the inference, a "no-data" version of this model is used where only a single sample is considered. In this case, then, the problem solves for the posterior of this sample's label given the LF votes on this sample $P(l | \lambda_0, ..., \lambda_K)$. The subscript is dropped for the remainder of this text, since single-sample (*e.g.*, "no data") models are being explored here.

#### Majority Vote Model

A "majority vote" assumption is made, where it is assumed:

1. The LFs can be split into two sets: a "positive" set of size $K_+$ (*e.g.,* LFs trying to identify positive samples) and a "negative" set of size $K_-$ (*e.g.,* LFs trying to identify negative samples).

2. Each LF has equivalent performance compared to the label they vote for.

Note that, in some embodiments, a negative is a not positive, *e.g.,* merely indicates that the subject cannot be phenotyped as having the target medical condition. Similarly, in some embodiments, a negative is affirmation that the subject does not have the target medical condition. Accordingly, in some embodiments, the negative set can include both types of negative labeling functions.

Since the "no data" domain is being considered here, it is assumed that there is no good reason to prefer one LF to another and no way to reason about their respective performances. This is very much the sort of assumption underlying a pure majority vote solution, where it only matters which set of LFs (positive or negative) has more votes without regard to individual performances, *e.g.,* each vote is weighted equally. Assumption 2 above, which underlies both traditional majority vote methods and this model, is unlikely to hold in general and it will be relaxed in a later section.

Taking this assumption simplifies the PGM significantly and ends up such that decisions made from this model (*e.g.,* at p > 0.5) will generally amount to the same end result as taking a majority vote but provide reasonable probability scores instead of a binary decision.

### Specifying the Statistical Model

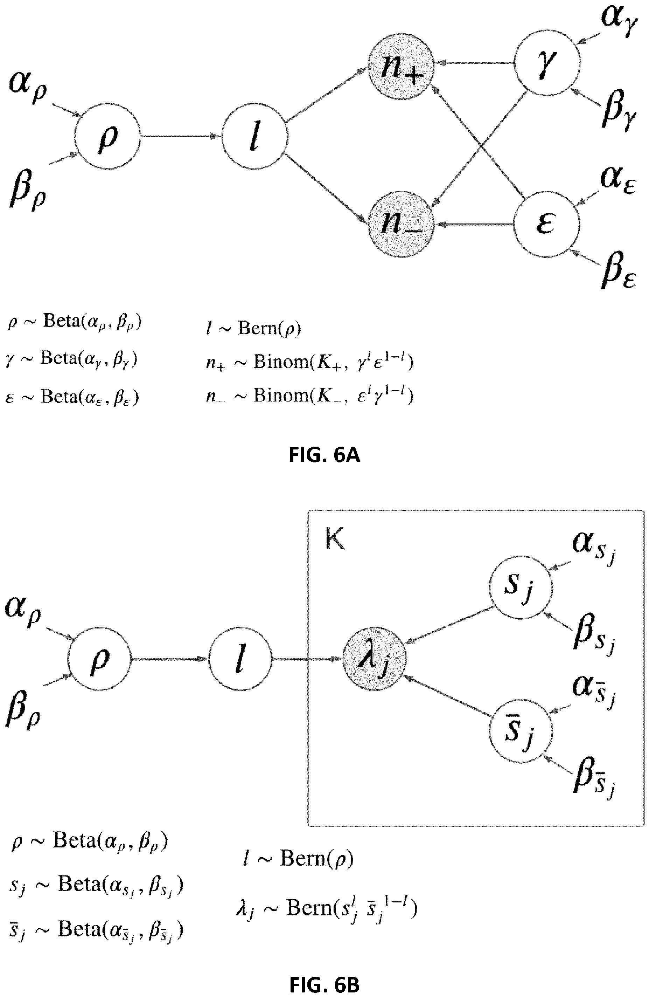

Observables are defined as $n_+$ (the number of "positive" LF votes) and $n_-$ (the number of "negative" LF votes). Since equivalent performance for all of these LFs is assumed, let $\gamma$ denote an LF's true positive rate (compared to the label it is voting for) and $\varepsilon$ its false positive rate. Note here that $\gamma$ for a "positive" LF is $P(\lambda = 1|l = 1)$ whereas for a "negative" LF it is instead $P(\lambda = 1|l = 0)$. This is purely for notational convenience at this point, and the model is generalized to different performances, the more general s and $\bar{s}$ notation described above will be used. The PGM for this model is shown in **Figure 6A**.

### Solving for the Posterior

The PGM should be solved for $P(l = 1|n_+, n_-)$. This can be rewritten as $P(l = 1|n_+, n_-) = \frac{P(l=1,n_+,n_-)}{P(n_+,n_-)} = \frac{P(l=1,n_+,n_-)}{P(l=1,n_+,n_-)+P(l=0,n_+,n_-)}$. Therefore, if $P(l, n_+, n_-)$ can be solved for by marginalizing the full joint probability over all nuisance parameters, the desired probability can be derived directly. In this PGM, there is a joint probability given by:

$$P(l, n_+, n_-, \rho, \gamma, \varepsilon) = P(\rho)P(\gamma)P(\varepsilon)P(l|\rho)P(n_+|\gamma, \varepsilon)P(n_+|\gamma, \varepsilon),$$

where each distribution is defined in the PGM diagram shown in Figure 6A. Marginalization is given by:

$$P(l, n_+, n_-) = \iiint_0^1 d\rho \, d\gamma \, d\varepsilon \cdot P(l, n_+, n_-, \rho, \gamma, \varepsilon),$$

where the solution to this integral is given by a product of beta functions. Plugging this into the equation above for the desired posterior and simplifying yields:

$$P(l = 1|n_+, n_-) = \frac{\alpha_\rho \tilde{B}_\gamma(n_+)\tilde{B}_\varepsilon(n_-)}{\alpha_\rho \tilde{B}_\gamma(n_+)\tilde{B}_\varepsilon(n_-) + \beta_\rho \tilde{B}_\gamma(n_-)\tilde{B}_\varepsilon(n_+)},$$

where the beta function $\tilde{B}_x(n_y) \equiv B(a_x + n_y, \beta_x + K_y - n_y)$.

### Generalized Majority Vote Model

The assumptions from the previous section can now be relaxed. In particular, it is almost certain that separate LFs will have different performances. Moreover, it is desirable to use different priors on different LFs when applicable. For example, sometimes there is a high certainty that a given LF is very precise, e.g., where a specific medication is only used for a particular medical condition. In such a case, it would be beneficial to set a prior for the LF to encode this knowledge, since it would be highly unlikely that the medication was recommended / prescribed for a different medical condition.

In this model, grouping LFs into "positive" LFs and "negative" LFs that each have a single observable $n_+$ or $n_-$ is no longer viable because LF priors can vary within a set of LFs. Instead, the observable must be enumerated across each LF. The voting variable described above, $\lambda_j$, can be chosen as this observable, which is 1 if the LF votes and 0 if it does not. Correspondingly, $s_j$ and $\overline{s}_j$ are used as performance variables here. The PGM for this model is shown in **Figure 6B**.

### *Solving for the Posterior*

The difference between this PGM and the majority vote PGM illustrated in Figure 6A is that the binomials by a product of Bernoulli variables have been replaced with potentially different probabilities. The derivation of the generalized majority voting model is similar to the majority voting model except that the result is a product of $K$ beta functions, rather than just 2 beta functions, in the term(s) in the numerator and denominator. Furthermore, by using the beta function identity

$B(x, y + 1) = B(x,y)\frac{y}{x+y}$ , the solution can be further simplified. Specifically:

$$P(l = 1|\lambda_0,\ldots,\lambda_K) = \frac{\alpha_\rho \prod_j \omega(s_j)}{\alpha_\rho \prod_j \omega(s_j) + \beta_\rho \prod_j \omega(\overline{s}_j)},$$

where:
$$\omega(s_j) \equiv \frac{\alpha_{x_j}^{\lambda_j} \beta_{x_j}^{1-\lambda_j}}{\alpha_{x_j}+\beta_{x_j}}.$$

## REFERENCES CITED AND ALTERNATIVE EMBODIMENTS

All references cited herein are incorporated herein by reference in their entirety and for all purposes to the same extent as if each individual publication or patent or patent application was specifically and individually indicated to be incorporated by reference in its entirety for all purposes.

Another aspect of the present disclosure provides a computer system comprising one or more processors, and a non-transitory computer-readable medium including computer-executable instructions that, when executed by the one or more processors, cause the processors to perform a method according to any one of the embodiments disclosed herein, and/or any combinations, modifications, substitutions, additions, or deletions thereof as will be apparent to one skilled in the art.

Another aspect of the present disclosure provides a non-transitory computer-readable storage medium having stored thereon program code instructions that, when executed by a processor, cause the processor to perform the method according to any one of the embodiments disclosed herein, and/or any combinations, modifications, substitutions, additions, or deletions thereof as will be apparent to one skilled in the art.

The present invention can be implemented as a computer program product that comprises a computer program mechanism embedded in a non-transitory computer readable storage medium. For instance, the computer program product could contain the program modules shown in any combination in FIG. 1 and/or as described elsewhere within the application. These program modules can be stored on a CD-ROM, DVD, magnetic disk storage product, USB key, or any other non-transitory computer readable data or program storage product.

Many modifications and variations of this disclosure can be made without departing from its spirit and scope, as will be apparent to those skilled in the art. The specific embodiments described herein are offered by way of example only. The embodiments were chosen and described in order to best explain the principles of the invention and its practical applications, to thereby enable others skilled in the art to best utilize the invention and various embodiments with various modifications as are suited to the particular use contemplated. The disclosure is to be limited only by the terms of the appended claims, along with the full scope of equivalents to which such claims are entitled.

### Claims

1. A method for training a model for phenotyping subjects with respect to a medical condition, comprising:
   at a computer system that includes one or more processors and memory:

   generating a plurality of labeling functions for the medical condition, wherein each respective labeling function in the plurality of labeling functions comprises a corresponding set of one or more criterion that, when satisfied, indicate a presence of the medical condition; and
   assigning, to each respective subject in a first plurality of subjects, a corresponding label indicating a status of the medical condition by evaluating first information from a corresponding medical record for the respective subject using an ensemble model comprising the plurality of labeling functions to obtain as output from the ensemble

EP 4 531 057 A1

model a prediction for the status of the medical condition, wherein the evaluating comprises natural language processing of at least a portion of the respective medical record.

2. The method of claim 1, wherein the method further comprises, prior to generating the plurality of labeling functions: filtering a second plurality of subjects in accordance with a first set of characteristics for the medical condition, wherein the filtering comprises applying an extraction filter to medical records corresponding to the second plurality of subjects, to obtain the first plurality of subjects, wherein the first plurality of subjects is a subset of the second plurality of subjects.

3. The method of claim 1 or 2, wherein the method further comprises: training a classification model for phenotyping subjects with respect to the medical condition using, for each respective subject in the first plurality of subjects, (i) the corresponding label as a dependent variable and (ii) second information from the corresponding medical record as independent variables.

4. The method according to any one of claims 1-3, wherein:

the extraction filter comprises a plurality of criteria related to the first set of characteristics for the medical condition; and
applying the extraction filter to the plurality of medical records comprises, for each respective medical record in a second plurality of medical records, evaluating the respective medical record for respective criterion in the plurality of criteria, wherein satisfaction of a single respective criterion is sufficient for inclusion or exclusion of the respective medical record in a first plurality of medical records,
optionally wherein applying the extraction filter comprises, for each respective subject in the second plurality of subjects, evaluating a first data type for a first respective criterion in the plurality of criteria and evaluating a second data type for a second respective criterion in the plurality of criteria.

5. The method of claim 4, further comprising:

evaluating, for one or more respective subjects in the second plurality of subjects excluded from the first plurality of subjects, whether the corresponding medical record indicates the presence of the medical condition independent of the evaluation of the respective medical record during application of the extraction filter; and
upon a determination that the respective medical record indicates the presence of the medical condition, revising the plurality of criteria related to the first set of characteristics to be more inclusive when applying the extraction filter.

6. The method of claim 4, wherein the first data type is structured data in an electronic health record (EHR) and the second type of data is unstructured data in the EHR.

7. The method according to any one of claims 4-6, wherein applying the extraction filter comprises evaluating at least three data types selected from:

structured electronic health record (EHR) data;
unstructured EHR data;
laboratory results;
prescribed medications; and
performed medical procedures.

8. The method according to any one of claims 4-7, wherein the plurality of criteria comprises (i) a first criterion that when satisfied excludes a respective subject from the first plurality of subjects and (ii) a second criterion that when satisfied results in inclusion of the respective subject in the first plurality of subjects, provided no criterion in the plurality of criteria excludes the respective subject from the first plurality of subjects.

9. The method according to any one of claims 1-10, wherein the plurality of labeling functions comprises:

a respective labeling function for each respective subgroup in a plurality subgroups of subjects with the medical condition, or
a first respective labeling function comprising a corresponding set of one or more criterion that, when satisfied, indicate a presence of the medical condition.

10. The method of claim 9, wherein the generating the plurality of labeling functions comprises, for the first respective labeling function:

i) defining a first set of one or more criterion that, when satisfied, indicate the presence of the medical condition,
ii) determining, for each respective subject in a first subset of the first plurality of subjects, whether the corresponding medical record for the respective subject satisfies the first set of one or more criterion,
iii) evaluating, for a respective subject in the first subset of the first plurality of subjects determined to satisfy the first set of one or more criteria whether the corresponding medical record indicates the absence of the medical condition, independent of the determination of whether the corresponding medical record satisfies the first set of one or more criterion, and
iv) upon a determination that the corresponding medical record indicates the absence of the medical condition, revising the first set of one or more criteria to be less inclusive, optionally wherein the revising includes adding an exclusion criterion to the first set of one or more criterion.

11. The method according to any one of claims 1-10, wherein the plurality of labeling functions comprises a second respective labeling function comprising a corresponding set of one or more criterion that, when satisfied, indicate an absence of the medical condition.

12. The method of claim 11, wherein the generating the plurality of labeling functions comprises, for the second respective labeling function:

i) defining a second set of one or more criterion that, when satisfied, indicate the absence of the medical condition,
ii) determining, for each respective subject in a second subset of the first plurality of subjects, whether the corresponding medical record satisfies the second set of one or more criterion,
iii) evaluating, for a respective subject in the second subset of the first plurality of subjects determined to satisfy the second set of one or more criteria whether the corresponding medical record indicates the presence of the medical condition, independent of the determination of whether the respective medical record satisfies the second set of one or more criterion, and
iv) upon a determination that the respective medical record indicates the presence of the medical condition, revising the second set of one or more criteria to be more inclusive, optionally wherein the revising includes adding an inclusion criterion to the second set of one or more criterion.

13. The method according to any one of claims 1-12, wherein the ensemble model comprises:

1) an aggregate voting model based on the evaluation of each respective labeling function, optionally wherein the aggregate voting model is a weighted voting model, or
2) a probabilistic graphical model.

14. The method according to any one of claims 1-13, wherein, for each respective subject in the first plurality of subjects, evaluating the ensemble model comprising the plurality of labeling functions comprises evaluating a first data type for a first respective criterion in the plurality of criteria and evaluating a second data type for a second respective criterion in the plurality of criteria, optionally wherein the first data type is structured data in an electronic health record (EHR) and the second type of data is unstructured data in the EHR.

15. The method according to any one of claims 1-14, wherein, for a respective subject in the plurality of subjects, the applying comprises natural language processing of unstructured clinical notes from the HER, optionally wherein the natural language processing comprises:

1) matching one or more regular expressions against the corresponding unstructured clinical notes,
2) inputting at least a portion of the unstructured clinical notes into a machine learning model trained to identify language related to the clinical condition, or
3) inputting at least a portion of the unstructured clinical notes into a large language model.

16. The method according to any one of claims 3-15, wherein the classification model is a neural network, a support vector machine, a Naive Bayes model, a nearest neighbor model, a boosted trees model, a random forests model, or a clustering model.

17. The method according to any one of claims 3-16, wherein the second information:

comprises at least two data types,
comprises structured electronic health record (EHR) data and unstructured EHR data,
comprises at least three data types selected from:

structured data in an electronic health record (EHR);
unstructured data in an EHR;
laboratory results;
prescribed medications; and
performed medical procedures,

comprises a data type that is not part of the first information, optionally wherein the data type in the second information that is not part of the first information is laboratory results, or
comprises all or a portion of the first information.

18. The method according to any one of claims 1-17, wherein the medical condition is pulmonary hypertension, optionally wherein the second information comprises electrocardiogram results.

19. The method according to any one of claims 1-18, wherein the method further comprises:

identifying, from a plurality of previously undiagnosed subjects, one or more respective subjects having the medical condition by inputting, for each respective subject in the plurality of subjects, corresponding second information for the respective subject into the classification model to receive as output a corresponding indication of whether the respective subject has the medical condition, optionally wherein the plurality of subjects are associated with a medical service provider, or
predicting whether a test subject has the medical condition by inputting second information for the test subject into the classification model to receive as output an indication of whether the test subject has the medical condition.

20. A computer system, comprising:

one or more processors; and
a non-transitory computer-readable medium including computer-executable instructions that, when executed by the one or more processors, cause the processors to perform the method according to any one of claims 1-19.

21. A non-transitory computer-readable storage medium having stored thereon program code instructions that, when executed by a processor, cause the processor to perform the method according to any one of claims 1-19.

| System 100 | Operating system 30 |
| | Network communication module 32 |
| | Electronic Health Records 40 |
| | EHR 1 41-1 |
| | EHR 2 41-2 |
| | ⋮ |
| | EHR A 41-A |
| | Optional filter module 50 |
| | Filter 51 |
| | Criteria 1 52-1 |
| | Criteria 2 52-2 |
| | ⋮ |
| | Criteria B 52-B |
| | Weak labeling module 60 |
| | Label functions 61 |
| | Label function 1 61-1 |
| | Label function 2 61-2 |
| | ⋮ |
| | Label function C 61-C |
| | Ensemble model 63 |
| | Optional training module 70 |
| | Hyperparameter search algorithm 71 |
| | Parameter training algorithm 72 |
| | Classification model 80 |
| | Hyperparameters 81 |
| | Model parameters 82 |
| | Phenotyping module 90 |

Non-persistent memory 111

114

Network 105

Processing core 102

Persistent memory 112

User interface 106
Display 108
Keyboard 110

Network interface 104

**FIG. 1A**

Electronic Health Records 40

**Non-persistent memory 111**

EHR 1 41-1
- Structured clinical data 42-1
- Unstructured clinical data 43-1
- Medications 44-1
- Medical procedures 45-1
- Laboratory results 46-1
- Weak label 47-1
- Classification 48-1

EHR 2 41-2
- Structured clinical data 42-2
- Unstructured clinical data 43-2
- Medications 44-2
- Medical procedures 45-2
- Laboratory results 46-2
- Weak label 47-2
- Classification 48-2

⋮

EHR A 41-A
- Structured clinical data 42-A
- Unstructured clinical data 43-A
- Medications 44-A
- Medical procedures 45-A
- Laboratory results 46-A
- Weak label 47-A
- Classification 48-A

# FIG. 1B

Optional filter module 50

Filter 51

Criteria 1 52-1

Characteristic 1-1 53-1-1

Characteristic 1-2 53-1-2

⋮

Characteristic 1-D 53-1-D

Criteria 2 52-2

Characteristic 2-1 53-2-1

Characteristic 2-2 53-2-2

⋮

Characteristic 2-E 53-2-E

⋮

Criteria B 52-B

Characteristic B-1 53-B-1

Characteristic B-2 53-B-2

⋮

Characteristic B-F 53-B-F

Non-persistent memory 111

**FIG. 1C**

Weak labeling module 60

Label functions 61

Label function 1 61-1

Criterion 1 62-1-1

Criterion 2 62-1-2

⋮

Criterion G 62-1-G

Label function 2 61-2

Criterion 1 62-2-1

Criterion 2 62-2-2

⋮

Criterion H 62-2-H

⋮

Label function C 61-C

Criterion 1 62-C-1

Criterion 2 62-C-2

⋮

Criterion I 62-C-I

Non-persistent memory 111

## FIG. 1D

## 200

*(202)* Filtering a second plurality of subjects in accordance with a first set of characteristics for the medical condition, where the filtering includes applying an extraction filter to medical records corresponding to the second plurality of subjects, to obtain the first plurality of subjects, where the first plurality of subjects is a subset of the second plurality of subjects.

*(204)* The extraction filter includes a plurality of criteria related to the first set of characteristics for the medical condition. Applying the extraction filter to the medical records includes, for each respective medical record in a second plurality of medical records, evaluating the respective medical record for respective criterion in the plurality of criteria, where satisfaction of a single respective criterion is sufficient for inclusion or exclusion of the respective medical record in a first plurality of medical records.

*(206)* Evaluating, for one or more respective subjects in the second plurality of subjects excluded from the first plurality of subjects, whether the corresponding medical record indicates the presence of the medical condition independent of the evaluation of the respective medical record during application of the extraction filter, and upon a determination that the respective medical record indicates the presence of the medical condition, revising the plurality of criteria related to the first set of characteristics to be more inclusive when applying the extraction filter.

*(208)* Applying the extraction filter includes, for each respective subject in the second plurality of subjects, evaluating a first data type for a first respective criterion in the plurality of criteria and evaluating a second data type for a second respective criterion in the plurality of criteria.

*(210)* The first data type is structured data in an electronic health record (EHR) and the second type of data is unstructured data in the EHR.

*(212)* Applying the extraction filter includes evaluating at least three data types selected from structured electronic health record (EHR) data, unstructured HER data, laboratory results, prescribed medications, and performed medical procedures.

**A**

## FIG. 2A

A

*(202 continued)*

*(204 continued)*

*(214)* The plurality of criteria includes (i) a first criterion that when satisfied excludes a respective subject from the first plurality of subjects and (ii) a second criterion that when satisfied results in inclusion of the respective subject in the first plurality of subjects, provided no criterion in the plurality of criteria excludes the respective subject from the first plurality of subjects.

*(216)* The second plurality of subjects includes at least 10,000 subjects.

*(218)* The medical condition is pulmonary hypertension.

*(220)* Generating a plurality of labeling functions for the medical condition, where each respective labeling function in the plurality of labeling functions includes a corresponding set of one or more criterion that, when satisfied, indicate a presence or an absence of the medical condition.

*(222)* The plurality of labeling functions includes a respective labeling function for each respective subgroup in a plurality subgroups of subjects with the medical condition.

B

**FIG. 2B**

B

**(220 continued)**

**(224)** The plurality of labeling functions includes a first respective labeling function including a corresponding set of one or more criterion that, when satisfied, indicate a presence of the medical condition.

**(226)** Generating the plurality of labeling functions includes, for the first respective labeling function, i) defining a first set of one or more criterion that, when satisfied, indicate the presence of the medical condition, ii) determining, for each respective subject in a first subset of the first plurality of subjects, whether the corresponding medical record for the respective subject satisfies the first set of one or more criterion, iii) evaluating, for a respective subject in the first subset of the first plurality of subjects determined to satisfy the first set of one or more criteria whether the corresponding medical record indicates the absence of the medical condition, independent of the determination of whether the corresponding medical record satisfies the first set of one or more criterion, and iv) upon a determination that the corresponding medical record indicates the absence of the medical condition, revising the first set of one or more criteria to be less inclusive.

**(228)** The revising includes adding an exclusion criterion to the first set of one or more criterion.

C

**FIG. 2C**

C

_(220 continued)_

_(230)_ The plurality of labeling functions includes a second respective labeling function including a corresponding set of one or more criterion that, when satisfied, indicate an absence of the medical condition.

_(232)_ Generating the plurality of labeling functions includes, for the second respective labeling function, i) defining a second set of one or more criterion that, when satisfied, indicate the absence of the medical condition, ii) determining, for each respective subject in a second subset of the first plurality of subjects, whether the corresponding medical record satisfies the second set of one or more criterion, iii) evaluating, for a respective subject in the second subset of the first plurality of subjects determined to satisfy the second set of one or more criteria whether the corresponding medical record indicates the presence of the medical condition, independent of the determination of whether the corresponding medical record satisfies the second set of one or more criterion, and iv) upon a determination that the respective medical record indicates the presence of the medical condition, revising the second set of one or more criteria to be more inclusive.

_(234)_ The revising includes adding an inclusion criterion to the second set of one or more criterion.

_(236)_ The plurality of label functions includes at least 10 labeling functions.

D

**FIG. 2D**

D

*(236)* Assigning, to each respective subject in a first plurality of subjects, a corresponding label indicating a status of the medical condition by evaluating first information from a corresponding medical record for the respective subject using an ensemble model including the plurality of labeling functions to obtain as output from the ensemble model a prediction for the status of the medical condition, where the evaluating includes natural language processing of at least a portion of the respective medical record.

*(238)* The first plurality of subjects includes at least 10,000 subjects.

*(240)* The ensemble model includes an aggregate voting model based on the evaluation of each respective labeling function.

*(242)* The aggregate voting model is a weighted voting model.

*(244)* The ensemble model includes a probabilistic graphical model.

*(246)* Evaluating the ensemble model including the plurality of labeling functions includes, for each respective subject in the first plurality of subjects, evaluating a first data type for a first respective criterion in the plurality of criteria and evaluating a second data type for a second respective criterion in the plurality of criteria.

*(248)* The first data type is structured data in an electronic health record (EHR) and the second type of data is unstructured data in the EHR.

E

**FIG. 2E**

E

**(236 continued)**

**(250)** Applying the extraction filter includes evaluating at least three data types selected from structured electronic health record (EHR) data, unstructured EHR data, laboratory results, prescribed medications, and performed medical procedures.

**(252)** Applying the extraction filter includes, for a respective subject in the plurality of subjects, natural language processing of unstructured clinical notes from the EHR.

**(254)** The natural language processing includes matching one or more regular expressions against the corresponding unstructured clinical notes.

**(256)** The natural language processing includes inputting at least a portion of the unstructured clinical notes into a machine learning model trained to identify language related to the clinical condition.

**(258)** The natural language processing includes inputting at least a portion of the unstructured clinical notes into a large language model.

F

**FIG. 2F**

F

*(258)* Training a classification model for phenotyping subjects with respect to the medical condition using, for each respective subject in the first plurality of subjects, (i) the corresponding label as a dependent variable and (ii) second information from the corresponding medical record as independent variables.

*(260)* The classification model is a neural network, a support vector machine, a Naive Bayes model, a nearest neighbor model, a boosted trees model, a random forests model, or a clustering model.

*(262)* The second information includes at least two data types.

*(264)* The second information includes structured electronic health record (EHR) data and unstructured HER data.

*(266)* The second information includes at least three data types selected from structured data in an electronic health record (EHR), unstructured data in an EHR, laboratory results, prescribed medications, and performed medical procedures.

*(268)* The second information includes a data type that is not part of the first information.

*(270)* The data type in the second information that is not part of the first information is laboratory results.

*(272)* The second information includes all or a portion of the first information.

*(274)* The medical condition is pulmonary hypertension and the second information includes electrocardiogram results.

G

H

FIG. 2G

G

*(274)* Identifying, from a plurality of previously undiagnosed subjects, one or more respective subjects having the medical condition by inputting, for each respective subject in the plurality of subjects, corresponding second information for the respective subject into the classification model to receive as output a corresponding indication of whether the respective subject has the medical condition.

*(276)* The plurality of subjects are associated with a medical service provider.

H

*(278)* Predicting whether a test subject has the medical condition by inputting second information for the test subject into the classification model to receive as output an indication of whether the test subject has the medical condition.

FIG. 2H

**FIG. 3**

FIG. 4A

FIG. 4B

**EHR Population**

Meds  Procedures

Lab Values  Notes

Signal Aggregation

**FIG. 4C**

Procedures

Notes

Meds

Lab Values

EHR Population

FIG. 4D

FIG. 4E

FIG. 4F

EP 4 531 057 A1

Potential Missed Cases^

Extraction Filter*

EHR Population

^ **Assumption** is that drop in Sensitivity of filter is *negligible*.
* Extraction filter is *union* of regex hits and broadest version of structured features.

FIG. 4G

502 Total true patients with PH   504 Cumulative unique patients identified by structured data   506 Patients meeting each structured criteria

**FIG. 5**

$$\rho \sim \text{Beta}(\alpha_\rho, \beta_\rho) \qquad l \sim \text{Bern}(\rho)$$
$$\gamma \sim \text{Beta}(\alpha_\gamma, \beta_\gamma) \qquad n_+ \sim \text{Binom}(K_+, \gamma^l \varepsilon^{1-l})$$
$$\varepsilon \sim \text{Beta}(\alpha_\varepsilon, \beta_\varepsilon) \qquad n_- \sim \text{Binom}(K_-, \varepsilon^l \gamma^{1-l})$$

**FIG. 6A**

$$\rho \sim \text{Beta}(\alpha_\rho, \beta_\rho)$$
$$s_j \sim \text{Beta}(\alpha_{s_j}, \beta_{s_j}) \qquad l \sim \text{Bern}(\rho)$$
$$\bar{s}_j \sim \text{Beta}(\alpha_{\bar{s}_j}, \beta_{\bar{s}_j}) \qquad \lambda_j \sim \text{Bern}(s_j^l \, \bar{s}_j^{\,1-l})$$

**FIG. 6B**

**EP 4 531 057 A1**

Europäisches
Patentamt

European
Patent Office

Office européen
des brevets

# EUROPEAN SEARCH REPORT

**Application Number**

EP 24 20 3337

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHENGHAN ZHANG ET AL: "PheME: A deep ensemble framework for improving phenotype prediction from multi-modal data", ARXIV.ORG, CORNELL UNIVERSITY LIBRARY, 201 OLIN LIBRARY CORNELL UNIVERSITY ITHACA, NY 14853, 19 March 2023 (2023-03-19), XP091462463, * The whole document, in particular: Pages: 2, 3. Figures: Fig. 1. * | 1-21 | INV. G16H50/70 G16H50/20 G06N20/20 |
| X | US 2020/250580 A1 (HARMAN GREGORY [US]) 6 August 2020 (2020-08-06) * The whole document, in particular: Paragraphs: [0005] - [0008], [0018], [0021], [0023]. Figures: Fig. 1. * | 1-21 | |

----- 

**TECHNICAL FIELDS SEARCHED (IPC)**

G16H
G06N

The present search report has been drawn up for all claims

| Place of search | Date of completion of the search | Examiner |
|---|---|---|
| Munich | 6 February 2025 | Zacharias, Malte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 3337

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2025

| Patent document cited in search report | Publication date | Patent family member(s) | Publication date |
|---|---|---|---|
| US 2020250580 A1 | 06-08-2020 | US 2020250580 A1 | 06-08-2020 |
| | | WO 2020159649 A1 | 06-08-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 1969149 W
- US 20210090694 A
- US 202080365232 A
- US 17908621
- US 17926721
- US 17927921
- US 20210115511 A
- US 32398621
- US 35223121
- US 20200098448 A
- US 20200210852 A
- US 1969161 W
- US 07498420
- US 11043283 B
- US 40502521
- US 93023420
- US 11081210 B
- US 30494021
- US 20200258601 A
- US 20200395097 A
- US 10957041 B
- US 10975445 B
- US 49251821
- US 20210057042 A
- US 20200075169 A

- US 20200118644 A
- US 20210098078 A
- US 11043304 B
- US 2118619 W
- US 30203021
- US 20210057071 A
- US 11145416 B
- US 45587621
- US 20200335102 A
- US 10395772 B
- US 10902952 B
- US 20210151192 A
- US 54604921
- US 20200381087 A
- US 20200135303 A
- US 20200211716 A
- US 20210155989 A
- US 2056930 W
- US 20210172931 A
- US 2020063619 W
- US 30197521
- US 20210118559 A
- US 789363
- US 20180002 W

**Non-patent literature cited in the description**

- **COOREVITS et al.** Electronic health records: new opportunities for clinical research. *J Intern Med.*, 2013, vol. 274 (6), 547-560
- **PENDERGRASS** ; **CRAWFORD**. Using Electronic Health Records to Generate Phenotypes For Research. *Curr Protoc Hum Genet.*, 2019, vol. 100 (1), e80
- **COWIE et al.** Electronic health records to facilitate clinical research. *Clin Res Cardiol.*, 2017, vol. 106 (1), 1-9
- **XIAO et al.** Opportunities and challenges in developing deep learning models using electronic health records data: a systematic review. *J Am Med Inform Assoc.*, 2018, vol. 25 (10), 1419-1428
- Imagenet classification with deep convolutional neural networks. **KRIZHEVSKY et al.** Advances in Neural Information Processing Systems. Curran Associates, Inc., 2012, vol. 2, 1097-1105
- **ZEILER**. ADADELTA: an adaptive learning rate method. *CoRR*, 2012, vol. abs/1212.5701

- Neurocomputing: Foundations of research. **RUMEL-HART et al.** Learning Representations by Back-propagating Errors. MIT Press, 1988, 696-699
- **VINCENT et al.** Stacked denoising autoencoders: Learning useful representations in a deep network with a local denoising criterion. *J Mach Learn Res*, 2010, vol. 11, 3371-3408
- **LAROCHELLE et al.** Exploring strategies for training deep neural networks. *J Mach Learn Res*, 2009, vol. 10, 1-40
- **HASSOUN**. Fundamentals of Artificial Neural Networks. Massachusetts Institute of Technology, 1995
- **DUDA et al.** Pattern Classification. John Wiley & Sons, Inc., 2001
- **HASTIE et al.** The Elements of Statistical Learning. Springer-Verlag, 2001
- **DRAGHICI**. Data Analysis Tools for DNA Microarrays. Chapman & Hall/CRC, 2003

- **MOUNT**. Bioinformatics: sequence and genome analysis. Cold Spring Harbor Laboratory Press, 2001
- **CRISTIANINI** ; **SHAWE-TAYLOR**. An Introduction to Support Vector Machines. Cambridge University Press, 2000
- A training algorithm for optimal margin classifiers. **BOSER et al.** Proceedings of the 5th Annual ACM Workshop on Computational Learning Theory. ACM Press, 1992, 142-152
- **VAPNIK**. Statistical Learning Theory. Wiley, 1998
- **DUDA**. Pattern Classification. John Wiley & Sons, Inc., 2001, vol. 259, 262-265
- **HASTIE**. The Elements of Statistical Learning. Springer, 2001
- **FUREY et al.** *Bioinformatics*, 2000, vol. 16, 906-914
- **NG et al.** On discriminative vs. generative classifiers: A comparison of logistic regression and naive Bayes. *Advances in Neural Information Processing Systems*, 2002, vol. 14
- **HASTIE et al.** The elements of statistical learning: data mining, inference, and prediction. Springer, 2001
- **DUDA**. Pattern Classification. John Wiley & Sons, Inc, 2001
- **DUDA et al.** Pattern Classification. John Wiley & Sons, 2001
- **DUDA**. Pattern Classification. John Wiley & Sons, Inc., 2001, 395-396
- **DUDA**. Pattern Classification. John Wiley & Sons, Inc., 2001, 396-408, 411-412
- Random Forests--Random Features. **BREIMAN**. Technical Report. Statistics Department, U.C. Berkeley, September 1999, vol. 567
- **AGRESTI**. An Introduction to Categorical Data Analysis. John Wiley & Son, 1996, 103-144
- **MCLACHLAN et al.** *Bioinformatics*, 2002, vol. 18 (3), 413-422
- **SCHLIEP et al.** *Bioinformatics*, 2003, vol. 19 (1), i255-i263
- **DUDA** ; **HART**. Pattern Classification and Scene Analysis. John Wiley & Sons, Inc., 1973, 211-256
- **FERNANDES et al.** Transfer Learning with Partial Observability Applied to Cervical Cancer Screening. *Pattern Recognition and Image Analysis: 8th Iberian Conference Proceedings*, 2017, 243-250
- **KEERTHANA et al.** Text Detection and Recognition: A Review. *IRJET*, 2020, vol. 7 (8), 2156-2169
- **ROKACH L. et al.** *Information Retrieval Journal*, 2008, vol. 11 (6), 499-538
- *List of Targeted Therapy Drugs Approved for Specific Types of Cancer*, 31 October 2022
- **LUCIANO et al.** The Translational Medicine Ontology and Knowledge Base: driving personalized medicine by bridging the gap between bench and bedside. *Journal of Biomedical Semantics*, 2011, vol. 2 (2), S1
- *NCIthesaurus*, 28 November 2022
- **MASOUDI-SOBHANZADEH et al.** Drug databases and their contributions to drug repurposing. *Genomics*, 2020, vol. 112 (2), 1087-1095
- **BULUS et al.** Comparison of String-Matching Algorithms in Web Documents. *UniTech*, 2017
- **CAMPELLO et al.** Hierarchical density estimates for data clustering, visualization, and outlier detection. *CM Trans Knowl Disc Data*, 2015, vol. 10 (1), 5
- **BLONDEL et al.** Fast unfolding of communities in large networks. *J stat mech: theor exp*, 2008, vol. 2008 (10), 10008
- **TRAAG et al.** From Louvain to Leiden: guaranteeing well-connected communities. *Sci Rep*, 2019, vol. 9, 5233
- **DEVLIN et al.** BERT: Pre-training of Deep Bidirectional Transformers for Language Understanding. *arXiv:1810.04805v2*, 2018
- A survey of dimension reduction techniques. **FODOR**. Technical Report UCRL-ID-148494. Center for Applied Scientific Computing, Lawrence Livermore National, 2002
- Dimension Reduction. **CUNNINGHAM**. Technical Report UCD-CSI-2007-7. University College Dublin, 2007
- **ZAHORIAN et al.** Nonlinear Dimensionality Reduction Methods for Use with Automatic Speech Recognition. *Speech Technologies*, 2011, ISBN 978-953-307-996-7
- **LAKSHMI et al.** *2016 IEEE 6th International Conference on Advanced Computing (IACC)*, 2016, ISBN 978-1-4673-8286-1, 31-34
- **FRUCHTERMAN, T. M.** ; **REINGOLD, E. M.** Graph drawing by force-directed placement. *Software: Practice and experience*, 1991, vol. 21 (11), 1129-1164
- **ROWEIS, S. T.** ; **SAUL, L. K.** Nonlinear dimensionality reduction by locally linear embedding. *Science*, 2000, vol. 290 (5500), 2323-2326
- **TENENBAUM, J. B.** ; **DE SILVA, V.** ; **LANGFORD, J. C.** A global geometric framework for nonlinear dimensionality reduction. *Science*, 2000, vol. 290 (5500), 2319-2323
- **WANG et al.** Adaptive Manifold Learning. *Advances in Neural Information Processing Systems*, 2004, vol. 17
- **MANDRAS SA et al.** Pulmonary Hypertension: A Brief Guide for Clinicians. *Mayo Clin Proc.*, 2020, vol. 95 (9), 1978-88

- **CAMPELLO et al.** Hierarchical density estimates for data clustering, visualization, and outlier detection. *ACM Trans Knowl Disc Data*, 2015, vol. 10 (1), 5
- A survey of dimension reduction techniques. **FODOR**. Lawrence Livermore National, Technical Report UCRL-ID-148494. Center for Applied Scientific Computing, 2002
- **RATNER A. et al.** Snorkel: Rapid Training Data Creation with Weak Supervision. *PVLDB*, 2017, vol. 11 (3)
- **KOLLER** ; **FRIEDMAN**. Probabilistic Graphical Models. MIT Press, 2009